# EUROPEAN PATENT APPLICATION

(11) **EP 3 053 573 A1**
(43) Date of publication of application: **10.08.2016**
(21) Application number: 15193532.7
(22) Date of filing: 06.11.2015
(51) Int. Cl.: A61K 9/00, A61K 47/26, A61K 47/32, A61K 9/08

(54) **LIQUID PHARMACEUTICAL COMPOSITION**

(30) Priority: 06.02.2015 EP 15154239
(71) Applicant: ARES TRADING S.A., CH-1170 Aubonne (CH)
(72) Inventor: RINALDI, Gianluca, 00015 Monterotondo (IT); FRATARCANGELI, Silvia, 03024 Ceprano (Frosinone) (IT); DEL RIO, Alessandra, 00144 Rome (IT)
(74) Representative: Holmes, Michael Anthony

(57) **Abstract**

The present invention relates to novel liquid pharmaceutical compositions of adalimumab, which include adalimumab or a biosimilar thereof, and at least one component selected from the group consisting of: a polyvinylpyrrolidone (PVP) surfactant, an inositol sugar stabiliser, and a gluconate salt toncifier. Such a combination of components furnishes formulations having a stability (e.g. on storage and when exposed to stress) which is comparable to or an improvement upon those known in the art, and with fewer ingredients. Such advances will help adalimumab treatments to become more widely available at lower cost, and prolong the viability of pre-loaded delivery devices (e.g. pre-filled syringes) to reduce unnecessary waste of the drug.

## Description

### INTRODUCTION

The present invention relates to a novel protein formulation. In particular, the invention relates to a liquid pharmaceutical composition of adalimumab, to a method of manufacturing the composition, to a kit including the composition, to a package including the composition, to a method of manufacturing the package, and to methods of treatment using the composition and/or package.

### BACKGROUND

Treatment of tumour necrosis factor-alpha (TNF-α)-related autoimmune diseases, such as rheumatoid arthritis, psoriasis and other autoimmune diseases, has been archieved through the use of FDA-approved drugs such as Adalimumab (HUMIRA®, Abbott Corporation). Adalimumab is a human monoclonal antibody that inhibits human TNF-α activity so as to prevent it from activating TNF receptors, thereby downregulating inflammatory responses associated with autoimmune diseases. Approved medical indications for Adalimumab include rheumatoid arthritis, psoriatic arthritis, ankylosing spondylitis, Crohn's disease, ulcerative colitis, moderate to severe chronic psoriasis and juvenile idiopathic arthritis.

Adalimumab is generally delivered to a patient *via* subcutaneous injection, and is thus provided in a liquid form, typically in packages such as vials, preloaded syringes, or preloaded "pen devices". Commercially available pen devices (HUMIRA^{®} Pen) generally include a 1 mL pre-filled glass syringe, preloaded with 0.8mL of a sterile formulation of 40 mg Adalimumab (see below), with a fixed needle (either gray natural rubber or a latex free version) and a needle cover. Commercial formulations (HUMIRA^{®}) of Adalimumab contain the following ingredients:

| **Ingredient** | **Amount per container (mg) (filling volume = 0.8 mL)** | **Amount (mg/mL)** |
|---|---|---|
| Adalimumab | 40 | 50 |
| Citric Acid Monohydrate | 1.04 | 1.3 |
| Dibasic sodium phosphate dehydrate | 1.22 | 1.53 |
| Mannitol | 9.6 | 12 |
| Monobasic sodium phosphate dehydrate | 0.69 | 0.86 |
| Polysorbate 80 | 0.8 | 1 |
| Sodium chloride | 4.93 | 6.16 |
| Sodium citrate | 0.24 | 0.3 |
| WFI and sodium hydroxide | q.b. to adjust pH to 5.2 | q.b. to adjust pH to 5.2 |

Adalimumab, and its method of manufacture, is described in WO97/29131 (BASF) as D2E7, and elsewhere in the art.

Though the aforementioned commercial formulation of Adalimumab is stable (at least to some extent), the relevant antibody may be unstable over prolonged periods or under stressed conditions, thus precluding prolonged storage of said formulations. Such degradation of the formulation may be due to a variety of factors, including:
- **Physical effects,** such as:
   o Inadequate inhibition of aggregation of the relevant protein molecules (a function supposedly served by Tween-80);
   o Inadequate inhibition of precipitation;
   o Inadequate inhibition of adsorption of the relevant protein molecules at the interface of water and air or at the contact surface of any packaging material (a function supposedly served by Tween-80);
   o Inadequate regulation of osmotic pressure (a function supposedly served by mannitol);
- **Chemical effects,** such as:
   o Inadequate regulation of oxidation (a function supposedly served by mannitol and potentially undermined by Tween-80, which can promoted oxidation of double bonds);
   o Inadquate inhibition of photo-oxidation;
   o Inadquate inhibition of hydrolysis of ester bonds leading to the formation of acid, aldehyde and peroxide products, thus affecting the stability of the antibody;
   o Inadequate stabilisation and maintenance of pH;
   o Inadequate inhibition of protein fragmentation;
   o Inadequate inhibiition of protein unfolding;

Any, some, or all of the above factors can lead to either an unviable drug product (which may be unsafe for use in medical treatments) or a drug product whose viability is variable and unpredictable, especially in view of the variable stresses (agitation, heat, light) different batches of drug product may be exposed to during manufacture, transport, and storage.

In terms of the physical and chemical stabilisation of Adalimumab, the complex array of components within the aforementioned commercial formulations appears to perform below expectations, especially in view of the large number of components. Though this particular combination of excipients undoubtably represents a 'delicate balance' (given the interplay between various technical factors) and was the result of extensive research and development, in view of the risk of underperformance it is questionable whether such a large number of different excipients is justified, especially given that this inevitably increases processing and cost burdens, toxicity risks, and risks of deleterious interactions between components that could compromise the formulation. Even if the overall performance of the commercial formulations could not be surpassed, an alternative formulation having comparative performance but containing few components would represent a highly desirable replacement for the commercial formulations, for at least the aforesaid reasons.

In order to guarantee reproducible clinical performance of a protein-based pharmaceutical product, such products must remain in a stable and consistent form over time. It is well-established that molecular alterations can occur during every stage of the manufacturing process, including during the production of the final formulation and during storage. Molecular alterations can modifty a quality attribute of a biopharmaceutical product, resulting in an undesirable change in the identity, strength or purity of the product. Some such problems are outlined above.

The primary goal of formulation development is to provide a pharmaceutical composition that will support the stability of a biopharmaceutical protein during all stages of its production, storage, shipping and use. Formulation development for an innovative biopharmaceutical protein, or a biosimilar monoclonal antibody (mAb), is essential to its safety, clinical efficacy and commercial success.

There is therefore a need for the provision of alternative or improved liquid formulations of adalimumab. Desirably, any new formulations would solve at least one of the aforementioned problems and/or at least one problem inherent in the prior art, and may suitably solve two or more of said problems. Desirably, the problem(s) of the prior art may be solved whilst reducing the complexity of the formulation.

### SUMMARY OF THE INVENTION

According to a first aspect of the present invention there is provided a liquid pharmaceutical composition comprising adalimumab (which suitably includes any biosimilar thereof); ; wherein the composition optionally comprises (or excludes) any one or more additional components defined herein in relation to a liquid pharmaceutical composition (e.g. including any one or more of a buffer system, sugar stabiliser, and a surfactant; and/or substantially excluding any one or more of arginine, amino acids, and other buffers such as phosphate, etc.), optionally in any amount, concentration, or form stipulated herein; and wherein the composition optionally exhibits any one or more parameters or properties given herein in relation to a liquid pharmaceutical composition (e.g. pH, osmolality, aggregation, fragmentation, protein unfolding, turbity, etc.).

A further aspect of the invention provides a liquid pharmaceutical composition comprising: adalimumab; a sugar stabiliser; and a surfactant, on the proviso that the surfactant is a surfactant other than polysorbate 80 when the sugar stabiliser is trehalose; wherein the composition optionally comprises (or excludes) components and/or optionally exhibits parameters as set forth in the first aspect.A further aspect of the present invention provides a liquid pharmaceutical composition comprising adalimumab and at least one component selected from the group consisting of: a polyvinylpyrrolidone (PVP) surfactant, a monocyclic sugar stabiliser, and a metal (hydroxy)mono-/di-carboxylate salt tonicifier.

A further aspect of the present invention provides a liquid pharmaceutical composition comprising adalimumab and at least one component selected from the group consisting of: a polyvinylpyrrolidone (PVP) surfactant, an inositol sugar stabiliser, and a gluconate salt toncifier.

A further aspect of the invention provides a liquid pharmaceutical composition comprising: adalimumab; and a PVP surfactant (suitably as defined herein); wherein the composition optionally comprises (or excludes) components and/or optionally exhibits parameters as set forth in the first aspect.

A further aspect of the invention provides a liquid pharmaceutical composition comprising: adalimumab; and inositol; wherein the composition optionally comprises (or excludes) components and/or optionally exhibits parameters as set forth in the first aspect.

A further aspect of the invention provides a liquid pharmaceutical composition comprising: adalimumab; and a carboxylate salt tonicifier (e.g. a gluconate salt); wherein the composition optionally comprises (or excludes) components and/or optionally exhibits parameters as set forth in the first aspect.

A further aspect of the invention provides a liquid pharmaceutical composition comprising: adalimumab; inositol; and a carboxylate salt tonicifier (e.g. a gluconate salt); wherein the composition optionally comprises (or excludes) components and/or optionally exhibits parameters as set forth in the first aspect.

A further aspect of the invention provides a liquid pharmaceutical composition comprising: adalimumab; a buffer system (e.g. acetate buffer system); a sugar stabiliser (e.g. inositol); a surfactant; and a tonicifier (e.g. a gluconate salt); wherein the composition optionally comprises (or excludes) components and/or optionally exhibits parameters as set forth in the first aspect.

According to a second aspect of the present invention there is provided a liquid pharmaceutical composition comprising adalimumab; a buffer system (or buffering agent) (e.g. acetate buffer system or buffering agent); and a sugar stabiliser; wherein the composition is either (substantially or entirely) free of arginine (suitably L-arginine) or comprises arginine in a concentration of at most 0.1 mM.

According to a third aspect of the present invention there is provided a liquid pharmaceutical composition comprising adalimumab; a buffer system (or buffering agent) (e.g. acetate buffer system or buffering agent); and a sugar stabiliser; wherein the composition is either (substantially or entirely) free of arginine or comprises arginine in a molar ratio of arginine to buffering agent of at most 1 : 150 (i.e. less than or equal to one mole of arginine for every 150 moles of buffering agent).

According to a fourth aspect of the present invention there is provided a liquid pharmaceutical composition comprising adalimumab; a buffer system (or an buffering agent) (e.g. acetate buffer system or buffering agent); and a sugar stabiliser; wherein the composition is either (substantially or entirely) free of arginine or comprises arginine in a weight ratio of arginine to adalimumab of at most 1 : 3000 (i.e. less than or equal to one part by weight of arginine for every 3000 parts by weight adalimumab).

According to a fifth aspect of the present invention there is provided a package (e.g. pre-filled syringe, pen, intravenous bag, or a package/container containing any of the aforementioned) comprising a liquid pharmaceutical composition as defined herein.

According to a sixth aspect of the present invention there is provided a drug delivery device (e.g. pre-filled syringe or pen, or intravenous bag) comprising a liquid pharmaceutical composition as defined herein.

According to a seventh aspect of the present invention there is provided a kit of parts comprising a drug delivery device, a liquid pharmaceutical composition as defined herein (optionally contained in a package or container), and optionally a set of instructions with directions regarding the administration (e.g. sub-cutaneous) of the liquid pharmaceutical composition.

According to an eighth aspect of the present invention there is provided a method of manufacturing a liquid pharmaceutical composition, the method comprising mixing together adalimumab; and optionally any one or more additional components defined herein in relation to a liquid pharmaceutical composition, optionally in any amount, concentration, or form stipulated; and optionally adjusting any one or more parameters given herein in relation to a liquid pharmaceutical composition (e.g. pH, osmolality).

According to a ninth aspect of the present invention there is provided a liquid pharmaceutical composition obtainable by, obtained by, or directly obtained by a method of manufacturing a liquid pharmaceutical composition as defined herein.

According to a tenth aspect of the present invention there is provided a method of manufacturing a package or a drug delivery device, the method comprising incorporating a liquid pharmaceutical composition as defined herein within a package or drug delivery device.

According to an eleventh aspect of the present invention there is provided a package or a drug delivery device obtainable by, obtained by, or directly obtained by a method of manufacturing a package or a drug delivery device as defined herein.

According to a twelfth aspect of the present invention there is provided a method of treating a disease or medical disorder in a patient in need of such treatment, said method comprising administering to said patient a therapeutically effective amount of a liquid pharmaceutical composition as defined herein.

According to a thirteenth aspect of the present invention there is provided a liquid pharmaceutical composition as defined herein for use in therapy.

According to a fourteenth aspect of the present invention there is provided a use of a liquid pharmaceutical composition as defined herein in the manufacture of a medicament for the treatment of a disease or disorder.

According to a fifteenth aspect of the present invention there is provided a method of treating a tumour necrosis factor-alpha (TNF-α)-related autoimmune disease in a patient in need of such treatment, said method comprising administering to said patient a therapeutically effective amount of a liquid pharmaceutical composition as defined herein.

According to a sixteenth aspect of the present invention there is provided a liquid pharmaceutical composition as defined herein for use in the treatment of a tumour necrosis factor-alpha (TNF-α)-related autoimmune disease.

According to a seventeenth aspect of the present invention there is provided a use of a liquid pharmaceutical composition as defined herein in the manufacture of a medicament for the treatment of a tumour necrosis factor-alpha (TNF-α)-related autoimmune disease.

According to an eighteenth aspect of the present invention there is provided a method of treating rheumatoid arthritis, psoriatic arthritis, ankylosing spondylitis, Crohn's disease, ulcerative colitis, moderate to severe chronic psoriasis and/or juvenile idiopathic arthritis in a patient in need of such treatment, said method comprising administering to said patient a therapeutically effective amount of a liquid pharmaceutical composition as defined herein.

According to a ninteenth aspect of the present invention there is provided a liquid pharmaceutical composition as defined herein for use in the treatment of rheumatoid arthritis, psoriatic arthritis, ankylosing spondylitis, Crohn's disease, ulcerative colitis, moderate to severe chronic psoriasis and/or juvenile idiopathic arthritis.

According to a twentieth aspect of the present invention there is provided a use of a liquid pharmaceutical composition as defined herein in the manufacture of a medicament for the treatment of rheumatoid arthritis, psoriatic arthritis, ankylosing spondylitis, Crohn's disease, ulcerative colitis, moderate to severe chronic psoriasis and/or juvenile idiopathic arthritis.

In further aspects, the invention provides a liquid pharmaceutical composition, a package, a drug delivery device, a kit of parts, a method of manufacturing a liquid pharmaceutical composition, a method of manufacturing a package or a drug delivery device, a method of treating, a liquid pharmaceutical composition for use, and a use of a liquid pharmaceutical composition in the manufacture of a medicament, essentially as defined herein (including in any of the aforementionied twenty aspects) except that, rather than being specific to "adalimumab" (and biosimilars thereof), the invention may instead apply (and thereby be defined as relating) to any TNF-α-inhibiting antibody (anti-TNF-α antibody) (or any biosimilar thereof), albeit suitably an antibody that inhibits human TNF-α activity, and most suitably a human monoclonal antibody that inhibits human TNF-α activity. Suitably the anti-TNF-α antibody is a therapeutically effective medicament (at least when administered in appropriate quantities to a patient in need thereof) (or a biosimlar thereof - see below for definitions of biosimilars in relation to adalimumab, which applies equally to all anti-TNF-α antibodies), suitably one which has received FDA approval. As such, any reference herein to "adalimumab" may, unless incompatible therewith, be construed as a reference to any anti-TNF-α antibody for the purpose of these additional aspects of the invention (whether this relates to absolute or relative amounts, concentrations, parameters, or properties, or whether it relates to certain definitions, such as what constitutes a biosimilar).

One of these further aspects of the present invention provides a liquid pharmaceutical composition comprising an anti-TNF-α antibody (which suitably includes any biosimilar thereof); wherein the composition optionally comprises (or excludes) any one or more additional components defined herein in relation to a liquid pharmaceutical composition (e.g. including surfactant, excluding arginine, etc.), optionally in any amount, concentration, or form stipulated herein; and wherein the composition optionally exhibits any one or more parameters or properties given herein in relation to a liquid pharmaceutical composition (e.g. pH, osmolality, aggregation, fragmentation, protein unfolding, turbity, etc.).

In a particular embodiment, the anti-TNF-α antibody is selected from the group including adalimumab, infliximab, certolizumab pegol, golimumab.

Any features, including optional, suitable, and preferred features, described in relation to any particular aspect of the invention may also be features, including optional, suitable and preferred features, of any other aspect of the present invention.

### BRIEF DESCRIPTION OF THE FIGURES

FIG. 1 shows a graphical representation of the change in low molecular weight fragments/impurities (%LMW) over time, under stressed conditions, for each of the candidate formulations 1-4 and the control formulation.
FIG. 2 is a graph showing the formation of "acidic forms" (summation of all) for all of the relevant test formulations (including control) over the relevant time period under stressed conditions.
FIG. 3 illustrates the various fluorescence spectra for each of the tested formulations (including the control) at each point in time under stress testing and how decreases in intensity at the native λₘₐₓ of 355 nm is indicative of partial protein unfolding over time.
FIG. 4 illustrates overlayed fluorescence spectra for each of the tested formulations (including the control) after 4 weeks at 40°C.

### DETAILED DESCRIPTION OF THE INVENTION

### Definitions

Unless otherwise stated, the following terms used in the specification and claims have the following meanings set out below.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification, and the contents of all such papers and documents are incorporated herein by reference.

For the avoidance of doubt, it is hereby stated that the information described earlier in this specification under the heading "Background" is relevant to the invention and is to be read as part of the disclosure of the invention.

Suitably, unless stated otherwise, where reference is made to a parameter (e.g. pH, pKa, etc.) or state of a material (e.g. liquid, gas, etc.) which may depend on pressure and/or temperature, suitably in the absence of further clarification such a reference refers to said parameter at standard ambient temperature and pressure (SATP). SATP is a temperature of 298.15 K (25 °C, 77 °F) and an absolute pressure of 100 kPa (14.504 psi, 0.987 atm).

Unless stated otherwise, any molecular weight values (including weight averaged molecular weight values) recited herein are given in g/mol.

Unless stated otherwise, any reference herein to an "average" value is intended to relate to the mean value.

References herein to "adalimumab" include the originator drug substance (as commercially available), adalimumab as defined in WO97/29131 (BASF) (particularly D2E7 therein) and elsewhere in the art, and also biosimilars thereof. D2E7 of WO97/29131 "has a light chain CDR3 domain comprising the amino acid sequence of SEQ ID NO: 3 and a heavy chain CDR3 domain comprising the amino acid sequence of SEQ ID NO: 4". Preferably, the D2E7 antibody has a light chain variable region (LCVR) comprising the amino acid sequence of SEQ ID NO: 1 and a heavy chain variable region (HCVR) comprising the amino acid sequence of SEQ ID NO: 2. WO97/29131 gives details of each of these sequence listings. References herein to "adalimumab" may include biosimilars which, for instance, may share at least 75%, suitably at least 80%, suitably at least 85%, suitably at least 90%, suitably at least 95%, suitably at least 96%, suitably at least 97%, suitably at least 98% or most suitably at least 99% protein sequence identity with any one of protein sequences disclosed in either WO97/29131 (especially in relation to D2E7) or elsewhere in relation to "adalimumab". Alternatively or additionally, references herein to "adalimumab" may include biosimilars which exhibit at least 75%, suitably at least 80%, suitably at least 85%, suitably at least 90%, suitably at least 95%, suitably at least 96%, suitably at least 97%, suitably at least 98% or most suitably at least 99% protein sequence homology with any one of protein sequences disclosed in either WO97/29131 (especially in relation to D2E7) or elsewhere in relation to "adalimumab". Alternatively or additionally, a biosimilar may have a (slightly) different glycosylation profile, even if the protein sequence is substantially the same or different to the extent specified above.

The term "biosimilar" (also know as follow-on biologics) is well known in the art, and the skilled person would readily appreciate when a drug substance would be considered a biosimilar of adalimumab. Furthermore, such "biosimilars" would need to be officially approved as a "biosimilar" for marketing before said "biosimilar" is sold on the open market. The term "biosimilar" is generally used to describe subsequent versions (generally from a different source) of "innovator biopharmaceutical products" ("biologics" whose drug substance is made by a living organisim or devived from a living organism or through recombinant DNA or controlled gene expression methodologies) that have been previously officially granted marketing authorisation. Since biologics have a high degree of molecular complexity, and are generally sensitive to changes in manufacturing processes (e.g. if different cell lines are used in their production), and since subsequent follow-on manufacturers generally do not have access to the originator's molecular clone, cell bank, know-how regarding the production process, nor to the active drug substance itself (only the innovator's commercialized drug product), any "biosimilar" may not be exactly the same as the innovator drug product.

For the purposes of various molar calculatations (e.g. for molar ratios between adalimumab and another component of the liquid pharmaceutical composition of the invention) the molecular weight of adalimumab may be taken to be 144190.3 g/mol (reference molecular weight) based on details disclosed on the CAS database for CAS # 331731-18-1, Adalimumab, where the molecular formula is taken as C₆₄₂₈H₉₉₁₂N₁₆₉₄O₁₉₈₇S₄₆. As such, a liquid pharmaceutical composition containing 50 mg/mL adalimumab may be considered a 0.347 mM (or 347 µM) solution of adalimumab. This is not intended to be in any way limiting regarding the nature of any biosimilars of adalimumab covered by the scope of the present invention, nor the level of glycosylation, either of which may effect the actual molecular weight. However, where a biosimilar does have a different molecular weight, the abovementioned reference molecular weight should be suitably used for the purposes of assessing whether or not such a biosimilar falls within the scope of any molar definitions stipulated within this specification. So the number of moles in a known weight of said biosimilar should be calculated, just for the purposes of this invention, using the above reference molecular weight.

Herein, the term "buffer" or "buffer solution" refers to a generally aqueous solution comprising a mixture of an acid (usually a weak acid, e.g. acetic acid, citric acid, imidazolium form of histidine) and its conjugate base (e.g. an acetate or citrate salt, for example, sodium acetate, sodium citrate, or histidine) or alternatively a mixture of a base (usually a weak base, e.g. histidine) and its conjugate acid (e.g. protonated histidine salt). The pH of a "buffer solution" will change very only slightly upon addition of a small quantity of strong acid or base due to the "buffering effect" imparted by the "buffering agent".

Herein, a "buffer system" comprises one or more buffering agent(s) and/or an acid/base conjugate(s) thereof, and more suitably comprises one or more buffering agent(s) and an acid/base conjugate(s) thereof, and most suitably comprises one buffering agent only and an acid/base conjugate thereof. Unless stated otherwise, any concentrations stipulated herein in relation to a "buffer system" (i.e. a buffer concentration) suitably refers to the combined concentration of the buffering agent(s) and/or acid/base conjugate(s) thereof. In other words, concentrations stipulated herein in relation to a "buffer system" suitably refer to the combined concentration of all the relevant buffering species (i.e. the species in dynamic equilibrium with one another, e.g. acetate/acetic acid). As such, a given concentration of an acetate buffer system generally relates to the combined concentration of acetate (or acetate salt(s), e.g. sodium acetate) and acetic acid. The overall pH of the composition comprising the relevant buffer system is generally a reflection of the equilibrium concentration of each of the relevant buffering species (i.e. the balance of buffering agent(s) to acid/base conjugate(s) thereof).

Herein, the term "buffering agent" refers to an acid or base component (usually a weak acid or weak base) of a buffer or buffer solution. A buffering agent helps maintain the pH of a given solution at or near to a pre-determined value, and the buffering agents are generally chosen to complement the pre-determined value. A buffering agent is suitably a single compound which gives rise to a desired buffering effect, especially when said buffering agent is mixed with (and suitably capable of proton exchange with) an appropriate amount (depending on the pre-determined pH desired) of its corresponding "acid/base conjugate", or if the required amount of its corresponding "acid/base conjugate" is formed *in situ -* this may be achieved by adding strong acid or base until the required pH is reached. By way of example:
- An acetate "buffering agent" is suitably an acetate salt, for example, sodium acetate, suitably mixed with its acid/base conjugate, acetic acid. Such a buffer system may be formed by simply mixing a given amount of sodium acetate with a given amount of acetic acid. Alternatively, however, such a buffer may be formed by adding a given amount of a base, suitably a strong base (e.g. sodium hydroxide) to the acetic acid until the desired pH (and thus the desired balance of sodium acetate/acetic acid) is reached. Herein, except where the contrary is stated, any concentrations given in relation to an acetate buffer or acetate buffering agent suitably refer to the combined concentration of the buffering agent(s) (e.g. sodium acetate) and/or acid/base conjugate(s) thereof (e.g. acetic acid).. The skilled person is readily able to calculate such concentrations. Such concentrations may be calculated by reference to the combined concentrations of buffering agent(s) and acid/base conjugate(s), where a buffer system is formed by simply mixing together buffering agent(s) and acid/base conjugate(s). Alternatively, where a buffer system is formed by mixing either the buffering agent(s) or acid/base conjugate(s) with a pH adjuster (e.g. strong acid or strong base) to produce a mixture of each, suitably such concentrations may be calculated by reference to the starting amounts/concentrations of the buffering agent(s) or acid/base conjugate(s) respectively. For example, where a buffer system is formed using a known amount/concentration of acetic acid which is mixed with a pH adjuster (e.g. sodium hydroxide) until the desired pH is reached, the concentration of the buffer system may be calculated by reference to the initial amount of acetic acid.

Herein, an "acid/base conjugate" refers to the conjugate acid or conjugate base (whichever is relevant at a particular pH - typically the conjugate acid in the context of the present invention) of a particular "buffering agent". The acid/base conjugate of an acetate buffering agent (e.g. sodium acetate) is suitably acetic acid.

Herein, the term "buffering species" refers to the particular species (excluding any associated counteranions or countercations - i.e. ignore sodium ions for sodium acetate/acetic acid systems) of a given buffer system which are in dynamic equilibrium with (and proton-exchange with) one another. For example, acetate anions and acetic acid together constitute the "acetate buffering species" of a "acetate buffer system".

Since it is somewhat difficult to define quantities (whether absolute or relative) of a buffer system by reference to weight (since the total weight will depend on the desired pH, which will affect the amount of counterions present), herein weight-based quantities may instead be determined by reference to a theoretical weight of the relevant "buffering species". At least two species are present in any given set of "buffering species" (in relative amounts that can only be determined by reference to the pH), each with a different molecular weight (which usually differs by just 1). Therefore, to enable viable weight calculations and references, for the purposes of this specification the weight of any given set of "buffering species" is given as a theoretical weight based on just one of the buffering species, namely the most acidic of the buffering species (i.e. the most protonated form at any given pH). So the weight of a given set of "buffering species" is quoted as the weight of acid-species equivalents. By way of example, in an acetate buffer system the acetate buffering species may consist of acetate anions (ignore countercations) and acetic acid. The weight of the "buffering species" is therefore calculated as if acetic acid was the only species present in the buffer system (even though acetate is clearly present alongside acetic acid). Thus, any reference to a weight or weight ratio involving a "acetate buffering species" suitably refers to the theoretical weight of acetic acid equivalents within the buffer system. As such, where a composition is formed by adding a pH adjuster (e.g. sodium hydroxide) to a fixed amount of acetic acid, the original weight of acetic acid may be considered to be the weight of the "buffering species" regardless of the ultimate pH. Alternatively, if the concentration (i.e. molarity) of a buffer system is known, this can be converted into a weight of "buffering species" by reference to the molecular weight of the most acidic form of the relevant buffering species (e.g. acetic acid), and ignoring the fact that acetate anions are also present.

Unless stated otherwise, references herein to an "amino acid" or "amino acids", whether specific (e.g. methionine, arginine, histidine) or general (e.g. any amino acid), in the context of their presence or otherwise within compositions (especially topical liquid compositions of the invention) relate to the corresponding free amino acid(s) (regardless of its/their protonation state and/or salt form, all of which may be included by a single reference to "amino acid(s)", whether specific or general, though for consistency amounts are suitably calculated by reference to the free amino acid *per se).* This may suitably include natural and/or artificial amino acids. Unless stated to the contrary, such references are not intended to relate to amino acid residue(s) covalently incorporated as part of a larger compound (as opposed to a composition comprising multiple compounds), such as a peptide or protein (where such amino acid residues are linked *via* peptide bonds). By way of example, a composition defined as being *"free of arginine"* does not contain any free arginine (or free arginine salts, such as arginine hydrochloride) but it may still include one or more proteins (e.g. adalimumab) which do themselves comprise arginine residues. A composition defined as comprising "methionine" contains free methionine (or a salt thereof), regardless of whether or not one or more proteins are present which themselves comprise methionine residues within their overall structure.

Unless stated otherwise, references herein to any one or more "amino acids", whether specific or general, suitably relate to the L- stereoisomers, or at least to a mixture comprising the L-stereoisomers.

The term "substantially free", when used in relation to a given component of a composition (e.g. "a liquid pharmaceutical composition substantially free of arginine"), refers to a composition to which essentially none of said component has been added. As explained above, such references have no bearing on the presence of amino acid residue(s) within a protein structure. When a composition is "substantially free" of a given component, said composition suitably comprises no more than 1 wt% of said component, suitably no more than 0.1 wt% of said component, suitably no more than 0.01 wt% of said component, suitably no more than 0.001 wt% of said component, suitably no more than 0.0001 wt% of said component, suitably no more than 0.00001 wt%, suitably no more than 0.000001 wt%, suitably no more than 0.0000001 wt% thereof, most suitably no more than 0.0001 parts per billion (by weight).

The term "entirely free", when used in relation to a given component of a composition (e.g. "a liquid pharmaceutical composition substantially free of arginine"), refers to a composition containing none of said component. As explained above, such references have no bearing on the presence of amino acid residue(s) within a protein structure.

Herein, in the context of the present specification, a "strong acid" is suitably one having a pKₐ of -1.0 or less, whereas a "weak acid" is suitably one having a pKₐ of 2.0 or more. Herein, in the context of the present specification, a "strong base" is suitably one whose conjugate acid has a pKₐ of 12 or higher (suitably 14 or higher), whereas a "weak base" is suitably one whose conjugate acid has a pKₐ of 10 or less.

Herein, a "stabiliser" refers to a component which facilitates maintainance of the structural integrity of the biopharmaceutical drug, particularly during freezing and/or lyophilization and/or storage (especially when exposed to stress). This stabilising effect may arise for a variety of reasons, though typically such stabilisers may act as osmolytes which mitigate against protein denaturation. Typical stabilisers include amino acids (i.e. free amino acids not part of a peptide or protein - e.g. glycine, arginine, histidine, aspartic acid, lysine) and sugar stabilisers, such as a sugar polyol (e.g. mannitol, sorbitol), and/or a disaccharide (e.g. trehalose, sucrose, maltose, lactose), though the liquid pharmaceutical compositions of the invention include a stabiliser, at least one of which is a sugar stabiliser (i.e. either a sugar polyol or a disaccharide). Most suitably the at least one sugar stabiliser is a non-reducing sugar (be it a sugar polyol or a disaccharide).

Herein, a "non-reducing sugar" is generally a sugar without any aldehyde moieties or without the capability of forming an aldehyde moiety (e.g. through isomerism or tautomerism).

Herein, a "tonicity modifier" or "tonicifier" refers to a reagent whose inclusion within a composition suitably contributes to (or increases) the overall osmolality and osmolarity of the composition. Suitably, a tonicifier, as used herein includes an agent which functions to render a solution similar in osmotic characteristics to physiologic fluids.

Herein, references to specific amounts of a given component of a composition, especially a buffering agent, stabiliser, amino acid, surfactant, or tonicifier, suitably relate to the amounts of the pure anhydrous form of the relevant component (or compositions formed by using said amounts of the pure anhydrous form), even though such a component may be used in a non-anhydrous form when forming the composition. Amounts of any corresponding non-anhydrous forms (e.g. monohydrates, dihydrates, etc.) may be readily calculated by simply using the appropriate multiplier. For instance, unless stated otherwise (as per the Examples, where quantities relate to trehalose dihydrate), amounts stipulated in relation to trehalose refer to the anhydrous form of trehalose (or compositions formed by using the stipulated amounts/concentrations of anhydrous trehalose), which has a molecular weight of 342.296 g/mol, so to calculate the corresponding amount of trehalose dihydrate needed to form the same composition (less water would have to be added) it is necessary to multiply the stipulated amount by 378.33/342.296, since 378.33 is the molecular weight of trehalose dihydrate. The skilled person would readily understand how to judiciously adjust the quantity of diluent/water depending on the form of the components used, in order to derive the target concentrations.

Herein, the term "pharmaceutical composition" refers to a formulation of a pharmaceutical active which renders the biological activity of the active ingredient therapeutically effective, but which does not include other ingredients which are obviously toxic to a subject to which the formulation are intended to be administered.

Herein, the term "stable" generally refers to the physical stability and/or chemical stability and/or biological stability of a component, typically an active or composition thereof, during preservation/storage.

It is to be appreciated that references to "treating" or "treatment" include prophylaxis as well as the alleviation of established symptoms of a condition. "Treating" or "treatment" of a state, disorder or condition therefore includes: (1) preventing or delaying the appearance of clinical symptoms of the state, disorder or condition developing in a human that may be afflicted with or predisposed to the state, disorder or condition but does not yet experience or display clinical or subclinical symptoms of the state, disorder or condition, (2) inhibiting the state, disorder or condition, *i.e.,* arresting, reducing or delaying the development of the disease or a relapse thereof (in case of maintenance treatment) or at least one clinical or subclinical symptom thereof, or (3) relieving or attenuating the disease, *i.e*., causing regression of the state, disorder or condition or at least one of its clinical or subclinical symptoms.

In the context of the present invention, a "therapeutically effective amount" or "effective amount" of the antibody means an amount that is effective, when administered to a mammal for treating a disease or disorder, in prophylactic and therapeutic aspect and the antibody is effective in treatment of the diseases concerned.

The "therapeutically effective amount" will vary depending on the compound, the disease and its severity and the age, weight, etc., of the mammal to be treated.

The term "human TNF-α" refers to the human cytokine which exists in a 17kD secreted form and a 26kD membrane-associated form, and in a biologically active form, TNF-α could be observed as a trimer of covalently-bound 17kD molecule. Its specific structure can be found in Pennica, D. et al. (1984) Nature 312: 724-729; Davis, J. M. et al. (1987) Biochemistry 26, 1322-1326; and Jones, E. Y. et al. (1989) Nature 338: 225-228.

The term "recombinant human antibody" is intended to include a human antibody prepared, expressed, produced or isolated using a recombinant method.

Herein, amounts stipulated for components and ingredients, whether specified in terms of "parts", ppm (parts per million), percentages (%, e.g. wt%), or ratios, are intended to be by weight, unless stated otherwise.

Where the quantity or concentration of a particular component of a given composition is specified as a weight percentage (wt% or %w/w), said weight percentage refers to the percentage of said component by weight relative to the total weight of the composition as a whole. It will be understood by those skilled in the art that the sum of weight percentages of all components of a composition (whether or not specified) will total 100 wt%. However, where not all components are listed (e.g. where compositions are said to "comprise" one or more particular components), the weight percentage balance may optionally be made up to 100 wt% by unspecified ingredients (e.g. a diluent, such as water, or other non-essentially but suitable additives).

Herein, unless stated otherwise, the term "parts" (e.g. parts by weight, pbw) when used in relation to multiple ingredients/components, refers to relative ratios between said multiple ingredients/components. Expressing molar or weight ratios of two, three or more components gives rise to the same effect (e.g. a molar ratio of x, y, and z is *x₁ : y₁ : z₁* respectively, or a range *x₁-x₂ : y₁-y₂ : z₁-z₂*). Though in many embodiments the amounts of individual components within a composition may be given as a "wt%" value, in alternative embodiments any or all such wt% values may be converted to parts by weight (or relative ratios) to define a multi-component composition. This is so because the relative ratios between components is often more important than the absolute concentrations thereof in the liquid pharmaceutical compositions of the invention. Where a composition comprising multiple ingredients is described in terms of parts by weight alone (i.e. to indicate only relative ratios of ingredients), it is not necessary to stipulate the absolute amounts or concentrations of said ingredients (whether *in toto* or individually) because the advantages of the invention can stem from the relative ratios of the respective ingredients rather than their absolute quantities or concentrations. However, in certain embodiments, such compositions consists essentially of or consist of the stipulated ingredients and a diluents (e.g. water).

Where a composition is said to comprise a plurality of stipulated ingredients (optionally in stipulated amounts of concentrations), said composition may optionally include additional ingredients other than those stipulated. However, in certain embodiments, a composition said to comprise a plurality of stipulated ingredients may in fact consist essentially of or consist of all the stipulated ingredients.

Herein, where a composition is said to "consists essentially of" a particular component, said composition suitably comprises at least 85 wt% of said component, suitably at least 90 wt% thereof, suitably at least 95 wt% thereof, most suitably at least 99 wt% thereof. Suitably, a composition said to "consist essentially of" a particular component consists of said component save for one or more trace impurities.

Herein, the term "particle size" or "pore size" refers respectively to the length of the longest dimension of a given particle or pore. Both sizes may be measured using a laser particle size analyser and/or electron microscopes (e.g. tunneling electron microscope, TEM, or scanning electron microscope, SEM). The particle count (for any given size) can be obtained using the protocols and equipment outlined in the Examples, which relates to the particle count of sub-visible particles.

In this specification the term "alkyl" includes both straight and branched chain alkyl groups. References to individual alkyl groups such as "propyl" are specific for the straight chain version only and references to individual branched chain alkyl groups such as "isopropyl" are specific for the branched chain version only. For example, "(1-6C)alkyl" includes (1-4C)alkyl, (1-3C)alkyl, propyl, isopropyl and t-butyl. A similar convention applies to other radicals, for example "phenyl(1-6C)alkyl" includes phenyl(1-4C)alkyl, benzyl, 1-phenylethyl and 2-phenylethyl.

The term "(m-nC)" or "(m-nC) group" used alone or as a prefix, refers to any group having m to n carbon atoms.

"(3-8C)cycloalkyl" means a hydrocarbon ring containing from 3 to 8 carbon atoms, for example, cyclopropyl, cyclobutyl, cyclopentyl, cyclohexyl, cycloheptyl or bicyclo[2.2.1]heptyl.

"(3-8C)cycloalkenyl" means a hydrocarbon ring containing at least one double bond, for example, cyclobutenyl, cyclopentenyl, cyclohexenyl or cycloheptenyl, such as 3-cyclohexen-1-yl, or cyclooctenyl.

The term "halo" refers to fluoro, chloro, bromo and iodo.

The term "heterocyclyl", "heterocyclic" or "heterocycle" means a non-aromatic saturated or partially saturated monocyclic, fused, bridged, or spiro bicyclic heterocyclic ring system(s). The term heterocyclyl includes both monovalent species and divalent species. Monocyclic heterocyclic rings contain from about 3 to 12 (suitably from 3 to 7) ring atoms, with from 1 to 5 (suitably 1, 2 or 3) heteroatoms selected from nitrogen, oxygen or sulfur in the ring. Bicyclic heterocycles contain from 7 to 17 member atoms, suitably 7 to 12 member atoms, in the ring. Bicyclic heterocycles contain from about 7 to about 17 ring atoms, suitably from 7 to 12 ring atoms. Bicyclic heterocyclic(s) rings may be fused, spiro, or bridged ring systems. Examples of heterocyclic groups include cyclic ethers such as oxiranyl, oxetanyl, tetrahydrofuranyl, dioxanyl, and substituted cyclic ethers. Heterocycles containing nitrogen include, for example, azetidinyl, pyrrolidinyl, piperidinyl, piperazinyl, tetrahydrotriazinyl, tetrahydropyrazolyl, and the like. Typical sulfur containing heterocycles include tetrahydrothienyl, dihydro-1,3-dithiol, tetrahydro-2H-thiopyran, and hexahydrothiepine. Other heterocycles include dihydro-oxathiolyl, tetrahydro-oxazolyl, tetrahydro-oxadiazolyl, tetrahydrodioxazolyl, tetrahydro-oxathiazolyl, hexahydrotriazinyl, tetrahydro-oxazinyl, morpholinyl, thiomorpholinyl, tetrahydropyrimidinyl, dioxolinyl, octahydrobenzofuranyl, octahydrobenzimidazolyl, and octahydrobenzothiazolyl. For heterocycles containing sulfur, the oxidized sulfur heterocycles containing SO or SO2 groups are also included. Examples include the sulfoxide and sulfone forms of tetrahydrothienyl and thiomorpholinyl such as tetrahydrothiene 1,1-dioxide and thiomorpholinyl 1,1-dioxide. A suitable value for a heterocyclyl group which bears 1 or 2 oxo (=O) or thioxo (=S) substituents is, for example, 2-oxopyrrolidinyl, 2-thioxopyrrolidinyl, 2-oxoimidazolidinyl, 2-thioxoimidazolidinyl, 2-oxopiperidinyl, 2,5-dioxopyrrolidinyl, 2,5-dioxoimidazolidinyl or 2,6-dioxopiperidinyl. Particular heterocyclyl groups are saturated monocyclic 3 to 7 membered heterocyclyls containing 1, 2 or 3 heteroatoms selected from nitrogen, oxygen or sulfur, for example azetidinyl, tetrahydrofuranyl, tetrahydropyranyl, pyrrolidinyl, morpholinyl, tetrahydrothienyl, tetrahydrothienyl 1,1-dioxide, thiomorpholinyl, thiomorpholinyl 1,1-dioxide, piperidinyl, homopiperidinyl, piperazinyl or homopiperazinyl. As the skilled person would appreciate, any heterocycle may be linked to another group via any suitable atom, such as via a carbon or nitrogen atom. However, reference herein to piperidino or morpholino refers to a piperidin-1-yl or morpholin-4-yl ring that is linked via the ring nitrogen.

By "bridged ring systems" is meant ring systems in which two rings share more than two atoms, see for example Advanced Organic Chemistry, by Jerry March, 4th Edition, Wiley Interscience, pages 131-133, 1992. Examples of bridged heterocyclyl ring systems include, aza-bicyclo[2.2.1]heptane, 2-oxa-5-azabicyclo[2.2.1]heptane, aza-bicyclo[2.2.2]octane, aza-bicyclo[3.2.1]octane and quinuclidine.

"Heterocyclyl(1-6C)alkyl" means a heterocyclyl group covalently attached to a (1-6C)alkylene group, both of which are defined herein.

The term "heteroaryl" or "heteroaromatic" means an aromatic mono-, bi-, or polycyclic ring incorporating one or more (for example 1-4, particularly 1, 2 or 3) heteroatoms selected from nitrogen, oxygen or sulfur. The term heteroaryl includes both monovalent species and divalent species. Examples of heteroaryl groups are monocyclic and bicyclic groups containing from five to twelve ring members, and more usually from five to ten ring members. The heteroaryl group can be, for example, a 5- or 6-membered monocyclic ring or a 9- or 10-membered bicyclic ring, for example a bicyclic structure formed from fused five and six membered rings or two fused six membered rings. Each ring may contain up to about four heteroatoms typically selected from nitrogen, sulfur and oxygen. Typically the heteroaryl ring will contain up to 3 heteroatoms, more usually up to 2, for example a single heteroatom. In one embodiment, the heteroaryl ring contains at least one ring nitrogen atom. The nitrogen atoms in the heteroaryl rings can be basic, as in the case of an imidazole or pyridine, or essentially non-basic as in the case of an indole or pyrrole nitrogen. In general the number of basic nitrogen atoms present in the heteroaryl group, including any amino group substituents of the ring, will be less than five.

"Heteroaryl(1-6C)alkyl" means a heteroaryl group covalently attached to a (1-6C)alkylene group, both of which are defined herein. Examples of heteroaralkyl groups include pyridin-3-ylmethyl, 3-(benzofuran-2-yl)propyl, and the like.

The term "aryl" means a cyclic or polycyclic aromatic ring having from 5 to 12 carbon atoms. The term aryl includes both monovalent species and divalent species. Examples of aryl groups include, but are not limited to, phenyl, biphenyl, naphthyl and the like. In particular embodiment, an aryl is phenyl.

The term "aryl(1-6C)alkyl" means an aryl group covalently attached to a (1-6C)alkylene group, both of which are defined herein. Examples of aryl-(1-6C)alkyl groups include benzyl, phenylethyl, and the like

This specification also makes use of several composite terms to describe groups comprising more than one functionality. Such terms will be understood by a person skilled in the art. For example heterocyclyl(m-nC)alkyl comprises (m-nC)alkyl substituted by heterocyclyl.

Wherever groups with large carbon chains are disclosed (e.g. (1-12C)alkyl, (1-8C)alkenyl, etc.), such groups may optionally be shortened, for instance containing a between 1 and 5 carbons (e.g. (1-5C)alkyl or (1-5C)alkenyl), or contain between 1 and 3 carbons (e.g. (1-3C)alkyl or (1-3C)alkenyl instead of (1-12C)alkyl or (1-8C)alkenyl).

The term "optionally substituted" refers to either groups, structures, or molecules that are substituted and those that are not substituted.

Where optional substituents are chosen from "one or more" groups it is to be understood that this definition includes all substituents being chosen from one of the specified groups or the substituents being chosen from two or more of the specified groups.

Herein, unless stated otherwise, all chemical nomenclature may be defined in accordance with IUPAC definitions.

Suitably any excipients (e.g. buffer, stabilizer, tonicifier, surfactant, etc.) defined herein, especially where they are defined as being present within a pharmaceutical composition of the invention, are pharmaceutically acceptable excipients.

### Liquid Pharmaceutical Composition

The present invention provides a liquid pharmaceutical composition, suitably as defined herein. The composition suitably comprises a human monoclonal antibody, suitably one which inhibits human TNF-α activity, suitably so as to prevent it from activating TNF receptors. Most suitably the liquid pharmaceutical composition comprises adalimumab, which in itself suitably includes any biosimilar thereof.

The composition suitably comprises a buffer system (or buffering agent), suitably an acetate buffer system (or an acetate buffering agent).

The composition suitably comprises a sugar stabiliser. The composition suitably comprises inositol.

The composition suitably comprises a surfactant. Suitably the surfactant is a surfactant other than polysorbate 80 where the composition comprises trehalose as a sugar stabiliser. The composition suitably comprises a PVP surfactant.

The composition suitably comprises a tonicifier. The composition suitably comprises a carboxylate salt tonicifier, suitably a metal carboxylate salt tonicifier, suitably a metal gluconate salt tonicifier, suitably sodium gluconate.

The composition is suitably (substantially or entirely) free of arginine or comprises arginine either in a concentration of at most 0.1 mM, in a molar ratio of arginine to acetate buffering agent (or a acetate buffer system) of at most 1 : 150, or in a weight ratio of arginine to adalimumab of at most 1 : 3000 (i.e. less than or equal to one part by weight of arginine for every 3000 parts by weight acetate buffering agent). Alternatively or in addition, the composition may suitably include any one or more additional components defined herein in relation to a liquid pharmaceutical composition (e.g. including surfactant, excluding arginine, etc.), optionally in any amount, concentration, or form stipulated herein; and wherein the composition optionally exhibits any one or more parameters or properties given herein in relation to a liquid pharmaceutical composition (e.g. pH, osmolality).

Recent research by the inventors suggests that the liquid pharmaceutical compositions of the invention represent clinically viable and potentially improved alternatives to the corresponding liquid formulations of the prior art, for example, the commercially available formulation Humira®. In particular, recent experiments performed and/or commissioned by the inventors suggest that the liquid pharmaceutical compositions of the invention will withstand a significant amount of stress (e.g. thermal, mechanical and light stress). As such, these compositions are expected to exhibit good stability (thereby retaining their clinical viability) under the kind of manufacture, transport, and storage conditions pharmaceutical products are typically exposed to during their lifetime. That such good stability performance can be achieved using less complex formulations with fewer excipients (which can in turn reduce the burden of processing, analysis, etc.) was considered surprising in view of the general teachings of the prior art, and potentially offers pharmaceutical products with reduced side effects.

### Adalimumab

Adalimumab, which is commercially available in HUMIRA® formulations, and its method of manufacture, is described in WO97/29131 (BASF) as D2E7, and elsewhere in the art. It is described as having "a light chain CDR3 domain comprising the amino acid sequence of SEQ ID NO: 3 and a heavy chain CDR3 domain comprising the amino acid sequence of SEQ ID NO: 4" (WO97/29131). Furthermore, the D2E7 antibody is described as having a light chain variable region (LCVR) comprising the amino acid sequence of SEQ ID NO: 1 and a heavy chain variable region (HCVR) comprising the amino acid sequence of SEQ ID NO: 2 (WO97/29131).

The medical indications and function of Adalimumab, are elucidated hereinbefore.

In the context of the invention "adalimumab" includes biosimilars, as defined herein before, and the skilled person would readily appreciate the scope of the term "adalimumab" in the context of the invention.

In an embodiment, the liquid pharmaceutical composition comprises adalimumab at a concentration of from about 5 to about 150 mg/ml, suitably from about 25 to about 75 mg/mL. For example, the adalimumab may be present in the formulation at a concentration of about 25, about 30, about 35, about 40, about 45, about 50, about 55, about 60, about 65, about 70 or about 75 mg/ml. In an embodiment, the adalimumab is present at a concentration from about 45 to about 55 mg/ml. In an embodiment, the adalimumab is present at a concentration of about 50 mg/ml.

### Buffer System, Buffering Agent, and pH

Suitably, the liquid pharmaceutical composition is a buffered solution whose pH is stabilised by a buffering agent (or a buffer system), suitably in combination with an acid/base conjugate of the buffering agent. As such, the liquid pharmaceutical composition suitably comprises a buffering agent (or buffer system) as defined herein. Preferably, the liquid pharmaceutical composition additionally comprises an acid/base conjugate, wherein said acid/base conjugate corresponds to the conjugate acid or conjugate base of the buffering agent, depending on whether the buffering agent is itself a base or acid respectively. Collectively, the buffering agent and its acid/base conjugate may be considered a "buffer system". The liquid pharmaceutical composition thus suitably comprises a "buffer system" (suitably comprising a buffering agent(s) and an acid/base conjugate(s) thereof), and any concentrations stipulated in relation to the buffer system generally relate to the combined concentrations of the buffering agent(s) and any acid/base conjugate(s) thereof. Any "buffer system" suitably comprises a weak acid and a weak base (see above definitions).

Suitably, the buffering agent is an acetate buffering agent. Suitably the acetate buffering agent is an acetate salt, suitably comprising anionic acetate (i.e. AcO⁻) and one or more pharmaceutically acceptable countercations. A suitable acetate salt may include a metal acetate salt (e.g. an alkali metal acetate or an alkaline earth metal acetate), or a non-meteal acetate salt (e.g. ammonium acetate, triethylammonium acetate). In a particular embodiment, the buffering agent (and the acetate salt) is sodium acetate.

Suitably, the liquid pharmaceutical composition comprises an acid/base conjugate of the buffering agent, most suitably acetic acid as the conjugate acid of an acetate salt. The combination of the buffering agent and its acid/base conjugate constitute a buffer system. Suitably, the liquid pharmaceutical composition comprises the buffering agent and its corresponding acid/base conjugate, suitably such that together the buffering agent and its acid/base conjugate are present at a level (i.e. absolute amount or concentration) and in a relative amount (or concentration) sufficient to provide the desired pH for the composition. The buffer system may be formed by simply mixing the buffering agent with its acid/base conjugate or may alternatively be formed by mixing an acid or base with either the buffering agent or its acid/base conjugate in order to form *in situ* the desired mixture of buffering agent and acid/base conjugate. For example, the buffer system may be formed by simply mixing the acetate buffering agent (e.g. sodium acetate) with its acid/base conjugate (i.e. acetic acid), suitably in a ratio appropriate to furnish the desired pH. Alternatively, the buffer system may be formed by adding a base (e.g. sodium hydroxide) to the acid/base conjugate (i.e. acetic acid) of the acetate buffering agent, suitably in an amount appropriate to furnish the desired pH and mixture of the buffering agent (e.g. sodium acetate) and corresponding acid/base conjugate (i.e. acetic acid). Alternatively, either method of forming the buffer system may be employed, and pH may be judiciously adjusted by either adding further acid (suitably strong acid, such as HCl) or further base (suitably strong base, such as sodium hydroxide).

Most suitably, the buffer system is an acetate buffer system, suitably comprising an acetate salt and acetic acid.

Suitably, the liquid pharmaceutical composition comprises at most one buffering agent. Suitably, the liquid pharmaceutical composition comprises at most one buffer system.

Suitably, the liquid pharmaceutical composition has a pH greater than or equal to 5.0. Suitably, the liquid pharmaceutical composition has a pH less than or equal to 6.7.

In a particular embodiment, especially where the buffering agent is an acetate buffering agent (or buffer system is an acetate buffer system), the liquid pharmaceutical composition has a pH between 5.0 and 5.5. In a particular embodiment, the liquid pharmaceutical composition has a pH between 5.1 and 5.3. In a particular embodiment, the liquid pharmaceutical composition has a pH of about 5.2.

Suitably, the liquid pharmaceutical composition comprises a buffer system (suitably an acetate buffer system, suitably comprising an acetate buffering agent) at a concentration of from about 2 to about 50 mM. In an embodiment, the buffer system is present at a concentration of from about 3 to about 40 mM. In an embodiment, the buffer system is present at a concentration of between 5 and 14 mM, most suitably about 10 mM. In an embodiment, the buffer system is present at a concentration of 10 mM. In an embodiment, the liquid pharmaceutical composition comprises a sodium acetate/acetic acid buffer system at a concentration of 10 mM. This includes where the "buffering agent(s)" (e.g. sodium acetate) is formed by the addition of a strong base (e.g. sodium hydroxide) to the conjugate acid of the buffering agent(s) (e.g. acetic acid).

Suitably, the liquid pharmaceutical composition comprises the buffering species (suitably acetate buffering species) at a concentration of from about 0.120 mg/mL to about 3.0 mg/mL. In an embodiment, the buffering species are present at a concentration of between 0.30 mg/mL and 0.84 mg/mL, most suitably about 0.60 mg/mL. This includes where the "buffering agent" (e.g. sodium acetate) is formed by the addition of a strong base (e.g. sodium hydroxide) to the conjugate acid of the buffering agent (e.g. acetic acid).

Suitably, the liquid pharmaceutical composition comprises the buffer system (suitably the acetate buffer system) in a molar ratio of buffer system to adalimumab of from about 5:1 to about 145:1. In an embodiment, the buffer system is present in a molar ratio of buffer system to adalimumab of from about 14:1 to about 40:1, most suitably about 29:1. In an embodiment, the buffer system is present at a concentration of 29:1. This includes where the "buffering agent(s)" (e.g. sodium acetate) is formed by the addition of a strong base (e.g. sodium hydroxide) to the conjugate acid of the buffering agent (e.g. acetic acid).

The inventors' research suggests that the liquid pharmaceutical compositions of the invention, which suitably include a buffer system, will perform particularly well under stressing conditions, especially in relation to fragmentation and protein unfolding, which can be important indicators of stability and drug product viability. Furthermore, liquid pharmaceutical compositions whose acetate buffer system maintains a steady pH 5.2 are thought to be especially suitable.

### Sugar Stabiliser

Suitably, the liquid pharmaceutical composition comprises a stabiliser, most suitably a sugar stabiliser. Suitably, such a component facilitates maintainance of the structural integrity of the biopharmaceutical drug, particularly during freezing and/or lyophilization and/or storage (especially when exposed to stress).

The liquid pharmaceutical composition may comprise one or more sugar stabilisers, though in preferred embodiments only a single sugar stabiliser is present.

Suitably, the sugar stabiliser is a sugar (i.e. a saccharide, for example, a monosaccharide or disaccharide) or a sugar polyol (i.e. sugar alcohol, for example, a polyhydric alcohol). A sugar polyol is suitably distinct from a sugar *per se* in that a sugar polyol is typically a reduced form of a sugar *per se.* The core structure of a sugar polyol is suitably free of (or incapable of adopting a molecular structure comprising) any anomeric carbon centres and/or glycosidic linkages.

Suitably, the sugar stabiliser is a sugar polyol (including sugar alcohols) and/or a disaccharide.

The sugar stabiliser is suitably selected from the group including trehalose, mannitol, sucrose, sorbitol, maltose, lactose, xylitol, arabitol, erythritol, lactitol, maltitol, inositol. More suitably, the sugar stabiliser is selected from the group including trehalose, mannitol, sucrose, maltose, lactose, xylitol, arabitol, erythritol, lactitol, maltitol, inositol. More suitably, the sugar stabiliser is selected from the group including trehalose, maltose, lactose, xylitol, arabitol, erythritol, lactitol, maltitol, inositol. Most suitably, the sugar stabiliser is selected from the group consisting of trehalose and inositol.

In a particular embodiment, the sugar stabiliser is a non-reducing sugar, optionally a non-reducing sugar listed anywhere herein. In a particular embodiment, the sugar stabiliser is trehalose.

In a particular embodiment, the sugar stabiliser is a sugar polyol, optionally a sugar polyol listed anywhere herein.

In a particular embodiment, the sugar stabiliser is a sugar or sugar polyol, on the proviso that the sugar stabiliser is not trehalose. In a particular embodiment, the sugar stabiliser is a sugar or sugar polyol, on the proviso that the sugar stabiliser is neither trehalose, mannitol, nor sorbitol. In a particular embodiment, the sugar stabiliser is a sugar or sugar polyol, on the proviso that the sugar stabiliser is neither trehalose, sucrose, mannitol, nor sorbitol.

In a particular embodiment, the sugar stabiliser is selected from the group including:
- a sugar comprising (or capable of adopting a molecular structure comprising) a single sugar ring (e.g. a single furanose and/or pyranose ring) with no further sugar rings (e.g. not sucrose, trehalose, lactose, etc.);
- a sugar polyol on the proviso that the sugar polyol is not a C₆-aliphatic sugar polyol (e.g. not mannitol, sorbitol, etc.)

In a particular embodiment, the sugar stabiliser is (or is capable of adopting a molecular structure charactarised as) a monocyclic sugar stabiliser. In a particular embodiment, the sugar stabiliser is (or is capable of adopting a molecular structure charactarised as) a monocyclic sugar or monocyclic sugar polyol - this is suitably a sugar or sugar polyol comprising only a single ring system (i.e. with no other ring systems as part of the molecule, e.g. not bicyclic or otherwise polycyclic, whether fused, non-fused, spiro, etc.). Suitably, the sugar stabiliser is (or is capable of adopting a molecular structure charactarised as) a monocyclic sugar or monocyclic sugar polyol comprising (or capable of adopting a molecular structure comprising) a single ring system (e.g. a single furanose, pyranose, or cycloalkyl ring system, e.g. cyclohexyl) with no further rings (e.g. not sucrose, trehalose, lactose, etc.). The monocyclic sugar stabiliser may, therefore, in addition to inositol and other sugars having a single ring system (such as glucose, fructose, and ribose), also include monocyclic sugars or monocyclic sugar polyols that are (covalently) attached (e.g. *via* an alcohol group or *via* an anomeric position of the monocyclic sugar stabiliser) to an acyclic sugar or sugar polyol which, for example, may therefore include compounds such as lactitol and maltitol. In some embodiments, the monocyclic sugar stabiliser, however, may exclude such monocyclic sugar stabilisers with attached acyclic sugars or sugar polyols.

In a particular embodiment, the sugar stabiliser is inositol. Inositol is a monocyclic sugar polyol comprising a cyclohexyl ring system. In a particular embodiment, the sugar stabiliser is *cis*-1,2,3,5-*trans*-4,6-cyclohexanehexol (also known as *myo*-inositol, meso-inositol or i-inositol). Inositol is thought to be a particularly advantageous sugar stabiliser for use in liquid adalimumab formulations.

Suitably, the liquid pharmaceutical composition comprises at most one sugar stabiliser, suitably at most one of either a sugar polyol and/or a disaccharide (especially non-reducing disaccharide). Suitably, the liquid pharmaceutical composition comprises either trehalose or inositol as the only sugar stabiliser.

Suitably the trehalose used to form the liquid pharmaceutical composition is trehalose dihydrate, though suitably any amounts stipulated in relation to trehalose pertain to pure, anhydrous trehalose. Such amounts may be converted into an amount of trehalose dihydrate by applying an appropriate multiplier. Moreover, for the purposes of assessing whether a given formulation falls within the scope of any of the trehalose quantity definitions given herein, an amount of trehalose dihydrate can be readily converted into a corresponding amount of pure, anhydrous trehalose (with an equal number of moles) through applying said multiplier in reverse. This principle may be adopted for any sugar stabiliser component (including, for example, inositol). Concentrations, when given as a molar concentration, will of course be the same regardless of the hydration state of the sugar stabiliser.

Suitably, the liquid pharmaceutical composition comprises the sugar stabilizer(s) (most suitably trehalose or inositol) at a concentration of from about 10 to about 400 mM, more suitably from about 50 to about 300 mM, more suitably from about 100 to about 250 mM. In a particular embodiment, the sugar stabilizer is suitably present at a concentration of between 190 and 210 mM, most suitably about 200 mM.

Where the sugar stabiliser is trehalose, the sugar stabilizer is suitably present at a concentration of between 190 and 210 mM, most suitably about 200 mM.

Where the sugar stabiliser is inositol, the sugar stabilizer is suitably present at a concentration of from about 25 to about 275 mM, suitably from about 25 to about 250 mM, more suitably from about 55 to about 225 mM. Suitably inositol may be present at a concentration of from about 25 to about 150 mM, suitably about 55 to 100 mM.

Suitably, the liquid pharmaceutical composition comprises the sugar stabilizer(s) (especially where it is inositol) at a concentration of from about 5 mg/mL to about 45 mg/mL, from about 8 mg/mL to about 75 mg/mL, suitably from about 10 mg/mL to 40 mg/mL, more suitably from about 18 mg/mL to about 55 mg/mL, more suitably from about 22 mg/mL to about 42 mg/mL. In a particular embodiment, the sugar stabiliser (especially where it is inositol) is present at a concentration of between 5 mg/mL and 25 mg/mL, suitably between 10 mg/mL and 18 mg/mL. In a particular embodiment, the sugar stabiliser (especially where it is inositol) is present at a concentration between 30 and 40 mg/mL.

Suitably, the liquid pharmaceutical composition comprises the sugar stabilizer(s) (especially where it is trehalose) at a concentration of from about 15 mg/mL to about 140 mg/mL, more suitably from about 35 mg/mL to about 100 mg/mL, more suitably from about 45 mg/mL to about 80 mg/mL. In an embodiment, the sugar stabilizer(s) (especially where it is trehalose) is present at a concentration of between 65 mg/mL and 72 mg/mL, most suitably about 68 mg/mL. In a particular embodiment, trehalose is present at a concentration of about 68 mg/mL (which equates to about 75.7 mg/mL trehalose dihydrate).

Suitably, the liquid pharmaceutical composition comprises the sugar stabilizer(s) (especially where it is trehalose) in a molar ratio of sugar stabilizer(s) to adalimumab of from about 145:1 to about 1150:1, more suitably from about 290:1 to about 860:1, more suitably from about 430:1 to about 720:1. In an embodiment, the sugar stabilizer(s) (especially where it is trehalose) is present at a molar ratio of sugar stabilizer(s) to adalimumab of from about 550:1 to about 605:1, most suitably about 576:1. In an embodiment, trehalose is present at a molar ratio of trehalose to adalimumab of about 576:1.

Suitably, the liquid pharmaceutical composition comprises the sugar stabilizer(s) (especially where it is inositol) in a molar ratio of sugar stabilizer(s) to adalimumab of from about 35:1 to about 1150:1, more suitably from about 70:1 to about 860:1, more suitably from about 140:1 to about 720:1. In an embodiment, the sugar stabilizer(s) (especially where it is inositol) is present at a molar ratio of sugar stabilizer(s) to adalimumab of from about 275:1 to about 300:1.

The sugar stabiliser(s) is suitably present at a concentration in which it is soluble within the liquid pharmaceutical composition.

The liquid pharmaceutical compositions of the invention including a sugar stabiliser as defined herein are thought to be especially suitable to stabilise the adalimumab within the formulation, especially with respect to aggregation, fragmentation and protein unfolding, which can be important indicators of stability and drug product viability.

### Surfactant

The liquid pharmaceutical composition of the invention suitably comprises a surfactant or one or more surfactants, suitably as defined herein.

The inclusion of a surfactant suitably contributes to stabilisation of the adalimumab protein.

Any suitable surfactant may be used. However, suitably the surfactant is a non-ionic surfactant, most suitably selected from the group consisting of a polyvinylpyrrolidone (PVP) surfactant, a polysorbate surfactant (polyoxyethylene glycol sorbitan alkyl esters), and a span surfactant (sorbitan alkyl esters). Suitably, however, the surfactant is a surfactant other than polysorbate 80 where trehalose is included in the composition as a sugar stabiliser. A "PVP surfactant" is suitably PVP, suitably PVP with a weight average molecular weight (M_{w}) suitable for imparting surfactant properties.

The surfactant(s) are suitably selected from a polyvinylpyrrolidone (PVP) surfactant (suitably PVP having an M_{w} of between 1900 and 3100), Polysorbate 20 (Polyoxyethylene (20) sorbitan monolaurate), Polysorbate 40 (Polyoxyethylene (20) sorbitan monopalmitate), Polysorbate 60 (Polyoxyethylene (20) sorbitan monostearate), Polysorbate 80 (Polyoxyethylene (20) sorbitan monooleate), Sorbitan monolaurate, Sorbitan monopalmitate, Sorbitan monostearate, Sorbitan tristearate, and/or Sorbitan monooleate.

In a particular embodiment, the surfactant(s) are selected from a polyvinylpyrrolidone (PVP) surfactant (suitably PVP having an M_{w} of between 1900 and 3100), Polysorbate 20, Polysorbate 40, Polysorbate 60, and/or Polysorbate 80. In a particular embodiment, the liquid pharmaceutical composition comprises a single surfactant selected from a polyvinylpyrrolidone (PVP) surfactant (suitably PVP having an M_{w} of between 1900 and 3100), Polysorbate 20, Polysorbate 40, Polysorbate 60, and Polysorbate 80.

In a particular embodiment, the surfactant is a polyvinylpyrrolidone (PVP) surfactant. Suitably the PVP surfactant has a weight average molecular weight M_{w} of between 1000 and 20000, more suitably between 1500 and 12000, more suitably between 1900 and 3100, most suitably between 2000 and 3000. In a particular embodiment, the surfactant is PVP with a M_{w} between 2000 and 3000 (e.g. Kollidon® 12 PF).

In a particular embodiment, the surfactant is polysorbate 80 or polysorbate 20. In a particular embodiment, the surfactant is polysorbate 80.

Suitably, the liquid pharmaceutical composition comprises the surfactant(s) (most suitably PVP or polysorbate 80) at a concentration of from about 0.0001 to about 5 mM (i.e. 0.1µM-5mM), more suitably from about 0.001 to about 2 mM, more suitably from about 0.01 to about 1.0 mM. In an embodiment, the surfactant(s) (especially where it is polysorbate 80) is present at a concentration of between 0.72 and 0.80 mM, most suitably about 0.76 mM. In an embodiment, polysorbate 80 is present at a concentration of 0.76 mM. In an embodiment, the surfactant(s) (especially where it is PVP) is present at a concentration of between 0.01 and 0.50 mM.

Suitably, the liquid pharmaceutical composition comprises the surfactant(s) (most suitably PVP or polysorbate 80) at a concentration of from about 0.001 mg/mL to about 5 mg/mL, more suitably from about 0.01 mg/mL to about 2 mg/mL, more suitably from about 0.05 mg/mL to about 1.5 mg/mL. In an embodiment, the surfactant(s) (especially where it is polysorbate 80) is present at a concentration of between 0.9 mg/mL and 1.1 mg/mL, most suitably about 1.0 mg/mL. In an embodiment, the surfactant(s) (especially where it is PVP) is present at a concentration of between 0.01 mg/mL and 1 mg/mL.

Suitably, the liquid pharmaceutical composition comprises the surfactant(s) (most suitably PVP or polysorbate 80) in a molar ratio of surfactant(s) to adalimumab of from about 1:3500 to about 15:1, more suitably from about 1:350 to about 6:1, more suitably from about 1:35 to about 3:1. In an embodiment, the surfactant(s) (especially where it is polysorbate 80) is present at a molar ratio of surfactant(s) to adalimumab of from about 2.1:1 to about 2.3:1, most suitably about 2.2:1. In an embodiment, the surfactant(s) (especially where it is PVP) is present at a molar ratio of surfactant(s) to adalimumab of from about 0.1:1 to about 2:1.

Though one or more surfactants may be included within the liquid pharmaceutical composition of the invention, most suitably only a single surfactant is present, most suitably a single non-ionic surfactant (suitably as defined herein).

Suitably the liquid pharmaceutical composition comprises a single surfactant, suitably as defined herein, whilst being either (substantially or entirely) free of any further surfactants or comprising one or more of said further surfactants in a (collective) concentration of at most 1 mM, more suitably at most 0.1mM, more suitably at most 0.01 mM, more suitably at most 0.001 mM, most suitably at most 0.0001 mM (i.e. these concentrations apply to any further surfactant(s) and not to the single surfactant). As such, where the surfactant is a PVP surfactant, the composition is suitably either (substantially or entirely) free of surfactants other than PVP surfactant(s) or comprises one or more of said surfactants other than PVP surfactant(s) in a (collective) concentration of at most 1 mM, more suitably at most 0.1mM, more suitably at most 0.01 mM, more suitably at most 0.001 mM, most suitably at most 0.0001 mM. Likewise, where the surfactant is polysorbate 80, the composition is suitably either (substantially or entirely) free of surfactants other than polysorbate 80 or comprises one or more of said surfactants other than polysorbate 80 in a (collective) concentration of at most 1 mM, more suitably at most 0.1mM, more suitably at most 0.01 mM, more suitably at most 0.001 mM, most suitably at most 0.0001 mM.

The liquid pharmaceutical compositions of the invention suitably employ certain surfactants to stabilise formulations more effectively.

### Diluent

The liquid pharmaceutical compositions of the invention may include any one or more pharmaceutically acceptable diluents, or mixture thereof. However, most suitably the liquid pharmaceutical composition is an aqueous pharmaceutical composition. Most suitably the diluent is water, and suitably water alone. The water is suitably water for injection (WFI).

Suitably the diluent may constitute the balance of ingredients in any liquid pharmaceutical composition, for instance so that the weight percentages total 100%. Suitably any concentrations given herein in relation to any component of the liquid pharmaceutical composition represent concentrations of said component in (and suitably dissolved in) the diluent in admixture with any other components.

The liquid pharmaceutical composition of the invention is suitably a solution, and is suitably (substantially or entirely) free of particulates or precipitates.

### Absent or low level components

### Low/No Arginine

Suitably the liquid pharmaceutical composition is either (substantially or entirely) free of arginine (suitably L-arginine) or comprises arginine in a concentration of at most 0.1 mM, more suitably at most 0.01mM, most suitably at most 0.001 mM.

Suitably the liquid pharmaceutical composition is either (substantially or entirely) free of arginine or comprises arginine in a molar ratio of arginine to buffer system of at most 1 : 150 (i.e. less than or equal to one mole of arginine for every 150 moles of buffer system), more suitably at most 1:1500, most suitably at most 1:15,000.

Suitably the liquid pharmaceutical composition is either (substantially or entirely) free of arginine or comprises arginine in a weight ratio of arginine to adalimumab of at most 1 : 3000 (i.e. less than or equal to one part by weight of arginine for every 3000 parts by weight adalimumab), more suitably at most 1:30,000, most suitably at most 1:300,000.

Suitably the liquid pharmaceutical composition is either (substantially or entirely) free of arginine or comprises arginine in a molar ratio of arginine to adalimumab of at most 1 : 3.75 (i.e. less than or equal to one mole of arginine for every 3.75 moles adalimumab), more suitably at most 1:37.5, most suitably at most 1:375.

As explained herein, such references to "arginine" in the context of their presence or otherwise within liquid pharmaceutical compositions relate to the corresponding free amino acid(s) and not amino acid residue(s) covalently incorporated as part of a larger compound, such as a peptide or protein.

The liquid pharmaceutical compositions of the invention are thought to offer sufficient stabilisation of adalimumab without the need for additional stabilisers, such as arginine, thereby avoiding the undesirable side effects associated therewith.

### Low / No Amino Acids

Suitably the liquid pharmaceutical composition is either (substantially or entirely) free of amino acids or comprises one or more amino acids in a (collective) concentration of at most 0.1 mM, more suitably at most 0.01mM, most suitably at most 0.001 mM.

Suitably the liquid pharmaceutical composition is either (substantially or entirely) free of amino acids or comprises one or more amino acids in a (collective) molar ratio of amino acids(s) to buffer system of at most 1 : 150 (i.e. less than or equal to one mole of amino acids(s) for every 150 moles of buffer system), more suitably at most 1:1500, most suitably at most 1:15,000.

Suitably the liquid pharmaceutical composition is either (substantially or entirely) free of amino acids or comprises one or more amino acids in a (collective) weight ratio of amino acids(s) to adalimumab of at most 1 : 3000 (i.e. less than or equal to one part by weight of amino acids(s) for every 3000 parts by weight adalimumab), more suitably at most 1:30,000, most suitably at most 1:300,000.

Suitably the liquid pharmaceutical composition is either (substantially or entirely) free of amino acids or comprises one or more amino acids in a (collective) molar ratio of amino acid(s) to adalimumab of at most 1 : 3.75 (i.e. less than or equal to one mole of amino acid(s) for every 3.75 moles adalimumab), more suitably at most 1:37.5, most suitably at most 1:375.

As explained herein, such references to "amino acids" in the context of their presence or otherwise within liquid pharmaceutical compositions relate to the corresponding free amino acid(s) and not amino acid residue(s) covalently incorporated as part of a larger compound, such as a peptide or protein.

Suitably, the amino acids referred to in this section (and deemed either absent or present in low quantities) may be natural and/or artificial amino acids, though they are preferably natural amino acids. In particular, the liquid pharmaceutical compositions are either (substantially or entirely) free of any amino acids selected from the group including: arginine, lysine, aspartic acid, and histidine; or comprises one or more of the aforesaid amino acids in an amount, concentration, molar ratio, or weight ratio as hereinbefore defined in relation to "amino acid(s)".

The liquid pharmaceutical compositions of the invention are thought to offer sufficient stabilisation of adalimumab without the need for additional stabilisers, such as amino acids, thereby avoiding the undesirable side effects associated therewith.

### Low / No Phosphate

Suitably the liquid pharmaceutical composition is either (substantially or entirely) free of phosphate buffering agents (e.g. sodium dihydrogen phosphate, disodium hydrogen phosphate) or comprises a phosphate buffer system in a concentration of at most 0.1 mM, more suitably at most 0.01mM, most suitably at most 0.001 mM.

Suitably the liquid pharmaceutical composition is either (substantially or entirely) free of phosphate buffering agents (e.g. sodium dihydrogen phosphate, disodium hydrogen phosphate) or comprises a phosphate buffer system in a molar ratio of phosphate buffer system to any non-phosphatebuffer systems present of at most 1 : 150 (i.e. less than or equal to one mole of phosphate buffering agent for every 150 moles of non-phosphate buffer system present), more suitably at most 1:1500, most suitably at most 1:15,000.

Suitably the liquid pharmaceutical composition is either (substantially or entirely) free of phosphate buffering agents or comprises a phosphate buffer system in a molar ratio of phosphate buffer system to adalimumab of at most 1 : 3.75 (i.e. less than or equal to one mole of phosphate buffer system for every 3.75 moles adalimumab), more suitably at most 1:37.5, most suitably at most 1:375.

References to "phosphate buffering agents" in the context of their presence or otherwise within liquid pharmaceutical compositions relate to any phosphate salts in any form or protonation state, including phosphate, monohydrogen phosphate, and dihydrogen phosphate. It does, however, suitably exclude any phosphate moieties or residues that may be covalently incorporated as part of a larger compound, such as a phosphorylated or glycosylated peptide or protein.

The liquid pharmaceutical compositions of the invention are thought to enable the provision of stable formulations without the use of phosphate buffering agents, which can in some circumstances complicate the production and quality control process.

### Optional additional components and features

### Tonicifier

The liquid pharmaceutical composition of the invention suitably comprises a "tonicity modifier" (or "tonicifier") or one or more tonicifiers, suitably as defined herein.

The inclusion of a tonicifier suitably contributes to (or increases) the overall osmolality and osmolarity of the composition. Suitably a tonicifier is present within the composition in a quantity or concentration sufficient for the composition to be (substantially) isotonic with body fluids. Suitably a tonicifier is present within the composition in a quantity or concentration sufficient for the composition to have an osmolarity or osmolality within a range defined herein.

Any suitable tonicifier may be used. However, suitably the tonicifier is a salt tonicifier (i.e. comprising ionic species). Such a salt tonicifier may be a carboxylate salt tonicifier (e.g. a gluconate salt tonicifier). Suitably the tonicifier is a metal salt tonicifier. Suitably the tonicifier is a water-soluble metal salt (e.g. sodium chloride, sodium gluconate, potassium chloride, magnesium chloride, calcium chloride). Suitably the tonicifier(s) is non-buffering (i.e. gives rise to little or no buffering effect). As such, any metal salt tonicifiers are suitably not buffering agents.

The liquid pharmaceutical composition may comprise one or more tonicifiers, though preferably only a single "tonicifier" as such is present (notwithstanding any tonicifying effects imparted to the composition by components intended to serve another function as defined herein).

Suitably, the tonicifier is or comprises a metal salt (preferably a non-buffering water-soluble metal salt). Suitably, said metal salt is or comprises a metal halide, suitably an alkali or an alkaline earth metal halide, suitably an alkali metal chloride (e.g. sodium chloride); or a metal carboxylate salt (e.g. a metal salt of a carboxylate derived from the oxidation of a sugar, for example: sodium gluconate, which is the sodium salt of a carboxylate derived from the oxidation of glucose).

In a particular embodiment, the tonicifier is a carboxylic tonicifier, be it a metal salt, non-metal salt, or free-acid of a carboxylic acid. The carboxylic tonicifier suitable comprises (or comprises a salt of) a tonicifying carboxylic acid. The tonicifying carboxylic acid may comprises one or more carboxylic acid (and/or carboxylate) moieties, suitably one or two carboxylic acid (and/or carboxylate) moieties (e.g. mono-/dicarboxylic acid). Most suitably, the tonicifying carboxylic acid is a monocarboxylic acid.

The tonicifying carboxylic acid is suitably a hydroxy carboxylic acid (which suitably encompasses a monohydroxy carboxylic acid or any polyhydroxy carboxylic acid). Suitably, the tonicifying carboxylic acid comprises at least one hydroxy group, suitably at least two hydroxy groups, suitably at least three hydroxy groups, suitably at least four hydroxy groups. Suitably, the tonicifying carboxylic acid comprises at most ten hydroxy groups, suitably at most six hydroxy groups. In a particular embodiment, the tonicifying carboxylic acid comprises exactly five hydroxy groups.

The tonicifying carboxylic acid suitably comprises x carboxylic acid (and/or carboxylate) moieties and *y* hydroxy groups such that the sum of x and *y* (i.e. x + y) is at least 2, suitably at least 3, more suitably at least 4, suitably at most 10, more suitably at most 8, most suitably exactly 6.

The tonicifying carboxylic acid suitably comprises x carboxylic acid (and/or carboxylate) moieties and *y* hydroxy groups such that the ratio of x to *y* (i.e. x : y) is between 1:10 and 1:1, more suitably between 1:7 and 1:3, most suitably exactly 1:5 (i.e. 5 alcohol groups per carboxylate/carboxylic acid group). Suitably the tonicifying carboxylic acid has a greater number of hydroxyl groups than carboxylic acid (and/or carboxylate) groups, suitably at least four times more.

The tonicifying carboxylic acid suitably has *'cAtoms'* carbon atoms and *'oAtoms'* oxygen atoms, such that the ratio of *cAtoms* to *oAtoms* (i.e. *cAtoms : oAtoms*) is between 5:1 to 1:5, more suitably between 2:1 and 1:2, and most suitably exactly 6:7.

The tonicifying carboxylic acid is suitably free of any nitrogen atoms. The tonicifying carboxylic acid is suitably free of any amine moieties (whether primary, secondary, tertiary, or quaternary). The tonicifying carboxylic acid is suitably free of any sulfur atoms.

The tonicifying carboxylic acid is suitably free of any aryl (e.g. phenyl) and heteroaryl (e.g. pyridyl, imidazole) groups.

Suitably, the tonicifying carboxylic acid is a hydroxycarboxylic acid (i.e. a hydroxycarboxylic acid with unspecified numbers of hydroxy and/or carboxylic acid groups). In a particular embodiment, tonicifying carboxylic acid is a (polyhydroxy)carboxylic acid (i.e. a hydroxycarboxylic acid with at least two hydroxy groups and an unspecified number of carboxylic acid groups). In a particular embodiment, the tonicifying carboxylic acid is a (hydroxy)mono-/di-carboxylic acid (i.e. a hydroxycarboxylic acid with an unspecified number of hydroxy groups but either one or two carboxylic acid groups, i.e. not zero carboxylic acid groups and not three or more). In a particular embodiment, the tonicifying carboxylic acid is a (hydroxy)monocarboxylic acid (i.e. a hydroxycarboxylic acid with an unspecified number of hydroxy groups but just one carboxylic acid group).

In a particular embodiment, the tonicifying carboxylic acid is gluconic acid. Suitably the carboxylic tonicifier is a gluconate salt.

The free acid form of the tonicifying carboxylic acid is suitably water soluble, suitably having a solubility of at least 50g per 1 L water, suitably at least 70g per 1 L of water, suitably at least 100 g per 1 L of water.

The carboxylic tonicifier as a whole is suitably water soluble, suitably having a solubility of at least 100g per 1 L water, suitably at least 200 g per 1 L of water, suitably at least 500 g per 1 L of water.

The tonicifying carboxylic acid suitably has a molecular weight (M_{w}) of at most 300 g/mol, suitably at most 200 g/mol. The tonicifying carboxylic acid suitably has a molecular weight (M_{w}) of at least 100 g/mol, suitably at least 150 g/mol.

Most suitably the carboxylic tonicifier is a metal salt of the tonicifying carboxylic acid. As such, the carboxylic tonicifier suitably comprises a metal species (suitably metal cations, suitably metal(I) cations or metal (II) cations, most suitably metal (I) cations) and a carboxylate of the tonicifying carboxylic acid.

In a particular embodiment, the carboxylic tonicifier is a metal carboxylate salt tonicifier. In a particular embodiment, the carboxylic tonicifier is a metal hydroxycarboxylate (i.e. a metal salt of a hydroxycarboxylic acid with unspecified numbers of hydroxy and/or carboxylic acid groups). In a particular embodiment, the carboxylic tonicifier is a metal (polyhydroxy)carboxylate (i.e. a metal salt of a hydroxycarboxylic acid with at least two hydroxy groups and an unspecified number of carboxylic acid groups). In a particular embodiment, the carboxylic tonicifier is a metal (hydroxy)mono-/di-carboxylate (i.e. a metal salt of a hydroxycarboxylic acid with an unspecified number of hydroxy groups but either one or two carboxylic acid groups, i.e. not zero carboxylic acid groups and not three or more). In a particular embodiment, the carboxylic tonicifier is a metal (hydroxy)monocarboxylate (i.e. a metal salt of a hydroxycarboxylic acid with an unspecified number of hydroxy groups but just one carboxylic acid group).

Suitably the (metal) carboxylate salt may be a salt of a (tonicifying) carboxylic acid (i.e. a deprotonated form thereof) defined by R₁CO₂H, wherein R₁ is selected from the group consisting of (1-12C)alkyl, (2-12C)alkenyl, (2-12C)alkynyl, (3-8C)cycloalkyl, (3-8C)cycloalkenyl, (1-12C)alkyl-(3-8C)cycloalkyl, (1-12C)alkyl-(3-8C)cycloalkenyl, (3-8C)cycloalkyl-(1-12C)alkyl, (3-8C)cycloalkenyl-(1-12C)alkyl, aryl, (1-12C)alkyl-aryl, aryl-(1-12C)alkyl, heteroaryl, (1-12C)alkyl-heteroaryl, heteroaryl-(1-12C)alkyl, heterocyclyl, (1-12C)alkyl- heterocyclyl, and heterocyclyl-(1-12C)alkyl; wherein any CH, CH₂ or CH₃ group within a R¹ group optionally bears on each said CH, CH₂ or CH₃ group one or more substituents selected from the group consisting of hydroxy, mercapto, amino, cyano, carboxy, carbamoyl, ureido, and (1-6C)alkoxy. R₁ is more suitably (1-6C)alkyl, most suitably pentyl. Suitably any optional substitutents of R₁ are hydroxyl.

In a particular embodiment, the tonicifier is or comprises a carboxylate salt tonicifier (suitably a metal carboxylate salt tonicifier) (e.g. a metal salt of a carboxylate /carboxylic acid, optionally derived from the oxidation of a sugar, for example: sodium gluconate, which is the sodium salt of a carboxylate derived from the oxidation of glucose).

Suitably, the tonicifier is a metal carboxylate salt selected from the group consisting of sodium gluconate, magnesium gluconate, and calcium gluconate.

In a particular embodiment, the tonicifier is or comprises sodium chloride and/or sodium gluconate. In a particular embodiment, the tonicifier is sodium chloride. In a particular embodiment, the tonicifier is sodium gluconate. Sodium chloride and sodium gluconate are thought to be particularly advantageous stabilisers for use alongside an acetate buffering agent/buffer system in liquid adalimumab formulations.

Suitably, the liquid pharmaceutical composition comprises the tonicifier(s) (most suitably sodium chloride or sodium gluconate) at a concentration of from about 10 to about 250 mM, more suitably from about 20 to about 200 mM, more suitably from about 25 to about 75 mM. In an embodiment, the tonicifier(s) is present at a concentration of between 40 and 60 mM, most suitably about 50 mM. In an embodiment, sodium chloride is present at a concentration of 50 mM. In a particular embodiment, sodium gluconate is present at a concentration between 45 mM and 230 mM.

Suitably, the liquid pharmaceutical composition comprises the tonicifier(s) at a concentration of from about 0.5 mg/mL to about 100 mg/mL.

Suitably, the liquid pharmaceutical composition comprises the tonicifier(s) (especially where the tonicifier is sodium chloride) at a concentration of from about 0.5 mg/mL to about 12 mg/mL, more suitably from about 1.2 mg/mL to about 5 mg/mL, more suitably from about 1.5 mg/mL to about 4.4 mg/mL. In an embodiment, the tonicifier(s) (especially where the tonicifier is sodium chloride) is present at a concentration of between 2.7 mg/mL and 3.1 mg/mL, most suitably about 2.9 mg/mL.

Suitably, the liquid pharmaceutical composition comprises the tonicifier(s) (especially where the tonicifier is sodium gluconate) at a concentration of from about 5 mg/mL to about 100 mg/mL, more suitably from about 10 mg/mL to about 50 mg/mL, suitably from about 20 mg/mL to about 30 mg/mL. In a particular embodiment, the tonicifier (especially where it is sodium gluconate) is present at a concentration from about 5 mg/mL to about 15 mg/mL.

Suitably, the liquid pharmaceutical composition comprises the tonicifier(s) (most suitably sodium chloride or sodium gluconate) in a molar ratio of tonicifier to adalimumab of from about 30:1 to about 725:1, suitably from about 30:1 to about 580:1, more suitably from about 60:1 to about 290:1, more suitably from about 70:1 to about 220:1. In an embodiment, the tonicifier(s) (especially where it is sodium chloride) is present at a molar ratio of tonicifier to adalimumab of from about 115:1 to about 175:1, most suitably about 145:1. In a particular embodiment, the tonicifier(s) (especially where it is sodium gluconate) is present at a molar ratio of tonicifier to adalimumab of from about 135:1 to about 667:1.

The tonicifier(s) is suitably present at a concentration that, in combination with the other components of the liquid pharmaceutical composition (especially in combination with the sugar stabiliser(s)), provides the composition with an osmolality as defined herein.

### Osmolality

Suitably, the osmolality of the liquid pharmaceutical composition is between 200 and 400 mOsm/kg, suitably between 250 and 400 mOsm/kg, more suitably between 220 and 390 mOsm/kg, more suitably between 230 and 350 mOsm/kg, more suitably between 240 and 340 mOsm/kg, more suitably between 260 and 320 mOsm/kg, most suitably between 280 and 310 mOsm/kg. Suitably the relative amounts and concentrations of the various components of the composition may be judiciously tuned to achieve the desired osmolality, and the particular novel combination of components allows this to be largely achieved without undermining other important parmeters.

### Methods of stabilising antibody

The present invention also provides a method of stabilising liquid adalimumab compositions (chemically and/or physically optionally in relation to any one or more of the aforementioned parameters/properties), comprising mixing with adalimumab with any relevant components required to form a liquid pharmaceutical composition as defined herein. Different embodiments will suitably require different combinations of components to be mixed, potentially in different amounts, and the skilled person can readily deduce such combinations and amounts by reference to the foregoing disclosure relating to the liquid pharmaceutical composition. Such different combinations of components may stabilise liquid adalimumab compositions in different respects. For instance, mixing adalimumab with the aforementioned components to form a liquid pharmaceutical composition as defined herein may stabilise adalimumab by:
i) Increasing the protein unfolding temperature of adalimumab;
ii) Inhibiting the formation of aggregates;
iii) Inhibiting the formation of fragments;
iv) Inhibiting the formation of sub-visible particles (either ≤25 microns or ≤10 microns);
v) Inhibiting turbidification;
vi) Inhibiting pH changes;
vii) Inhibiting photo-oxidation; and/or
viii) Reducing instability upon freeze/thaw cycles.

As such, the present invention provides a method of achieving one, some, or all of the following benefits:
i) Increased protein unfolding temperatures for adalimumab;
ii) Inhibition of formation of aggregates;
iii) Inhibition of formation of fragments;
iv) Inhibition of formation of sub-visible particles (either ≤25 microns or ≤10 microns);
v) Inhibition of turbidification;
vi) Inhibition of pH changes;
vii) Inhibition of photo-oxidation;
viii) Reduced instability upon freeze/thaw cycles; and/or
ix) Stabilisation of the isoform profile (especially with respect to the "main peak" as defined herein);
the method comprising manufacturing a liquid pharmaceutical composition of adalimumab as defined herein.

Suitably, the liquid pharmaceutical compositions of the invention have a shelf life of at least 6 months, suitably at least 12 months, suitably at least 18 months, more suitably at least 24 months. Suitably, the liquid pharmaceutical compositions of the invention have a shelf life of at least 6 months, suitably at least 12 months, suitably at least 18 months, more suitably at least 24 months, at a temperature of 2-8°C.

### Enabling the skilled person to optimise key stability properties

The novel combination of components disclosed for use in liquid pharmaceutical compositions of the invention enables the skilled person to produce (and judiciously fine-tune) compositions which exhibit comparable or enhanced properties relative to compositions of the prior art. In particular, the present disclosure now provides the skilled person with all the necessary tools to optimise formulation stability, and in particular optimise one or more of: inhibition of aggregation, fragmentation, protein unfolding, precipitation, pH slippage, and oxidation (especially photo-oxidation). Furthermore, the skilled person is given guidance on how to achieve such optimisations (through judiciously varying the compositions) and how, in the process, to minimise any detrimental side-effects. The present disclosure enables the skilled person to work across the scope of the invention to produce a variety of specific compositions which exhibit comparable or improved properties relative to compositions of the prior art, and this can be achieved using fewer components.

### Particular Embodiments

In certain embodiments, the liquid pharmaceutical composition comprises adalimumab and also any one of the combinations of Components 1, 2, and/or 3 stipulated in Table A below. In embodiments where one or more of the Components is left blank, the composition should be considered to require only those of Components 1, 2, and/or 3 that are actually stipulated, although it will be understood that such embodiments need not necessarily exclude compositions containing a non-stipulated component. By way of example, embodiment A.4 requires that the pharmaceutical composition comprise both a PVP surfactant and a monocyclic sugar stabiliser, but not necessarily a Component 3.

**Table A - Various embodiments (A.1-A.25) of liquid pharmaceutical compositions comprising adalimumab in combination with any stipulated Components.**

| **Embodiment No.** | **Component 1 (if required)** | **Component 2 (if required)** | **Component 3 (if required)** | **Corresponding component Set** |
|---|---|---|---|---|
| **A.1** | PVP surfactant | | | {1} |
| **A.2** | | Monocyclic SS | | {2} |
| **A.3** | | | Metal carbox | {3} |
| **A.4** | PVP surfactant | Monocyclic SS | | {1,2} |
| **A.5** | PVP surfactant | | Metal carbox | {1,3} |
| **A.6** | | Monocyclic SS | Metal carbox | {2,3} |
| **A.7** | PVP surfactant | Monocyclic SS | Metal carbox | {1,2,3} |
| **A.8** | PVP₁₉₀₀₋₃₁₀₀ | | | {1} |
| **A.9** | | Inositol | | {2} |
| **A.10** | | | Na(gluc) | {3} |
| **A.11** | PVP₁₉₀₀₋₃₁₀₀ | Monocyclic SS | | {1,2} |
| **A.12** | PVP₁₉₀₀₋₃₁₀₀ | | Metal carbox | {1,3} |
| **A.13** | PVP₁₉₀₀₋₃₁₀₀ | Inositol | | {1,2} |
| **A.14** | PVP₁₉₀₀₋₃₁₀₀ | | Na(gluc) | {1,3} |
| **A.15** | PVP surfactant | Inositol | | {1,2} |
| **A.16** | | Inositol | Metal carbox | {2,3} |
| **A.17** | | Inositol | Na(gluc) | {2,3} |
| **A.18** | PVP surfactant | | Na(gluc) | {1,3} |
| **A.19** | PVP₁₉₀₀₋₃₁₀₀ | Monocyclic SS | Metal carbox | {1,2,3} |
| **A.20** | PVP surfactant | Inositol | Metal carbox | {1,2,3} |
| **A.21** | PVP surfactant | Monocyclic SS | Na(gluc) | {1,2,3} |
| **A.22** | PVP₁₉₀₀₋₃₁₀₀ | Inositol | Metal carbox | {1,2,3} |
| **A.23** | PVP₁₉₀₀₋₃₁₀₀ | Monocyclic SS | Na(gluc) | {1,2,3} |
| **A.24** | PVP surfactant | Inositol | Na(gluc) | {1,2,3} |
| **A.25** | PVP₁₉₀₀₋₃₁₀₀ | Inositol | Na(gluc) | {1,2,3} |

| | | | | |
|---|---|---|---|---|
| "PVP surfactant" = a general PVP surfactant; "monocyclic SS" = a general monocyclic sugar stabiliser; "metal carbox" = a general metal (hydroxy)mono-/di-carboxylate salt tonicifier; "PVP₁₉₀₀₋₃₁₀₀" = a PVP surfactant having a weight average molecular weight of between 1900 and 3100; "Na(gluc)" = sodium gluconate | | | | |

**Table B - Various sub-embodiments (B.1-B.7) of the liquid pharmaceutical compositions of Table A, in which concentrations may be stipulated for one or more of the corresponding Components of the embodiments of Table A.**

| **Embodiment No.** | **Component 1 (PVP surfactant or PVP₁₉₀₀₋₃₁₀₀) conc.** | **Component 2 (monocyclic SS or inositol) conc.** | **Component 3 (metal carbox or Na(gluc)) conc.** | **Corresponding component Set** |
|---|---|---|---|---|
| **B.1** | 0.01 to 1 mg/mL | | | {1} |
| **B.2** | | 10 to 40 mg/mL | | {2} |
| **B.3** | | | 45 mM and 230 mM | {3} |
| **B.4** | 0.01 to 1 mg/mL | 10 to 40 mg/mL | | {1,2} |
| **B.5** | 0.01 to 1 mg/mL | | 45 mM and 230 mM | {1,3} |
| **B.6** | | 10 to 40 mg/mL | 45 mM and 230 mM | {2,3} |
| **B.7** | 0.01 to 1 mg/mL | 10 to 40 mg/mL | 45 mM and 230 mM | {1,2,3} |

In further embodiments, any, some, or all of the embodiments of Table A (A.1-A.25) may be combined with (or overlayed with), "where applicable" (i.e. where the embodiment of Table A comprises at least all of Components 1, 2, and/or 3 for which a concentration range is stipulated in Table B - see below), the embodimients set forth in Table B (B.1-B.7). As such, any, some, or all of the aforementioned embodiments (A.1-A.25) of Table A may be further defined by applying a concentration range or combination of concentration ranges, as set forth in Table B below, to one or more of Components 1 to 3. The concentration ranges (or combinations thereof) set forth in Table B may only be applied to embodiments of Table A which comprise at least all of the Components for which a concentration range is stipulated in Table B, which includes embodiments of Table A comprising more Components than Table B assigns a concentration range to (i.e. this leads to an embodiment defined by several components, some of which are defined by a concentration range and some of which have an undefined concentration). By way of example, embodiment A.7 may be combined with any of embodiments B.1-B.7 because A.7 makes a stipulation for all of Components 1 to 3. However, embodiment A.4 may only be combined with embodiments B.1, B.2, or B.4 because all other embodiments of Table B make a stipulation for Component 3, which is not stipulated by embodiment A.4. This is a practical illustration of the meaning of the aforementioned term "where applicable" (i.e. where the embodiment of Table A comprises at least all of Components 1, 2, and/or 3 for which a concentration range is stipulated in Table B - see above).

The liquid pharmaceutical composition suitably comprises or is characterised by adalimumab.

The liquid pharmaceutical composition suitably comprises or is characterised by a monocyclic sugar stabiliser; suitably wherein the monocyclic sugar stabiliser is selected from the group consisting of: Inositol, 10 to 40 mg/mL monocyclic sugar stabiliser, and 10 to 40 mg/mL Inositol.

The liquid pharmaceutical composition suitably comprises or is characterised by a metal (hydroxy)mono-/di-carboxylate salt tonicifier; suitably wherein the metal (hydroxy)mono-/di-carboxylate salt tonicifier is selected from the group consisting of: sodium gluconate, 45 mM to 230 mM metal (hydroxy)mono-/di-carboxylate salt tonicifier, and 45 mM to 230 mM sodium gluconate.

The liquid pharmaceutical composition suitably comprises or is characterised by a PVP surfactant; suitably wherein the PVP surfactant is selected from the group consisting of: a PVP surfactant having a weight average molecular weight of between 1900 and 3100, 0.01 to 1 mg/mL of a PVP surfactant, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a PVP surfactant.

In a particular embodiment, the liquid pharmaceutical composition comprises or is characterised by adalimumab, and the liquid pharmaceutical composition further comprises or is further characterised by: a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 0.01 to 1 mg/mL of a PVP surfactant.

In a particular embodiment, the liquid pharmaceutical composition comprises or is characterised by adalimumab, and the liquid pharmaceutical composition further comprises or is further characterised by: 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: a metal (hydroxy)mono-/di-carboxylate salt tonicifier, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a metal (hydroxy)mono-/di-carboxylate salt tonicifier, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: sodium gluconate.

In a particular embodiment, the liquid pharmaceutical composition comprises or is characterised by adalimumab, and the liquid pharmaceutical composition further comprises or is further characterised by: sodium gluconate, and a PVP surfactant.

In a particular embodiment, the liquid pharmaceutical composition comprises or is characterised by adalimumab, and the liquid pharmaceutical composition further comprises or is further characterised by: sodium gluconate, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: sodium gluconate, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: sodium gluconate, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 45 mM to 230 mM metal (hydroxy)mono-/dicarboxylate salt tonicifier.

In a particular embodiment, the liquid pharmaceutical composition comprises or is characterised by adalimumab, and the liquid pharmaceutical composition further comprises or is further characterised by: 45 mM to 230 mM metal (hydroxy)mono-/dicarboxylate salt tonicifier, and a PVP surfactant.

In a particular embodiment, the liquid pharmaceutical composition comprises or is characterised by adalimumab, and the liquid pharmaceutical composition further comprises or is further characterised by: 45 mM to 230 mM metal (hydroxy)mono-/dicarboxylate salt tonicifier, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 45 mM to 230 mM metal (hydroxy)mono-/dicarboxylate salt tonicifier, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: 45 mM to 230 mM metal (hydroxy)mono-/dicarboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 45 mM to 230 mM sodium gluconate.

In a particular embodiment, the liquid pharmaceutical composition comprises or is characterised by adalimumab, and the liquid pharmaceutical composition further comprises or is further characterised by: 45 mM to 230 mM sodium gluconate, and a PVP surfactant.

In a particular embodiment, the liquid pharmaceutical composition comprises or is characterised by adalimumab, and the liquid pharmaceutical composition further comprises or is further characterised by: 45 mM to 230 mM sodium gluconate, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 45 mM to 230 mM sodium gluconate, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: 45 mM to 230 mM sodium gluconate, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a monocyclic sugar stabiliser.

In a particular embodiment, the liquid pharmaceutical composition comprises or is characterised by adalimumab, and the liquid pharmaceutical composition further comprises or is further characterised by: a monocyclic sugar stabiliser, and a PVP surfactant.

In a particular embodiment, the liquid pharmaceutical composition comprises or is characterised by adalimumab, and the liquid pharmaceutical composition further comprises or is further characterised by: a monocyclic sugar stabiliser, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a monocyclic sugar stabiliser, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: a monocyclic sugar stabiliser, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a monocyclic sugar stabiliser, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: a monocyclic sugar stabiliser, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: a monocyclic sugar stabiliser, a metal (hydroxy)mono-/di-carboxylate salt tonicifier, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a monocyclic sugar stabiliser, a metal (hydroxy)mono-/di-carboxylate salt tonicifier, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: a monocyclic sugar stabiliser, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a monocyclic sugar stabiliser, and sodium gluconate.

In a particular embodiment, the liquid pharmaceutical composition comprises or is characterised by adalimumab, and the liquid pharmaceutical composition further comprises or is further characterised by: a monocyclic sugar stabiliser, sodium gluconate, and a PVP surfactant.

In a particular embodiment, the liquid pharmaceutical composition comprises or is characterised by adalimumab, and the liquid pharmaceutical composition further comprises or is further characterised by: a monocyclic sugar stabiliser, sodium gluconate, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a monocyclic sugar stabiliser, sodium gluconate, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: a monocyclic sugar stabiliser, sodium gluconate, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a monocyclic sugar stabiliser, and 45 mM to 230 mM metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: a monocyclic sugar stabiliser, 45 mM to 230 mM metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: a monocyclic sugar stabiliser, 45 mM to 230 mM metal (hydroxy)mono-/di-carboxylate salt tonicifier, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a monocyclic sugar stabiliser, 45 mM to 230 mM metal (hydroxy)mono-/di-carboxylate salt tonicifier, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: a monocyclic sugar stabiliser, 45 mM to 230 mM metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a monocyclic sugar stabiliser, and 45 mM to 230 mM sodium gluconate.

In a particular embodiment, the liquid pharmaceutical composition comprises or is characterised by adalimumab, and the liquid pharmaceutical composition further comprises or is further characterised by: a monocyclic sugar stabiliser, 45 mM to 230 mM sodium gluconate, and a PVP surfactant.

In a particular embodiment, the liquid pharmaceutical composition comprises or is characterised by adalimumab, and the liquid pharmaceutical composition further comprises or is further characterised by: a monocyclic sugar stabiliser, 45 mM to 230 mM sodium gluconate, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a monocyclic sugar stabiliser, 45 mM to 230 mM sodium gluconate, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: a monocyclic sugar stabiliser, 45 mM to 230 mM sodium gluconate, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: Inositol.

In a particular embodiment, the liquid pharmaceutical composition comprises or is characterised by adalimumab, and the liquid pharmaceutical composition further comprises or is further characterised by: Inositol, and a PVP surfactant.

In a particular embodiment, the liquid pharmaceutical composition comprises or is characterised by adalimumab, and the liquid pharmaceutical composition further comprises or is further characterised by: Inositol, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: Inositol, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: Inositol, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: Inositol, and a metal (hydroxy)mono-/dicarboxylate salt tonicifier.

In a particular embodiment, the liquid pharmaceutical composition comprises or is characterised by adalimumab, and the liquid pharmaceutical composition further comprises or is further characterised by: Inositol, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: Inositol, a metal (hydroxy)mono-/di-carboxylate salt tonicifier, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: Inositol, a metal (hydroxy)mono-/di-carboxylate salt tonicifier, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: Inositol, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: Inositol, and sodium gluconate.

In a particular embodiment, the liquid pharmaceutical composition comprises or is characterised by adalimumab, and the liquid pharmaceutical composition further comprises or is further characterised by: Inositol, sodium gluconate, and a PVP surfactant.

In a particular embodiment, the liquid pharmaceutical composition comprises or is characterised by adalimumab, and the liquid pharmaceutical composition further comprises or is further characterised by: Inositol, sodium gluconate, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: Inositol, sodium gluconate, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: Inositol, sodium gluconate, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: Inositol, and 45 mM to 230 mM metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: Inositol, 45 mM to 230 mM metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: Inositol, 45 mM to 230 mM metal (hydroxy)mono-/di-carboxylate salt tonicifier, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: Inositol, 45 mM to 230 mM metal (hydroxy)mono-/di-carboxylate salt tonicifier, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: Inositol, 45 mM to 230 mM metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: Inositol, and 45 mM to 230 mM sodium gluconate.

In a particular embodiment, the liquid pharmaceutical composition comprises or is characterised by adalimumab, and the liquid pharmaceutical composition further comprises or is further characterised by: Inositol, 45 mM to 230 mM sodium gluconate, and a PVP surfactant.

In a particular embodiment, the liquid pharmaceutical composition comprises or is characterised by adalimumab, and the liquid pharmaceutical composition further comprises or is further characterised by: Inositol, 45 mM to 230 mM sodium gluconate, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: Inositol, 45 mM to 230 mM sodium gluconate, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: Inositol, 45 mM to 230 mM sodium gluconate, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, and a PVP surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a metal (hydroxy)mono-/di-carboxylate salt tonicifier, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a metal (hydroxy)mono-/di-carboxylate salt tonicifier, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, and sodium gluconate.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, sodium gluconate, and a PVP surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, sodium gluconate, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, sodium gluconate, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, sodium gluconate, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, and 45 mM to 230 mM metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, 45 mM to 230 mM metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, 45 mM to 230 mM metal (hydroxy)mono-/di-carboxylate salt tonicifier, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, 45 mM to 230 mM metal (hydroxy)mono-/di-carboxylate salt tonicifier, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, 45 mM to 230 mM metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, and 45 mM to 230 mM sodium gluconate.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, 45 mM to 230 mM sodium gluconate, and a PVP surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, 45 mM to 230 mM sodium gluconate, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, 45 mM to 230 mM sodium gluconate, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, 45 mM to 230 mM sodium gluconate, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL Inositol.

In a particular embodiment, the liquid pharmaceutical composition comprises or is characterised by adalimumab, and the liquid pharmaceutical composition further comprises or is further characterised by: 10 to 40 mg/mL Inositol, and a PVP surfactant.

In a particular embodiment, the liquid pharmaceutical composition comprises or is characterised by adalimumab, and the liquid pharmaceutical composition further comprises or is further characterised by: 10 to 40 mg/mL Inositol, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL Inositol, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: 10 to 40 mg/mL Inositol, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL Inositol, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 10 to 40 mg/mL Inositol, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 10 to 40 mg/mL Inositol, a metal (hydroxy)mono-/di-carboxylate salt tonicifier, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL Inositol, a metal (hydroxy)mono-/di-carboxylate salt tonicifier, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: 10 to 40 mg/mL Inositol, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL Inositol, and sodium gluconate.

In a particular embodiment, the liquid pharmaceutical composition comprises or is characterised by adalimumab, and the liquid pharmaceutical composition further comprises or is further characterised by: 10 to 40 mg/mL Inositol, sodium gluconate, and a PVP surfactant.

In a particular embodiment, the liquid pharmaceutical composition comprises or is characterised by adalimumab, and the liquid pharmaceutical composition further comprises or is further characterised by: 10 to 40 mg/mL Inositol, sodium gluconate, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL Inositol, sodium gluconate, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: 10 to 40 mg/mL Inositol, sodium gluconate, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL Inositol, and 45 mM to 230 mM metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 10 to 40 mg/mL Inositol, 45 mM to 230 mM metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 10 to 40 mg/mL Inositol, 45 mM to 230 mM metal (hydroxy)mono-/di-carboxylate salt tonicifier, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL Inositol, 45 mM to 230 mM metal (hydroxy)mono-/di-carboxylate salt tonicifier, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: 10 to 40 mg/mL Inositol, 45 mM to 230 mM metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL Inositol, and 45 mM to 230 mM sodium gluconate.

In a particular embodiment, the liquid pharmaceutical composition comprises or is characterised by adalimumab, and the liquid pharmaceutical composition further comprises or is further characterised by: 10 to 40 mg/mL Inositol, 45 mM to 230 mM sodium gluconate, and a PVP surfactant.

In a particular embodiment, the liquid pharmaceutical composition comprises or is characterised by adalimumab, and the liquid pharmaceutical composition further comprises or is further characterised by: 10 to 40 mg/mL Inositol, 45 mM to 230 mM sodium gluconate, and a PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL Inositol, 45 mM to 230 mM sodium gluconate, and 0.01 to 1 mg/mL of a PVP surfactant.

In a particular embodiment, the liquid pharmaceutical composition comprises or is characterised by adalimumab, and the liquid pharmaceutical composition further comprises or is further characterised by: 10 to 40 mg/mL Inositol, 45 mM to 230 mM sodium gluconate, and 0.01 to 1 mg/mL PVP surfactant having a weight average molecular weight of between 1900 and 3100.

In a particular embodiment, the liquid pharmaceutical composition comprises or is characterised by a monocyclic sugar stabiliser, and the liquid pharmaceutical composition further comprises or is further characterised by: a PVP surfactant.

In a particular embodiment, the liquid pharmaceutical composition comprises or is characterised by a monocyclic sugar stabiliser, and the liquid pharmaceutical composition further comprises or is further characterised by: a PVP surfactant having a weight average molecular weight of between 1900 and 3100.

In a particular embodiment, the liquid pharmaceutical composition comprises or is characterised by a monocyclic sugar stabiliser, and the liquid pharmaceutical composition further comprises or is further characterised by: 0.01 to 1 mg/mL of a PVP surfactant.

In a particular embodiment, the liquid pharmaceutical composition comprises or is characterised by a monocyclic sugar stabiliser, and the liquid pharmaceutical composition further comprises or is further characterised by: 0.01 to 1 mg/mL PVP surfactant having a weight average molecular weight of between 1900 and 3100.

In a particular embodiment, the liquid pharmaceutical composition comprises or is characterised by a monocyclic sugar stabiliser, and the liquid pharmaceutical composition further comprises or is further characterised by: a metal (hydroxy)mono-/dicarboxylate salt tonicifier.

In a particular embodiment, the liquid pharmaceutical composition comprises or is characterised by a monocyclic sugar stabiliser, and the liquid pharmaceutical composition further comprises or is further characterised by: a metal (hydroxy)mono-/dicarboxylate salt tonicifier, and a PVP surfactant.

In a particular embodiment, the liquid pharmaceutical composition comprises or is characterised by a monocyclic sugar stabiliser, and the liquid pharmaceutical composition further comprises or is further characterised by: a metal (hydroxy)mono-/dicarboxylate salt tonicifier, and a PVP surfactant having a weight average molecular weight of between 1900 and 3100.

In a particular embodiment, the liquid pharmaceutical composition comprises or is characterised by a monocyclic sugar stabiliser, and the liquid pharmaceutical composition further comprises or is further characterised by: a metal (hydroxy)mono-/dicarboxylate salt tonicifier, and 0.01 to 1 mg/mL of a PVP surfactant.

In a particular embodiment, the liquid pharmaceutical composition comprises or is characterised by a monocyclic sugar stabiliser, and the liquid pharmaceutical composition further comprises or is further characterised by: a metal (hydroxy)mono-/dicarboxylate salt tonicifier, and 0.01 to 1 mg/mL PVP surfactant having a weight average molecular weight of between 1900 and 3100.

In a particular embodiment, the liquid pharmaceutical composition comprises or is characterised by a monocyclic sugar stabiliser, and the liquid pharmaceutical composition further comprises or is further characterised by: sodium gluconate.

In a particular embodiment, the liquid pharmaceutical composition comprises or is characterised by a monocyclic sugar stabiliser, and the liquid pharmaceutical composition further comprises or is further characterised by: sodium gluconate, and a PVP surfactant.

In a particular embodiment, the liquid pharmaceutical composition comprises or is characterised by a monocyclic sugar stabiliser, and the liquid pharmaceutical composition further comprises or is further characterised by: sodium gluconate, and a PVP surfactant having a weight average molecular weight of between 1900 and 3100.

In a particular embodiment, the liquid pharmaceutical composition comprises or is characterised by a monocyclic sugar stabiliser, and the liquid pharmaceutical composition further comprises or is further characterised by: sodium gluconate, and 0.01 to 1 mg/mL of a PVP surfactant.

In a particular embodiment, the liquid pharmaceutical composition comprises or is characterised by a monocyclic sugar stabiliser, and the liquid pharmaceutical composition further comprises or is further characterised by: sodium gluconate, and 0.01 to 1 mg/mL PVP surfactant having a weight average molecular weight of between 1900 and 3100.

In a particular embodiment, the liquid pharmaceutical composition comprises or is characterised by a monocyclic sugar stabiliser, and the liquid pharmaceutical composition further comprises or is further characterised by: 45 mM to 230 mM metal (hydroxy)mono-/di-carboxylate salt tonicifier.

In a particular embodiment, the liquid pharmaceutical composition comprises or is characterised by a monocyclic sugar stabiliser, and the liquid pharmaceutical composition further comprises or is further characterised by: 45 mM to 230 mM metal (hydroxy)mono-/di-carboxylate salt tonicifier, and a PVP surfactant.

In a particular embodiment, the liquid pharmaceutical composition comprises or is characterised by a monocyclic sugar stabiliser, and the liquid pharmaceutical composition further comprises or is further characterised by: 45 mM to 230 mM metal (hydroxy)mono-/di-carboxylate salt tonicifier, and a PVP surfactant having a weight average molecular weight of between 1900 and 3100.

In a particular embodiment, the liquid pharmaceutical composition comprises or is characterised by a monocyclic sugar stabiliser, and the liquid pharmaceutical composition further comprises or is further characterised by: 45 mM to 230 mM metal (hydroxy)mono-/di-carboxylate salt tonicifier, and 0.01 to 1 mg/mL of a PVP surfactant.

In a particular embodiment, the liquid pharmaceutical composition comprises or is characterised by a monocyclic sugar stabiliser, and the liquid pharmaceutical composition further comprises or is further characterised by: 45 mM to 230 mM metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by a monocyclic sugar stabiliser, and the liquid pharmaceutical composition further comprises or is further characterised by: 45 mM to 230 mM sodium gluconate.

In a particular embodiment, the liquid pharmaceutical composition comprises or is characterised by a monocyclic sugar stabiliser, and the liquid pharmaceutical composition further comprises or is further characterised by: 45 mM to 230 mM sodium gluconate, and a PVP surfactant.

In a particular embodiment, the liquid pharmaceutical composition comprises or is characterised by a monocyclic sugar stabiliser, and the liquid pharmaceutical composition further comprises or is further characterised by: 45 mM to 230 mM sodium gluconate, and a PVP surfactant having a weight average molecular weight of between 1900 and 3100.

In a particular embodiment, the liquid pharmaceutical composition comprises or is characterised by a monocyclic sugar stabiliser, and the liquid pharmaceutical composition further comprises or is further characterised by: 45 mM to 230 mM sodium gluconate, and 0.01 to 1 mg/mL of a PVP surfactant.

In a particular embodiment, the liquid pharmaceutical composition comprises or is characterised by a monocyclic sugar stabiliser, and the liquid pharmaceutical composition further comprises or is further characterised by: 45 mM to 230 mM sodium gluconate, and 0.01 to 1 mg/mL PVP surfactant having a weight average molecular weight of between 1900 and 3100.

In a particular embodiment, the liquid pharmaceutical composition comprises or is characterised by a metal (hydroxy)mono-/di-carboxylate salt tonicifier, and the liquid pharmaceutical composition further comprises or is further characterised by: a PVP surfactant.

In a particular embodiment, the liquid pharmaceutical composition comprises or is characterised by a metal (hydroxy)mono-/di-carboxylate salt tonicifier, and the liquid pharmaceutical composition further comprises or is further characterised by: a PVP surfactant having a weight average molecular weight of between 1900 and 3100.

In a particular embodiment, the liquid pharmaceutical composition comprises or is characterised by a metal (hydroxy)mono-/di-carboxylate salt tonicifier, and the liquid pharmaceutical composition further comprises or is further characterised by: 0.01 to 1 mg/mL of a PVP surfactant.

In a particular embodiment, the liquid pharmaceutical composition comprises or is characterised by a metal (hydroxy)mono-/di-carboxylate salt tonicifier, and the liquid pharmaceutical composition further comprises or is further characterised by: 0.01 to 1 mg/mL PVP surfactant having a weight average molecular weight of between 1900 and 3100.

In particular embodiments, the composition comprises the components stipulated in any one of the following paragraphs number i)-xxxiv):
i) adalimumab, a sugar stabiliser, and a surfactant, on the proviso that the surfactant is a surfactant other than polysorbate 80 when the sugar stabiliser is trehalose;
ii) adalimumab and a PVP surfactant;
iii) adalimumab and inositol;
iv) adalimumab, inositol, and a PVP surfactant;
v) adalimumab and a gluconate salt tonicifier;
vi) adalimumab, a gluconate salt tonicifier, and a PVP surfactant;
vii) adalimumab, inositol, and a gluconate salt tonicifier;
viii) adalimumab, inositol, a gluconate salt tonicifier, and a PVP surfactant;
ix) adalimumab and 0.01 to 1 mg/mL PVP surfactant;
x) adalimumab and 10 to 40 mg/mL inositol;
xi) adalimumab, 10 to 40 mg/mL inositol, and 0.01 to 1 mg/mL PVP surfactant;
xii) adalimumab and 10 to 50 mg/mL gluconate salt tonicifier;
xiii) adalimumab, 10 to 50 mg/mL gluconate salt tonicifier, and 0.01 to 1 mg/mL PVP surfactant;
xiv) adalimumab, 10 to 40 mg/mL inositol, and 10 to 50 mg/mL gluconate salt tonicifier;
xv) adalimumab, 10 to 40 mg/mL inositol, 10 to 50 mg/mL gluconate salt tonicifier, and 0.01 to 1 mg/mL PVP surfactant;
xvi) adalimumab, a buffer system (e.g. acetate buffer system), a sugar stabiliser (e.g. trehalose or inositol), and a surfactant (e.g. PVP or polysorbate 80)
xvii) adalimumab, a buffer system, a sugar stabiliser, a tonicifier, and a surfactant;
xviii) adalimumab, a buffer system, a sugar stabiliser, a tonicifier, and a surfactant, on the proviso that the surfactant is a surfactant other than polysorbate 80 when the sugar stabiliser is trehalose;
xix) adalimumab, a buffer system, a sugar stabiliser, a tonicifier, and a PVP surfactant;
xx) adalimumab, a buffer system, inositol, a tonicifier, and a surfactant;
xxi) adalimumab, a buffer system, inositol, a tonicifier, and a PVP surfactant;
xxii) adalimumab, a buffer system, a sugar stabiliser, a gluconate salt tonicifier, and a surfactant;
xxiii) adalimumab, a buffer system, inositol, a gluconate salt tonicifier, and a surfactant;
xxiv) adalimumab, a buffer system, a sugar stabiliser, a gluconate salt tonicifier, and a PVP surfactant;
xxv) adalimumab, a buffer system, inositol, a gluconate salt tonicifier, and a PVP surfactant.
xxvi) adalimumab, an acetate buffer system, a sugar stabiliser, a tonicifier, and a surfactant;
xxvii) adalimumab, an acetate buffer system, a sugar stabiliser, a tonicifier, and a surfactant, on the proviso that the surfactant is a surfactant other than polysorbate 80 when the sugar stabiliser is trehalose;
xxviii) adalimumab, an acetate buffer system, a sugar stabiliser, a tonicifier, and a PVP surfactant having a weight average molecular weight M_{w} of between 1000 and 20000;
xxix) adalimumab, an acetate buffer system, inositol, a tonicifier, and a surfactant;
xxx) adalimumab, an acetate buffer system, inositol, a tonicifier, and a PVP surfactant having a weight average molecular weight M_{w} of between 1000 and 20000;
xxxi) adalimumab, an acetate buffer system, a sugar stabiliser, a sodium gluconate tonicifier, and a surfactant;
xxxii) adalimumab, an acetate buffer system, inositol, a sodium gluconate tonicifier, and a surfactant;
xxxiii) adalimumab, an acetate buffer system, a sugar stabiliser, a sodium gluconate tonicifier, and a PVP surfactant having a weight average molecular weight M_{w} of between 1000 and 20000;
xxxiv) adalimumab, an acetate buffer system, inositol, a sodium gluconate tonicifier, and a PVP surfactant having a weight average molecular weight M_{w} of between 1000 and 20000.

In a particular embodiment, where the composition comprises adalimumab, inositol, and a gluconate salt (e.g. sodium gluconate), suitably inositol and the gluconate salt are present in concentrations sufficient to imbue the composition with an osmolality between 250 and 400 mOsm/kg.

In an embodiment, the liquid pharmaceutical composition comprises:
- adalimumab;
- an acetate buffer system;
- a sugar stabiliser (e.g. trehalose or inositol);
- a tonicifier (e.g. sodium chloride or sodium gluconate); and
- a surfactant (e.g. PVP or polysorbate 80);
   wherein the composition has a pH between 5.0 and 5.5.

In an embodiment, the liquid pharmaceutical composition comprises:
- adalimumab;
- an acetate buffer system;
- a sugar stabiliser (e.g. trehalose or inositol);
- a tonicifier (e.g. sodium chloride or sodium gluconate); and
- a PVP surfactant.

In an embodiment, the liquid pharmaceutical composition comprises:
- adalimumab;
- an acetate buffer system;
- inositol;
- sodium chloride or sodium gluconate; and
- a PVP surfactant.

In an embodiment, the liquid pharmaceutical composition comprises:
- adalimumab;
- an acetate buffer system;
- trehalose;
- sodium chloride or sodium gluconate; and
- a PVP surfactant.

In an embodiment, the liquid pharmaceutical composition comprises:
- adalimumab;
- an acetate buffer system;
- inositol (or, in some embodiments, trehalose); and
- polysorbate 80.

In an embodiment, the liquid pharmaceutical composition comprises:
- adalimumab;
- an acetate buffer system;
- inositol (or, in some embodiments, trehalose); and
- a tonicifier (e.g. sodium chloride); and
- polysorbate 80.

Suitably, the liquid pharmaceutical composition:
- is (substantially or entirely) free of arginine (suitably L-arginine); comprises arginine in a concentration of at most 0.1 mM;
- is (substantially or entirely) free of amino acids or comprises one or more amino acids in a (collective) concentration of at most 0.1 mM; and
- is (substantially or entirely) free of phosphate buffering agents (e.g. sodium dihydrogen phosphate, disodium hydrogen phosphate) or comprises a phosphate buffer system in a concentration of at most 0.1 mM.

Suitably, the liquid pharmaceutical composition:
- is (substantially or entirely) free of arginine (suitably L-arginine); comprises arginine in a concentration of at most 0.1 mM;
- is (substantially or entirely) free of amino acids or comprises one or more amino acids in a (collective) concentration of at most 0.1 mM;
- is (substantially or entirely) free of surfactants with the exception of polysorbate 80 and/or PVP (suitably PVP having a weight average molecular weight of between 1900 and 3100) or comprises one or more of said surfactants (excluding polysorbate 80 and/or PVP, suitably having a weight average molecular weight of between 1900 and 3100) in a (collective) concentration of at most 1 mM; and
- is (substantially or entirely) free of phosphate buffering agents (e.g. sodium dihydrogen phosphate, disodium hydrogen phosphate) or comprises a phosphate buffer system in a concentration of at most 0.1 mM.

It will be appreciated that the buffering agent (e.g. sodium acetate) or buffer system (e.g. sodium acetate/acetic acid) of any of the aforementioned embodiments may be directly incorporated into the compositions or may be produced *in situ,* for instance, *via* an acid base reaction, suitably by reacting a conjugate acid of the buffering agent (e.g. acetic acid) with a base (e.g. sodium hydroxide). Regardless of the method used to provide or produce the buffering agent or buffer system, suitably the resulting composition ultimately comprises an appropriate balance of the buffering agent and any acid/base conjugate to furnish the desired pH. The skilled person will be readily able to calculate or experimentally determine, without undue effort, the appropriate balance of buffering agent and acid/base conjugate, and/or the amount of base which needs to be added to a conjugate acid in order to produce the appropriate amount of buffering agent and furnish the desired pH.

In an embodiment, the liquid pharmaceutical composition comprises:
- adalimumab;
- an acetate buffer system (e.g. sodium acetate/acetic acid);
- a sugar stabiliser (e.g. trehalose or inositol);
- a tonicifier (e.g. sodium chloride or sodium gluconate);
- PVP (suitably PVP having a weight average molecular weight of between 1900 and 3100), suitably in a concentration between 0.01 and 1 mg/mL; and
- water (for injection).

In an embodiment, the liquid pharmaceutical composition comprises:
- adalimumab;
- an acetate buffer system (e.g. sodium acetate/acetic acid);
- inositol (suitably in a concentration from 10 to 40 mg/mL; or suitably 55 mM to 225mM inositol);
- a tonicifier (e.g. sodium chloride or sodium gluconate);
- a surfactant, suitably a surfactant selected from the group consisting of polysorbate 80 (suitably in a concentration from 0.5 mg/mL to about 1.5 mg/mL polysorbate 80) and PVP (especially PVP having a weight average molecular weight of between 1900 and 3100; suitably in a concentration from 0.01 mg/mL to 1 mg/mL PVP); and
- water (for injection).

In an embodiment, the liquid pharmaceutical composition comprises:
- adalimumab;
- an acetate buffer system (e.g. sodium acetate/acetic acid);
- inositol (suitably in a concentration from 10 to 40 mg/mL; or suitably 55 mM to 225mM inositol);
- a tonicifier (e.g. sodium chloride or sodium gluconate);
- PVP (especially PVP having a weight average molecular weight of between 1900 and 3100; suitably in a concentration from 0.01 mg/mL to 1 mg/mL PVP); and
- water (for injection).

In an embodiment, the liquid pharmaceutical composition comprises:
- adalimumab;
- an acetate buffer system (e.g. sodium acetate/acetic acid);
- inositol (suitably in a concentration from 10 to 40 mg/mL; or suitably 55 mM to 225mM inositol);
- sodium gluconate (suitably in a concentration between 45 mM and 230 mM);
- PVP (especially PVP having a weight average molecular weight of between 1900 and 3100; suitably in a concentration from 0.01 mg/mL to 1 mg/mL PVP); and
- water (for injection).

In an embodiment, the liquid pharmaceutical composition comprises:
- adalimumab;
- an acetate buffer system (e.g. sodium acetate/acetic acid);
- a sugar stabiliser (e.g. trehalose or inositol);
- sodium gluconate (suitably in a concentration between 45 mM and 230 mM);
- a surfactant, suitably a surfactant selected from the group consisting of polysorbate 80 (suitably in a concentration from 0.5 mg/mL to about 1.5 mg/mL polysorbate 80) and PVP (especially PVP having a weight average molecular weight of between 1900 and 3100; suitably in a concentration from 0.01 mg/mL to 1 mg/mL PVP); and
- water (for injection).

In an embodiment, the liquid pharmaceutical composition comprises:
- 40 to about 60 mg/mL adalimumab;
- 5 to about 15 mM acetate buffer system (e.g. sodium acetate/acetic acid);
- 10 to about 400 mM sugar stabiliser (e.g. trehalose or inositol);
- 10 to 250 mM tonicifier (e.g. sodium chloride or sodium gluconate);
- PVP (suitably PVP having a weight average molecular weight of between 1900 and 3100), suitably in a concentration between 0.01 and 1 mg/mL; and
- water (for injection);
   wherein the composition:
   - has a pH between 5.0 and 5.5;
   - is (substantially or entirely) free of arginine (suitably L-arginine) or comprises arginine in a concentration of at most 0.1 mM;
   - is (substantially or entirely) free of amino acids or comprises one or more amino acids in a (collective) concentration of at most 0.1 mM;
   - is (substantially or entirely) free of surfactants with the exception of PVP (suitably PVP having a weight average molecular weight of between 1900 and 3100) or comprises one or more of said surfactants (excluding PVP, suitably having a weight average molecular weight of between 1900 and 3100) in a (collective) concentration of at most 1 mM; and/or
   - is (substantially or entirely) free of phosphate buffering agents (e.g. sodium dihydrogen phosphate, disodium hydrogen phosphate) or comprises a phosphate buffer system in a concentration of at most 0.1 mM.

In an embodiment, the liquid pharmaceutical composition comprises:
- 40 to about 60 mg/mL adalimumab;
- 5 to about 15 mM acetate buffer system (e.g. sodium acetate/acetic acid);
- inositol (suitably in a concentration from 10 to 40 mg/mL; or suitably 55 mM to 225mM inositol);
- 10 to 250 mM tonicifier (e.g. sodium chloride or sodium gluconate);
- a surfactant selected from the group consisting of polysorbate 80 (suitably in a concentration from 0.5 mg/mL to about 1.5 mg/mL polysorbate 80) and PVP (especially PVP having a weight average molecular weight of between 1900 and 3100; suitably in a concentration from 0.01 mg/mL to 1 mg/mL PVP); and
- water (for injection);
   wherein the composition:
   - has a pH between 5.0 and 5.5;
   - is (substantially or entirely) free of arginine (suitably L-arginine) or comprises arginine in a concentration of at most 0.1 mM;
   - is (substantially or entirely) free of amino acids or comprises one or more amino acids in a (collective) concentration of at most 0.1 mM;
   - is (substantially or entirely) free of surfactants with the exception of polysorbate 80 and/or PVP (suitably PVP having a weight average molecular weight of between 1900 and 3100) or comprises one or more of said surfactants (excluding polysorbate 80 and/or PVP, suitably having a weight average molecular weight of between 1900 and 3100) in a (collective) concentration of at most 1 mM; and/or
   - is (substantially or entirely) free of phosphate buffering agents (e.g. sodium dihydrogen phosphate, disodium hydrogen phosphate) or comprises a phosphate buffer system in a concentration of at most 0.1 mM.

In an embodiment, the liquid pharmaceutical composition comprises:
- 40 to about 60 mg/mL adalimumab;
- 5 to about 15 mM acetate buffer system (e.g. sodium acetate/acetic acid);
- inositol (suitably in a concentration from 10 to 40 mg/mL; or suitably 55 mM to 225mM inositol);
- 10 to 250 mM tonicifier (e.g. sodium chloride or sodium gluconate);
- PVP (especially PVP having a weight average molecular weight of between 1900 and 3100; suitably in a concentration from 0.01 mg/mL to 1 mg/mL PVP); and
- water (for injection);
   wherein the composition:
   - has a pH between 5.0 and 5.5;
   - is (substantially or entirely) free of arginine (suitably L-arginine) or comprises arginine in a concentration of at most 0.1 mM;
   - is (substantially or entirely) free of amino acids or comprises one or more amino acids in a (collective) concentration of at most 0.1 mM;
   - is (substantially or entirely) free of surfactants with the exception of PVP (suitably PVP having a weight average molecular weight of between 1900 and 3100) or comprises one or more of said surfactants (excluding PVP, suitably having a weight average molecular weight of between 1900 and 3100) in a (collective) concentration of at most 1 mM; and/or
   - is (substantially or entirely) free of phosphate buffering agents (e.g. sodium dihydrogen phosphate, disodium hydrogen phosphate) or comprises a phosphate buffer system in a concentration of at most 0.1 mM.

In an embodiment, the liquid pharmaceutical composition comprises:
- 40 to about 60 mg/mL adalimumab;
- 5 to about 15 mM acetate buffer system (e.g. sodium acetate/acetic acid);
- inositol (suitably in a concentration from 10 to 40 mg/mL; or suitably 55 mM to 225mM inositol);
- sodium gluconate (suitably in a concentration between 45 mM and 230 mM);
- PVP (especially PVP having a weight average molecular weight of between 1900 and 3100; suitably in a concentration from 0.01 mg/mL to 1 mg/mL PVP); and
- water (for injection);
   wherein the composition:
   - has a pH between 5.0 and 5.5;
   - is (substantially or entirely) free of arginine (suitably L-arginine) or comprises arginine in a concentration of at most 0.1 mM;
   - is (substantially or entirely) free of amino acids or comprises one or more amino acids in a (collective) concentration of at most 0.1 mM;
   - is (substantially or entirely) free of surfactants with the exception of PVP (suitably PVP having a weight average molecular weight of between 1900 and 3100) or comprises one or more of said surfactants (excluding PVP, suitably having a weight average molecular weight of between 1900 and 3100) in a (collective) concentration of at most 1 mM; and/or
   - is (substantially or entirely) free of phosphate buffering agents (e.g. sodium dihydrogen phosphate, disodium hydrogen phosphate) or comprises a phosphate buffer system in a concentration of at most 0.1 mM.

In an embodiment, the liquid pharmaceutical composition comprises:
- 40 to about 60 mg/mL adalimumab;
- 5 to about 15 mM acetate buffer system (e.g. sodium acetate/acetic acid);
- 10 to about 400 mM sugar stabiliser (e.g. trehalose or inositol);
- sodium gluconate (suitably in a concentration between 45 mM and 230 mM);
- a surfactant selected from the group consisting of polysorbate 80 (suitably in a concentration from 0.5 mg/mL to about 1.5 mg/mL polysorbate 80) and PVP (especially PVP having a weight average molecular weight of between 1900 and 3100; suitably in a concentration from 0.01 mg/mL to 1 mg/mL PVP); and
- water (for injection);
   wherein the composition:
   - has a pH between 5.0 and 5.5;
   - is (substantially or entirely) free of arginine (suitably L-arginine) or comprises arginine in a concentration of at most 0.1 mM;
   - is (substantially or entirely) free of amino acids or comprises one or more amino acids in a (collective) concentration of at most 0.1 mM;
   - is (substantially or entirely) free of surfactants with the exception of polysorbate 80 and/or PVP (suitably PVP having a weight average molecular weight of between 1900 and 3100) or comprises one or more of said surfactants (excluding polysorbate 80 and/or PVP, suitably having a weight average molecular weight of between 1900 and 3100) in a (collective) concentration of at most 1 mM; and/or
   - is (substantially or entirely) free of phosphate buffering agents (e.g. sodium dihydrogen phosphate, disodium hydrogen phosphate) or comprises a phosphate buffer system in a concentration of at most 0.1 mM.

In an embodiment, the liquid pharmaceutical composition comprises:
- 25 to about 75 mg/mL adalimumab;
- 2 to about 50 mM sodium acetate/acetic acid buffer system;
- 100 to about 300 mM trehalose;
- 10 to about 200 mM sodium chloride;
- 0.001 to 2 mg/mL PVP having a weight average molecular weight of between 1900 and 3100; and
- water (for injection);
   wherein the composition:
   - has a pH between 5.0 and 5.5;
   - is (substantially or entirely) free of arginine (suitably L-arginine) or comprises arginine in a concentration of at most 0.1 mM;
   - is (substantially or entirely) free of amino acids or comprises one or more amino acids in a (collective) concentration of at most 0.1 mM;
   - is (substantially or entirely) free of surfactants with the exception of PVP having a weight average molecular weight of between 1900 and 3100 or comprises one or more of said surfactants (excluding PVP having a weight average molecular weight of between 1900 and 3100) in a (collective) concentration of at most 1 mM; and/or
   - is (substantially or entirely) free of phosphate buffering agents (e.g. sodium dihydrogen phosphate, disodium hydrogen phosphate) or comprises a phosphate buffer system in a concentration of at most 0.1 mM.

In an embodiment, the liquid pharmaceutical composition comprises:
- 25 to about 75 mg/mL adalimumab;
- 2 to about 50 mM sodium acetate/acetic acid buffer system;
- 5 to 45 mg/mL inositol (or 25 mM to 250 mM inositol);
- 10 to about 200 mM sodium chloride;
- 0.001 mg/mL to about 5 mg/mL polysorbate 80; and
- water (for injection);
   wherein the composition:
   - has a pH between 5.0 and 5.5;
   - is (substantially or entirely) free of arginine (suitably L-arginine) or comprises arginine in a concentration of at most 0.1 mM;
   - is (substantially or entirely) free of amino acids or comprises one or more amino acids in a (collective) concentration of at most 0.1 mM;
   - is (substantially or entirely) free of surfactants with the exception of polysorbate 80 or comprises one or more of said surfactants (excluding polysorbate 80) in a (collective) concentration of at most 1 mM; and/or
   - is (substantially or entirely) free of phosphate buffering agents (e.g. sodium dihydrogen phosphate, disodium hydrogen phosphate) or comprises a phosphate buffer system in a concentration of at most 0.1 mM.

In an embodiment, the liquid pharmaceutical composition comprises:
- 25 to about 75 mg/mL adalimumab;
- 2 to about 50 mM sodium acetate/acetic acid buffer system;
- 5 to 45 mg/mL inositol (or 25 mM to 250 mM inositol);
- 10 to about 200 mM sodium chloride;
- 0.001 to 2 mg/mL PVP having a weight average molecular weight of between 1900 and 3100; and
- water (for injection);
   wherein the composition:
   - has a pH between 5.0 and 5.5;
   - is (substantially or entirely) free of arginine (suitably L-arginine) or comprises arginine in a concentration of at most 0.1 mM;
   - is (substantially or entirely) free of amino acids or comprises one or more amino acids in a (collective) concentration of at most 0.1 mM;
   - is (substantially or entirely) free of surfactants with the exception of PVP having a weight average molecular weight of between 1900 and 3100 or comprises one or more of said surfactants (excluding PVP having a weight average molecular weight of between 1900 and 3100) in a (collective) concentration of at most 1 mM; and/or
   - is (substantially or entirely) free of phosphate buffering agents (e.g. sodium dihydrogen phosphate, disodium hydrogen phosphate) or comprises a phosphate buffer system in a concentration of at most 0.1 mM.

In an embodiment, the liquid pharmaceutical composition comprises:
- 25 to about 75 mg/mL adalimumab;
- 2 to about 50 mM sodium acetate/acetic acid buffer system;
- 5 to 45 mg/mL inositol (or 25 mM to 250 mM inositol);
- 10 to about 250 mM sodium gluconate;
- 0.001 to 2 mg/mL PVP having a weight average molecular weight of between 1900 and 3100; and
- water (for injection);
   wherein the composition:
   - has a pH between 5.0 and 5.5;
   - is (substantially or entirely) free of arginine (suitably L-arginine) or comprises arginine in a concentration of at most 0.1 mM;
   - is (substantially or entirely) free of amino acids or comprises one or more amino acids in a (collective) concentration of at most 0.1 mM;
   - is (substantially or entirely) free of surfactants with the exception of PVP having a weight average molecular weight of between 1900 and 3100 or comprises one or more of said surfactants (excluding PVP having a weight average molecular weight of between 1900 and 3100) in a (collective) concentration of at most 1 mM; and/or
   - is (substantially or entirely) free of phosphate buffering agents (e.g. sodium dihydrogen phosphate, disodium hydrogen phosphate) or comprises a phosphate buffer system in a concentration of at most 0.1 mM.

In an embodiment, the liquid pharmaceutical composition comprises:
- 40 to about 60 mg/mL adalimumab;
- 5 to about 15 mM sodium acetate/acetic acid buffer system;
- 175 to about 225 mM trehalose;
- 25 to about 75 mM sodium chloride;
- 0.01 to 1 mg/mL PVP having a weight average molecular weight of between 1900 and 3100; and
- water (for injection);
   wherein the composition:
   - has a pH between 5.1 and 5.3;
   - is (substantially or entirely) free of arginine (suitably L-arginine) or comprises arginine in a concentration of at most 0.001 mM;
   - is (substantially or entirely) free of amino acids or comprises one or more amino acids in a (collective) concentration of at most 0.001 mM.
   - is (substantially or entirely) free of surfactants with the exception of PVP having a weight average molecular weight of between 1900 and 3100 or comprises one or more of said surfactants (excluding PVP having a weight average molecular weight of between 1900 and 3100) in a (collective) concentration of at most 0.0001 mM; and/or
   - is (substantially or entirely) free of phosphate buffering agents (e.g. sodium dihydrogen phosphate, disodium hydrogen phosphate) or comprises a phosphate buffer system in a concentration of at most 0.001 mM.

In an embodiment, the liquid pharmaceutical composition comprises:
- 40 to about 60 mg/mL adalimumab;
- 5 to about 15 mM sodium acetate/acetic acid buffer system;
- 10 to 40 mg/mL inositol (or 55 mM to 225mM inositol);
- 25 to about 75 mM sodium chloride;
- 0.5 mg/mL to about 1.5 mg/mL polysorbate 80; and
- water (for injection);
   wherein the composition:
   - has a pH between 5.1 and 5.3;
   - is (substantially or entirely) free of arginine (suitably L-arginine) or comprises arginine in a concentration of at most 0.001 mM;
   - is (substantially or entirely) free of amino acids or comprises one or more amino acids in a (collective) concentration of at most 0.001 mM.
   - is (substantially or entirely) free of surfactants with the exception of polysorbate 80 or comprises one or more of said surfactants (excluding polysorbate 80) in a (collective) concentration of at most 0.0001 mM; and/or
   - is (substantially or entirely) free of phosphate buffering agents (e.g. sodium dihydrogen phosphate, disodium hydrogen phosphate) or comprises a phosphate buffer system in a concentration of at most 0.001 mM.

In an embodiment, the liquid pharmaceutical composition comprises:
- 40 to about 60 mg/mL adalimumab;
- 5 to about 15 mM sodium acetate/acetic acid buffer system;
- 10 to 40 mg/mL inositol (or 55 mM to 225mM inositol);
- 25 to about 75 mM sodium chloride;
- 0.01 to 1 mg/mL PVP having a weight average molecular weight of between 1900
   and 3100; and
- water (for injection);
   wherein the composition:
   - has a pH between 5.1 and 5.3;
   - is (substantially or entirely) free of arginine (suitably L-arginine) or comprises arginine in a concentration of at most 0.001 mM;
   - is (substantially or entirely) free of amino acids or comprises one or more amino acids in a (collective) concentration of at most 0.001 mM.
   - is (substantially or entirely) free of surfactants with the exception of PVP having a weight average molecular weight of between 1900 and 3100 or comprises one or more of said surfactants (excluding PVP having a weight average molecular weight of between 1900 and 3100) in a (collective) concentration of at most 0.0001 mM; and/or
   - is (substantially or entirely) free of phosphate buffering agents (e.g. sodium dihydrogen phosphate, disodium hydrogen phosphate) or comprises a phosphate buffer system in a concentration of at most 0.001 mM.

In an embodiment, the liquid pharmaceutical composition comprises:
- 40 to about 60 mg/mL adalimumab;
- 5 to about 15 mM sodium acetate/acetic acid buffer system;
- 10 to 40 mg/mL inositol (or 55 mM to 225mM inositol);
- 45 mM and 230 mM sodium gluconate;
- 0.01 to 1 mg/mL PVP having a weight average molecular weight of between 1900 and 3100; and
- water (for injection);
   wherein the composition:
   - has a pH between 5.1 and 5.3;
   - is (substantially or entirely) free of arginine (suitably L-arginine) or comprises arginine in a concentration of at most 0.001 mM;
   - is (substantially or entirely) free of amino acids or comprises one or more amino acids in a (collective) concentration of at most 0.001 mM.
   - is (substantially or entirely) free of surfactants with the exception of PVP having a weight average molecular weight of between 1900 and 3100 or comprises one or more of said surfactants (excluding PVP having a weight average molecular weight of between 1900 and 3100) in a (collective) concentration of at most 0.0001 mM; and/or
   - is (substantially or entirely) free of phosphate buffering agents (e.g. sodium dihydrogen phosphate, disodium hydrogen phosphate) or comprises a phosphate buffer system in a concentration of at most 0.001 mM.

In an embodiment, the liquid pharmaceutical composition comprises:
- 50 mg/mL adalimumab;
- 10 mM sodium acetate/acetic acid buffer system;
- 200 mM trehalose;
- 50 mM sodium chloride;
- 0.01 to 1 mg/mL PVP having a weight average molecular weight of between 1900 and 3100; and
- water (for injection);
   wherein the composition:
   o has a pH of 5.2;
   o is free of arginine;
   o is free of amino acids;
   o is free of surfactants with the exception of PVP having a weight average molecular weight of between 1900 and 3100; and
   o is free of phosphate buffering agents/systems.

In an embodiment, the liquid pharmaceutical composition comprises:
- 50 mg/mL adalimumab;
- 10 mM sodium acetate/acetic acid buffer system;
- 10 to 40 mg/mL inositol (or 55 mM to 225mM inositol);
- 50 mM sodium chloride;
- 1 mg/mL polysorbate 80; and
- water (for injection);
   wherein the composition:
   - has a pH of 5.2;
   - is free of arginine;
   - is free of amino acids;
   - is free of surfactants with the exception of polysorbate 80; and
   - is free of phosphate buffering agents/systems.

In an embodiment, the liquid pharmaceutical composition comprises:
- 50 mg/mL adalimumab;
- 10 mM sodium acetate/acetic acid buffer system;
- 10 to 40 mg/mL inositol (or 55 mM to 225mM inositol);
- 50 mM sodium chloride;
- 0.01 to 1 mg/mL PVP having a weight average molecular weight of between 1900 and 3100; and
- water (for injection);
   wherein the composition:
   - has a pH of 5.2;
   - is free of arginine;
   - is free of amino acids;
   - is free of surfactants with the exception of PVP having a weight average molecular weight of between 1900 and 3100; and
   - is free of phosphate buffering agents/systems.

In an embodiment, the liquid pharmaceutical composition comprises:
- 50 mg/mL adalimumab;
- 10 mM sodium acetate/acetic acid buffer system;
- 10 to 40 mg/mL inositol (or 55 mM to 225mM inositol);
- 45 mM and 230 mM sodium gluconate;
- 0.01 to 1 mg/mL PVP having a weight average molecular weight of between 1900 and 3100; and
- water (for injection);
   wherein the composition:
   - has a pH of 5.2;
   - is free of arginine;
   - is free of amino acids;
   - is free of surfactants with the exception of PVP having a weight average molecular weight of between 1900 and 3100; and
   - is free of phosphate buffering agents/systems.

In an embodiment, the liquid pharmaceutical composition consists essentially of:
- 25 to about 75 mg/mL adalimumab;
- 2 to about 50 mM sodium acetate/acetic acid buffer system;
- 100 to about 300 mM trehalose;
- 10 to about 200 mM sodium chloride;
- 0.001 to 2 mg/mL PVP having a weight average molecular weight of between 1900 and 3100; and
- water (for injection);
wherein the composition has a pH between 5.0 and 5.5.

In an embodiment, the liquid pharmaceutical composition consists essentially of:
- 25 to about 75 mg/mL adalimumab;
- 2 to about 50 mM sodium acetate/acetic acid buffer system;
- 5 to 45 mg/mL inositol (or 25 mM to 250 mM inositol);
- 10 to about 200 mM sodium chloride;
- 0.001 mg/mL to about 5 mg/mL polysorbate 80; and
- water (for injection);
wherein the composition has a pH between 5.0 and 5.5.

In an embodiment, the liquid pharmaceutical composition consists essentially of:
- 25 to about 75 mg/mL adalimumab;
- 2 to about 50 mM sodium acetate/acetic acid buffer system;
- 5 to 45 mg/mL inositol (or 25 mM to 250 mM inositol);
- 10 to about 200 mM sodium chloride;
- 0.001 to 2 mg/mL PVP having a weight average molecular weight of between 1900 and 3100; and
- water (for injection);
wherein the composition has a pH between 5.0 and 5.5.

In an embodiment, the liquid pharmaceutical composition consists essentially of:
- 25 to about 75 mg/mL adalimumab;
- 2 to about 50 mM sodium acetate/acetic acid buffer system;
- 5 to 45 mg/mL inositol (or 25 mM to 250 mM inositol);
- 10 to about 250 mM sodium gluconate;
- 0.001 to 2 mg/mL PVP having a weight average molecular weight of between 1900 and 3100; and
- water (for injection);
wherein the composition has a pH between 5.0 and 5.5.

In an embodiment, the liquid pharmaceutical composition consists essentially of:
- 40 to about 60 mg/mL adalimumab;
- 5 to about 15 mM sodium acetate/acetic acid buffer system;
- 175 to about 225 mM trehalose;
- 25 to about 75 mM sodium chloride;
- 0.01 to 1 mg/mL PVP having a weight average molecular weight of between 1900 and 3100; and
- water (for injection);
wherein the composition has a pH of between 5.1 and 5.3.

In an embodiment, the liquid pharmaceutical composition consists essentially of:
- 40 to about 60 mg/mL adalimumab;
- 5 to about 15 mM sodium acetate/acetic acid buffer system;
- 10 to 40 mg/mL inositol (or 55 mM to 225mM inositol);
- 25 to about 75 mM sodium chloride;
- 0.5 mg/mL to about 1.5 mg/mL polysorbate 80; and
- water (for injection);
wherein the composition has a pH of between 5.1 and 5.3.

In an embodiment, the liquid pharmaceutical composition consists essentially of:
- 40 to about 60 mg/mL adalimumab;
- 5 to about 15 mM sodium acetate/acetic acid buffer system;
- 10 to 40 mg/mL inositol (or 55 mM to 225mM inositol);
- 25 to about 75 mM sodium chloride;
- 0.01 to 1 mg/mL PVP having a weight average molecular weight of between 1900 and 3100; and
- water (for injection);
wherein the composition has a pH of between 5.1 and 5.3.

In an embodiment, the liquid pharmaceutical composition consists essentially of:
- 40 to about 60 mg/mL adalimumab;
- 5 to about 15 mM sodium acetate/acetic acid buffer system;
- 10 to 40 mg/mL inositol (or 55 mM to 225mM inositol);
- 45 mM and 230 mM sodium gluconate;
- 0.01 to 1 mg/mL PVP having a weight average molecular weight of between 1900 and 3100; and
- water (for injection);
wherein the composition has a pH of between 5.1 and 5.3.

In an embodiment, the liquid pharmaceutical composition consists essentially of:
- 50 mg/mL adalimumab;
- 10 mM sodium acetate/acetic acid buffer system;
- 200 mM trehalose;
- 50 mM sodium chloride;
- 0.01 to 1 mg/mL PVP having a weight average molecular weight of between 1900 and 3100; and
- water (for injection);
wherein the composition has a pH of 5.2.

In an embodiment, the liquid pharmaceutical composition consists essentially of:
- 50 mg/mL adalimumab;
- 10 mM sodium acetate/acetic acid buffer system;
- 10 to 40 mg/mL inositol (or 55 mM to 225mM inositol);
- 50 mM sodium chloride;
- 1 mg/mL polysorbate 80; and
- water (for injection);
wherein the composition has a pH of 5.2.

In an embodiment, the liquid pharmaceutical composition consists essentially of:
- 50 mg/mL adalimumab;
- 10 mM sodium acetate/acetic acid buffer system;
- 10 to 40 mg/mL inositol (or 55 mM to 225mM inositol);
- 50 mM sodium chloride;
- 0.01 to 1 mg/mL PVP having a weight average molecular weight of between 1900 and 3100; and
- water (for injection);
wherein the composition has a pH of 5.2.

In an embodiment, the liquid pharmaceutical composition consists essentially of:
- 50 mg/mL adalimumab;
- 10 mM sodium acetate/acetic acid buffer system;
- 10 to 40 mg/mL inositol (or 55 mM to 225mM inositol);
- 45 mM and 230 mM sodium gluconate;
- 0.01 to 1 mg/mL PVP having a weight average molecular weight of between 1900 and 3100; and
- water (for injection);
wherein the composition has a pH of 5.2.

In an embodiment, the liquid pharmaceutical composition consists essentially of:
- 50 mg/mL adalimumab;
- 10 mM sodium acetate/acetic acid buffer system;
- 200 mM trehalose;
- 50 mM sodium chloride;
- 0.01 to 1 mg/mL PVP having a weight average molecular weight of between 1900 and 3100; and
- water (for injection);
wherein the osmolality of the liquid pharmaceutical composition is between 200 and 400 mOsm/kg; and
wherein the composition has a pH of 5.2.

In an embodiment, the liquid pharmaceutical composition consists essentially of:
- 50 mg/mL adalimumab;
- 10 mM sodium acetate/acetic acid buffer system;
- 10 to 40 mg/mL inositol (or 55 mM to 225mM inositol);
- 50 mM sodium chloride;
- 1 mg/mL polysorbate 80; and
- water (for injection);
wherein the osmolality of the liquid pharmaceutical composition is between 200 and 400 mOsm/kg; and
wherein the composition has a pH of 5.2.

In an embodiment, the liquid pharmaceutical composition consists essentially of:
- 50 mg/mL adalimumab;
- 10 mM sodium acetate/acetic acid buffer system;
- 10 to 40 mg/mL inositol (or 55 mM to 225mM inositol);
- 50 mM sodium chloride;
- 0.01 to 1 mg/mL PVP having a weight average molecular weight of between 1900 and 3100; and
- water (for injection);
wherein the osmolality of the liquid pharmaceutical composition is between 200 and 400 mOsm/kg; and
wherein the composition has a pH of 5.2.

In an embodiment, the liquid pharmaceutical composition consists essentially of:
- 50 mg/mL adalimumab;
- 10 mM sodium acetate/acetic acid buffer system;
- 10 to 40 mg/mL inositol (or 55 mM to 225mM inositol);
- 45 mM and 230 mM sodium gluconate;
- 0.01 to 1 mg/mL PVP having a weight average molecular weight of between 1900 and 3100; and
- water (for injection);
wherein the osmolality of the liquid pharmaceutical composition is between 200 and 400 mOsm/kg; and
wherein the composition has a pH of 5.2.

Any amounts, concentrations, relative ratios, or ranges relating thereto (whether by weight or moles) disclosed hereinbefore with respect to a component that is stipulated in one of the foregoing specific embodiments may suitably replace any amounts, concentrations, relative ratios, or ranges relating thereto stipulated for the said component in the said embodiment, especially where the amounts, concentrations, relative ratios, or ranges relating thereto being replaced are broader in scope than the replacement amounts, concentrations, relative ratios, or ranges relating thereto. Where the amounts, concentrations, relative ratios, or ranges relating thereto (whether by weight or moles) for a given component are not specifically stipulated (or limited) in one of the foregoing specific embodiments, any amounts, concentrations, relative ratios, or ranges relating thereto (whether by weight or moles) disclosed hereinbefore with respect to that component may optionally be stipulated.

Likewise, any generically-defined component stipulated in relation to one of the foregoing specific embodiments may be suitably replaced by any of the more specific definitions disclosed herein in relation to said generically-defined component.

Suitably, the liquid pharmaceutical composition may be as set forth in any of the preceding embodiments, except that the absence or low levels of component(s) such as arginine, amino acids, surfactants (optionally with the exception of polysorbate 80), and phosphate buffering agents/systems, rather than being defined by reference to concentration(s) (i.e. molarity) may instead be defined by reference to corresponding molar ratios of the component to buffer system; corresponding weight ratios of the component to adalimumab; or corresponding molar ratios of the component to adalimumab. The skilled person will readily deduce for each component, from the relevant section of this specification relating to that specific component, which molar and weight ratios correspond to which concentrations, since herein the relevant molar and weight ratios are listed to respectively correspond to given concentrations. For example, in the case of arginine, the optional concentrations of "at most 0.1 mM, more suitably at most 0.01mM, most suitably at most 0.001 mM" respectively correspond with a molar ratio of arginine to buffer system of "at most 1 : 150...more suitably at most 1:1500, most suitably at most 1:15,000"; with "a weight ratio of arginine to adalimumab of at most 1 : 3000...more suitably at most 1:30,000, most suitably at most 1:300,000"; and with a molar ratio of arginine to adalimumab of at most 1 : 3.75...more suitably at most 1:37.5, most suitably at most 1:375". The same correspondences apply for amino acids, surfactants, and phosphate buffering agents/systems. The same applies *mutatis mutandis* to any components stipulated as being present within the compositions.

### Further Specific Embodiments

The liquid pharmaceutical composition suitably comprises or is characterised by adalimumab.

The liquid pharmaceutical composition suitably comprises or is characterised by a buffer system; suitably wherein the buffer system is selected from the group consisting of: 5 to about 15 mM buffer system, and 10 mM acetate buffer system.

The liquid pharmaceutical composition suitably comprises or is characterised by a sugar stabiliser; suitably wherein the sugar stabiliser is selected from the group consisting of: a monocyclic sugar stabiliser, either inositol or trehalose, Inositol, 10 to 40 mg/mL monocyclic sugar stabiliser, and 10 to 40 mg/mL Inositol.

The liquid pharmaceutical composition suitably comprises or is characterised by a tonicifier; suitably wherein the tonicifier is selected from the group consisting of: a metal (hydroxy)mono-/di-carboxylate salt tonicifier, either sodium gluconate or sodium chloride, and either 45 mM and 230 mM sodium gluconate or 25 mM to 75 mM sodium chloride.

The liquid pharmaceutical composition suitably comprises or is characterised by a surfactant; suitably wherein the surfactant is selected from the group consisting of: a PVP surfactant, a PVP surfactant having a weight average molecular weight of between 1900 and 3100 or Polysorbate 80, 0.01 to 2 mg/mL of either a PVP surfactant or Polysorbate 80, and 0.01 to 1 mg/mL PVP surfactant having a weight average molecular weight of between 1900 and 3100.

The liquid pharmaceutical composition suitably comprises or is characterised by wherein the composition has a pH between 5.0 and 5.5.

In a particular embodiment, the liquid pharmaceutical composition comprises or is characterised by adalimumab, and the liquid pharmaceutical composition further comprises or is further characterised by: wherein the composition has a pH between 5.0 and 5.5.

In a particular embodiment, the liquid pharmaceutical composition comprises or is characterised by adalimumab, and the liquid pharmaceutical composition further comprises or is further characterised by: a surfactant.

In a particular embodiment, the liquid pharmaceutical composition comprises or is characterised by adalimumab, and the liquid pharmaceutical composition further comprises or is further characterised by: a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a PVP surfactant.

In a particular embodiment, the liquid pharmaceutical composition comprises or is characterised by adalimumab, and the liquid pharmaceutical composition further comprises or is further characterised by: a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a tonicifier.

In a particular embodiment, the liquid pharmaceutical composition comprises or is characterised by adalimumab, and the liquid pharmaceutical composition further comprises or is further characterised by: a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a tonicifier, and a surfactant.

In a particular embodiment, the liquid pharmaceutical composition comprises or is characterised by adalimumab, and the liquid pharmaceutical composition further comprises or is further characterised by: a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a tonicifier, and a PVP surfactant.

In a particular embodiment, the liquid pharmaceutical composition comprises or is characterised by adalimumab, and the liquid pharmaceutical composition further comprises or is further characterised by: a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: either sodium gluconate or sodium chloride.

In a particular embodiment, the liquid pharmaceutical composition comprises or is characterised by adalimumab, and the liquid pharmaceutical composition further comprises or is further characterised by: either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: either sodium gluconate or sodium chloride, and a surfactant.

In a particular embodiment, the liquid pharmaceutical composition comprises or is characterised by adalimumab, and the liquid pharmaceutical composition further comprises or is further characterised by: either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser.

In a particular embodiment, the liquid pharmaceutical composition comprises or is characterised by adalimumab, and the liquid pharmaceutical composition further comprises or is further characterised by: a sugar stabiliser, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, and a surfactant.

In a particular embodiment, the liquid pharmaceutical composition comprises or is characterised by adalimumab, and the liquid pharmaceutical composition further comprises or is further characterised by: a sugar stabiliser, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, and a PVP surfactant.

In a particular embodiment, the liquid pharmaceutical composition comprises or is characterised by adalimumab, and the liquid pharmaceutical composition further comprises or is further characterised by: a sugar stabiliser, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a sugar stabiliser, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a sugar stabiliser, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a sugar stabiliser, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, and a tonicifier.

In a particular embodiment, the liquid pharmaceutical composition comprises or is characterised by adalimumab, and the liquid pharmaceutical composition further comprises or is further characterised by: a sugar stabiliser, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, a tonicifier, and a surfactant.

In a particular embodiment, the liquid pharmaceutical composition comprises or is characterised by adalimumab, and the liquid pharmaceutical composition further comprises or is further characterised by: a sugar stabiliser, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, a tonicifier, and a PVP surfactant.

In a particular embodiment, the liquid pharmaceutical composition comprises or is characterised by adalimumab, and the liquid pharmaceutical composition further comprises or is further characterised by: a sugar stabiliser, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a sugar stabiliser, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a sugar stabiliser, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a sugar stabiliser, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: a sugar stabiliser, a metal (hydroxy)mono-/dicarboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, a metal (hydroxy)mono-/dicarboxylate salt tonicifier, and a surfactant.

In a particular embodiment, the liquid pharmaceutical composition comprises or is characterised by adalimumab, and the liquid pharmaceutical composition further comprises or is further characterised by: a sugar stabiliser, a metal (hydroxy)mono-/dicarboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, a metal (hydroxy)mono-/dicarboxylate salt tonicifier, and a PVP surfactant.

In a particular embodiment, the liquid pharmaceutical composition comprises or is characterised by adalimumab, and the liquid pharmaceutical composition further comprises or is further characterised by: a sugar stabiliser, a metal (hydroxy)mono-/dicarboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, a metal (hydroxy)mono-/dicarboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a sugar stabiliser, a metal (hydroxy)mono-/dicarboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, a metal (hydroxy)mono-/dicarboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a sugar stabiliser, a metal (hydroxy)mono-/dicarboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, a metal (hydroxy)mono-/dicarboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a sugar stabiliser, a metal (hydroxy)mono-/dicarboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, and either sodium gluconate or sodium chloride.

In a particular embodiment, the liquid pharmaceutical composition comprises or is characterised by adalimumab, and the liquid pharmaceutical composition further comprises or is further characterised by: a sugar stabiliser, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, either sodium gluconate or sodium chloride, and a surfactant.

In a particular embodiment, the liquid pharmaceutical composition comprises or is characterised by adalimumab, and the liquid pharmaceutical composition further comprises or is further characterised by: a sugar stabiliser, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: a sugar stabiliser, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a sugar stabiliser, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a sugar stabiliser, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a sugar stabiliser, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: a sugar stabiliser, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: a sugar stabiliser, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: a sugar stabiliser, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a sugar stabiliser, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a sugar stabiliser, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a sugar stabiliser, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a sugar stabiliser, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser.

In a particular embodiment, the liquid pharmaceutical composition comprises or is characterised by adalimumab, and the liquid pharmaceutical composition further comprises or is further characterised by: a monocyclic sugar stabiliser, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, and a surfactant.

In a particular embodiment, the liquid pharmaceutical composition comprises or is characterised by adalimumab, and the liquid pharmaceutical composition further comprises or is further characterised by: a monocyclic sugar stabiliser, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, and a PVP surfactant.

In a particular embodiment, the liquid pharmaceutical composition comprises or is characterised by adalimumab, and the liquid pharmaceutical composition further comprises or is further characterised by: a monocyclic sugar stabiliser, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a monocyclic sugar stabiliser, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a monocyclic sugar stabiliser, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a monocyclic sugar stabiliser, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, and a tonicifier.

In a particular embodiment, the liquid pharmaceutical composition comprises or is characterised by adalimumab, and the liquid pharmaceutical composition further comprises or is further characterised by: a monocyclic sugar stabiliser, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, a tonicifier, and a surfactant.

In a particular embodiment, the liquid pharmaceutical composition comprises or is characterised by adalimumab, and the liquid pharmaceutical composition further comprises or is further characterised by: a monocyclic sugar stabiliser, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, a tonicifier, and a PVP surfactant.

In a particular embodiment, the liquid pharmaceutical composition comprises or is characterised by adalimumab, and the liquid pharmaceutical composition further comprises or is further characterised by: a monocyclic sugar stabiliser, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a monocyclic sugar stabiliser, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a monocyclic sugar stabiliser, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a monocyclic sugar stabiliser, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: a monocyclic sugar stabiliser, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: a monocyclic sugar stabiliser, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: a monocyclic sugar stabiliser, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a monocyclic sugar stabiliser, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a monocyclic sugar stabiliser, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a monocyclic sugar stabiliser, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, and either sodium gluconate or sodium chloride.

In a particular embodiment, the liquid pharmaceutical composition comprises or is characterised by adalimumab, and the liquid pharmaceutical composition further comprises or is further characterised by: a monocyclic sugar stabiliser, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, either sodium gluconate or sodium chloride, and a surfactant.

In a particular embodiment, the liquid pharmaceutical composition comprises or is characterised by adalimumab, and the liquid pharmaceutical composition further comprises or is further characterised by: a monocyclic sugar stabiliser, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: a monocyclic sugar stabiliser, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a monocyclic sugar stabiliser, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a monocyclic sugar stabiliser, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a monocyclic sugar stabiliser, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: a monocyclic sugar stabiliser, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: a monocyclic sugar stabiliser, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: a monocyclic sugar stabiliser, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a monocyclic sugar stabiliser, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a monocyclic sugar stabiliser, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a monocyclic sugar stabiliser, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a monocyclic sugar stabiliser, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose.

In a particular embodiment, the liquid pharmaceutical composition comprises or is characterised by adalimumab, and the liquid pharmaceutical composition further comprises or is further characterised by: either inositol or trehalose, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, and a surfactant.

In a particular embodiment, the liquid pharmaceutical composition comprises or is characterised by adalimumab, and the liquid pharmaceutical composition further comprises or is further characterised by: either inositol or trehalose, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, and a PVP surfactant.

In a particular embodiment, the liquid pharmaceutical composition comprises or is characterised by adalimumab, and the liquid pharmaceutical composition further comprises or is further characterised by: either inositol or trehalose, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: either inositol or trehalose, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: either inositol or trehalose, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: either inositol or trehalose, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, and a tonicifier.

In a particular embodiment, the liquid pharmaceutical composition comprises or is characterised by adalimumab, and the liquid pharmaceutical composition further comprises or is further characterised by: either inositol or trehalose, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, a tonicifier, and a surfactant.

In a particular embodiment, the liquid pharmaceutical composition comprises or is characterised by adalimumab, and the liquid pharmaceutical composition further comprises or is further characterised by: either inositol or trehalose, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, a tonicifier, and a PVP surfactant.

In a particular embodiment, the liquid pharmaceutical composition comprises or is characterised by adalimumab, and the liquid pharmaceutical composition further comprises or is further characterised by: either inositol or trehalose, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: either inositol or trehalose, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: either inositol or trehalose, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: either inositol or trehalose, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: either inositol or trehalose, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: either inositol or trehalose, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: either inositol or trehalose, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: either inositol or trehalose, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: either inositol or trehalose, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: either inositol or trehalose, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, and either sodium gluconate or sodium chloride.

In a particular embodiment, the liquid pharmaceutical composition comprises or is characterised by adalimumab, and the liquid pharmaceutical composition further comprises or is further characterised by: either inositol or trehalose, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, either sodium gluconate or sodium chloride, and a surfactant.

In a particular embodiment, the liquid pharmaceutical composition comprises or is characterised by adalimumab, and the liquid pharmaceutical composition further comprises or is further characterised by: either inositol or trehalose, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: either inositol or trehalose, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: either inositol or trehalose, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: either inositol or trehalose, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: either inositol or trehalose, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: either inositol or trehalose, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: either inositol or trehalose, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: either inositol or trehalose, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: either inositol or trehalose, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: either inositol or trehalose, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: either inositol or trehalose, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: either inositol or trehalose, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol.

In a particular embodiment, the liquid pharmaceutical composition comprises or is characterised by adalimumab, and the liquid pharmaceutical composition further comprises or is further characterised by: Inositol, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, and a surfactant.

In a particular embodiment, the liquid pharmaceutical composition comprises or is characterised by adalimumab, and the liquid pharmaceutical composition further comprises or is further characterised by: Inositol, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, and a PVP surfactant.

In a particular embodiment, the liquid pharmaceutical composition comprises or is characterised by adalimumab, and the liquid pharmaceutical composition further comprises or is further characterised by: Inositol, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: Inositol, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: Inositol, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: Inositol, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, and a tonicifier.

In a particular embodiment, the liquid pharmaceutical composition comprises or is characterised by adalimumab, and the liquid pharmaceutical composition further comprises or is further characterised by: Inositol, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, a tonicifier, and a surfactant.

In a particular embodiment, the liquid pharmaceutical composition comprises or is characterised by adalimumab, and the liquid pharmaceutical composition further comprises or is further characterised by: Inositol, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, a tonicifier, and a PVP surfactant.

In a particular embodiment, the liquid pharmaceutical composition comprises or is characterised by adalimumab, and the liquid pharmaceutical composition further comprises or is further characterised by: Inositol, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: Inositol, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: Inositol, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: Inositol, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, and a metal (hydroxy)mono-/dicarboxylate salt tonicifier.

In a particular embodiment, the liquid pharmaceutical composition comprises or is characterised by adalimumab, and the liquid pharmaceutical composition further comprises or is further characterised by: Inositol, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: Inositol, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: Inositol, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: Inositol, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: Inositol, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: Inositol, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, and either sodium gluconate or sodium chloride.

In a particular embodiment, the liquid pharmaceutical composition comprises or is characterised by adalimumab, and the liquid pharmaceutical composition further comprises or is further characterised by: Inositol, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, either sodium gluconate or sodium chloride, and a surfactant.

In a particular embodiment, the liquid pharmaceutical composition comprises or is characterised by adalimumab, and the liquid pharmaceutical composition further comprises or is further characterised by: Inositol, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: Inositol, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: Inositol, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: Inositol, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: Inositol, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: Inositol, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: Inositol, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: Inositol, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: Inositol, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: Inositol, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: Inositol, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: Inositol, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, and a surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, and a PVP surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, and a tonicifier.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a tonicifier, and a surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a tonicifier, and a PVP surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, and either sodium gluconate or sodium chloride.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either sodium gluconate or sodium chloride, and a surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol.

In a particular embodiment, the liquid pharmaceutical composition comprises or is characterised by adalimumab, and the liquid pharmaceutical composition further comprises or is further characterised by: 10 to 40 mg/mL Inositol, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, and a surfactant.

In a particular embodiment, the liquid pharmaceutical composition comprises or is characterised by adalimumab, and the liquid pharmaceutical composition further comprises or is further characterised by: 10 to 40 mg/mL Inositol, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, and a PVP surfactant.

In a particular embodiment, the liquid pharmaceutical composition comprises or is characterised by adalimumab, and the liquid pharmaceutical composition further comprises or is further characterised by: 10 to 40 mg/mL Inositol, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 to 40 mg/mL Inositol, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 to 40 mg/mL Inositol, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL Inositol, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, and a tonicifier.

In a particular embodiment, the liquid pharmaceutical composition comprises or is characterised by adalimumab, and the liquid pharmaceutical composition further comprises or is further characterised by: 10 to 40 mg/mL Inositol, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, a tonicifier, and a surfactant.

In a particular embodiment, the liquid pharmaceutical composition comprises or is characterised by adalimumab, and the liquid pharmaceutical composition further comprises or is further characterised by: 10 to 40 mg/mL Inositol, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, a tonicifier, and a PVP surfactant.

In a particular embodiment, the liquid pharmaceutical composition comprises or is characterised by adalimumab, and the liquid pharmaceutical composition further comprises or is further characterised by: 10 to 40 mg/mL Inositol, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 to 40 mg/mL Inositol, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 to 40 mg/mL Inositol, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL Inositol, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 10 to 40 mg/mL Inositol, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: 10 to 40 mg/mL Inositol, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 10 to 40 mg/mL Inositol, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 to 40 mg/mL Inositol, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 to 40 mg/mL Inositol, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL Inositol, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, and either sodium gluconate or sodium chloride.

In a particular embodiment, the liquid pharmaceutical composition comprises or is characterised by adalimumab, and the liquid pharmaceutical composition further comprises or is further characterised by: 10 to 40 mg/mL Inositol, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, either sodium gluconate or sodium chloride, and a surfactant.

In a particular embodiment, the liquid pharmaceutical composition comprises or is characterised by adalimumab, and the liquid pharmaceutical composition further comprises or is further characterised by: 10 to 40 mg/mL Inositol, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: 10 to 40 mg/mL Inositol, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 to 40 mg/mL Inositol, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 to 40 mg/mL Inositol, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL Inositol, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: 10 to 40 mg/mL Inositol, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: 10 to 40 mg/mL Inositol, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: 10 to 40 mg/mL Inositol, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 to 40 mg/mL Inositol, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 to 40 mg/mL Inositol, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 to 40 mg/mL Inositol, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 to 40 mg/mL Inositol, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system.

In a particular embodiment, the liquid pharmaceutical composition comprises or is characterised by adalimumab, and the liquid pharmaceutical composition further comprises or is further characterised by: a buffer system, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, and a surfactant.

In a particular embodiment, the liquid pharmaceutical composition comprises or is characterised by adalimumab, and the liquid pharmaceutical composition further comprises or is further characterised by: a buffer system, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, and a PVP surfactant.

In a particular embodiment, the liquid pharmaceutical composition comprises or is characterised by adalimumab, and the liquid pharmaceutical composition further comprises or is further characterised by: a buffer system, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, and a tonicifier.

In a particular embodiment, the liquid pharmaceutical composition comprises or is characterised by adalimumab, and the liquid pharmaceutical composition further comprises or is further characterised by: a buffer system, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a tonicifier, and a surfactant.

In a particular embodiment, the liquid pharmaceutical composition comprises or is characterised by adalimumab, and the liquid pharmaceutical composition further comprises or is further characterised by: a buffer system, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a tonicifier, and a PVP surfactant.

In a particular embodiment, the liquid pharmaceutical composition comprises or is characterised by adalimumab, and the liquid pharmaceutical composition further comprises or is further characterised by: a buffer system, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, and a metal (hydroxy)mono-/dicarboxylate salt tonicifier.

In a particular embodiment, the liquid pharmaceutical composition comprises or is characterised by adalimumab, and the liquid pharmaceutical composition further comprises or is further characterised by: a buffer system, a metal (hydroxy)mono-/dicarboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a metal (hydroxy)mono-/dicarboxylate salt tonicifier, and a surfactant.

In a particular embodiment, the liquid pharmaceutical composition comprises or is characterised by adalimumab, and the liquid pharmaceutical composition further comprises or is further characterised by: a buffer system, a metal (hydroxy)mono-/dicarboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a metal (hydroxy)mono-/dicarboxylate salt tonicifier, and a PVP surfactant.

In a particular embodiment, the liquid pharmaceutical composition comprises or is characterised by adalimumab, and the liquid pharmaceutical composition further comprises or is further characterised by: a buffer system, a metal (hydroxy)mono-/dicarboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a metal (hydroxy)mono-/dicarboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, a metal (hydroxy)mono-/dicarboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a metal (hydroxy)mono-/dicarboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, a metal (hydroxy)mono-/dicarboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a metal (hydroxy)mono-/dicarboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, a metal (hydroxy)mono-/dicarboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, and either sodium gluconate or sodium chloride.

In a particular embodiment, the liquid pharmaceutical composition comprises or is characterised by adalimumab, and the liquid pharmaceutical composition further comprises or is further characterised by: a buffer system, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either sodium gluconate or sodium chloride, and a surfactant.

In a particular embodiment, the liquid pharmaceutical composition comprises or is characterised by adalimumab, and the liquid pharmaceutical composition further comprises or is further characterised by: a buffer system, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: a buffer system, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: a buffer system, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: a buffer system, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: a buffer system, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, and a sugar stabiliser.

In a particular embodiment, the liquid pharmaceutical composition comprises or is characterised by adalimumab, and the liquid pharmaceutical composition further comprises or is further characterised by: a buffer system, a sugar stabiliser, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, and a surfactant.

In a particular embodiment, the liquid pharmaceutical composition comprises or is characterised by adalimumab, and the liquid pharmaceutical composition further comprises or is further characterised by: a buffer system, a sugar stabiliser, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, and a PVP surfactant.

In a particular embodiment, the liquid pharmaceutical composition comprises or is characterised by adalimumab, and the liquid pharmaceutical composition further comprises or is further characterised by: a buffer system, a sugar stabiliser, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, a sugar stabiliser, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, a sugar stabiliser, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, a sugar stabiliser, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, and a tonicifier.

In a particular embodiment, the liquid pharmaceutical composition comprises or is characterised by adalimumab, and the liquid pharmaceutical composition further comprises or is further characterised by: a buffer system, a sugar stabiliser, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, a tonicifier, and a surfactant.

In a particular embodiment, the liquid pharmaceutical composition comprises or is characterised by adalimumab, and the liquid pharmaceutical composition further comprises or is further characterised by: a buffer system, a sugar stabiliser, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, a tonicifier, and a PVP surfactant.

In a particular embodiment, the liquid pharmaceutical composition comprises or is characterised by adalimumab, and the liquid pharmaceutical composition further comprises or is further characterised by: a buffer system, a sugar stabiliser, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, a sugar stabiliser, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, a sugar stabiliser, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, a sugar stabiliser, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: a buffer system, a sugar stabiliser, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: a buffer system, a sugar stabiliser, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: a buffer system, a sugar stabiliser, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, a sugar stabiliser, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, a sugar stabiliser, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, a sugar stabiliser, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, and either sodium gluconate or sodium chloride.

In a particular embodiment, the liquid pharmaceutical composition comprises or is characterised by adalimumab, and the liquid pharmaceutical composition further comprises or is further characterised by: a buffer system, a sugar stabiliser, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, either sodium gluconate or sodium chloride, and a surfactant.

In a particular embodiment, the liquid pharmaceutical composition comprises or is characterised by adalimumab, and the liquid pharmaceutical composition further comprises or is further characterised by: a buffer system, a sugar stabiliser, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: a buffer system, a sugar stabiliser, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, a sugar stabiliser, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, a sugar stabiliser, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, a sugar stabiliser, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: a buffer system, a sugar stabiliser, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: a buffer system, a sugar stabiliser, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: a buffer system, a sugar stabiliser, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, a sugar stabiliser, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, a sugar stabiliser, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a sugar stabiliser, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, a sugar stabiliser, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, and a monocyclic sugar stabiliser.

In a particular embodiment, the liquid pharmaceutical composition comprises or is characterised by adalimumab, and the liquid pharmaceutical composition further comprises or is further characterised by: a buffer system, a monocyclic sugar stabiliser, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, and a surfactant.

In a particular embodiment, the liquid pharmaceutical composition comprises or is characterised by adalimumab, and the liquid pharmaceutical composition further comprises or is further characterised by: a buffer system, a monocyclic sugar stabiliser, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, and a PVP surfactant.

In a particular embodiment, the liquid pharmaceutical composition comprises or is characterised by adalimumab, and the liquid pharmaceutical composition further comprises or is further characterised by: a buffer system, a monocyclic sugar stabiliser, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, a monocyclic sugar stabiliser, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, a monocyclic sugar stabiliser, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, a monocyclic sugar stabiliser, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, and a tonicifier.

In a particular embodiment, the liquid pharmaceutical composition comprises or is characterised by adalimumab, and the liquid pharmaceutical composition further comprises or is further characterised by: a buffer system, a monocyclic sugar stabiliser, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, a tonicifier, and a surfactant.

In a particular embodiment, the liquid pharmaceutical composition comprises or is characterised by adalimumab, and the liquid pharmaceutical composition further comprises or is further characterised by: a buffer system, a monocyclic sugar stabiliser, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, a tonicifier, and a PVP surfactant.

In a particular embodiment, the liquid pharmaceutical composition comprises or is characterised by adalimumab, and the liquid pharmaceutical composition further comprises or is further characterised by: a buffer system, a monocyclic sugar stabiliser, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, a monocyclic sugar stabiliser, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, a monocyclic sugar stabiliser, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, a monocyclic sugar stabiliser, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: a buffer system, a monocyclic sugar stabiliser, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: a buffer system, a monocyclic sugar stabiliser, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: a buffer system, a monocyclic sugar stabiliser, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, a monocyclic sugar stabiliser, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, a monocyclic sugar stabiliser, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, a monocyclic sugar stabiliser, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, and either sodium gluconate or sodium chloride.

In a particular embodiment, the liquid pharmaceutical composition comprises or is characterised by adalimumab, and the liquid pharmaceutical composition further comprises or is further characterised by: a buffer system, a monocyclic sugar stabiliser, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, either sodium gluconate or sodium chloride, and a surfactant.

In a particular embodiment, the liquid pharmaceutical composition comprises or is characterised by adalimumab, and the liquid pharmaceutical composition further comprises or is further characterised by: a buffer system, a monocyclic sugar stabiliser, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: a buffer system, a monocyclic sugar stabiliser, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, a monocyclic sugar stabiliser, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, a monocyclic sugar stabiliser, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, a monocyclic sugar stabiliser, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: a buffer system, a monocyclic sugar stabiliser, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: a buffer system, a monocyclic sugar stabiliser, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: a buffer system, a monocyclic sugar stabiliser, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, a monocyclic sugar stabiliser, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, a monocyclic sugar stabiliser, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, a monocyclic sugar stabiliser, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, a monocyclic sugar stabiliser, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, and either inositol or trehalose.

In a particular embodiment, the liquid pharmaceutical composition comprises or is characterised by adalimumab, and the liquid pharmaceutical composition further comprises or is further characterised by: a buffer system, either inositol or trehalose, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, and a surfactant.

In a particular embodiment, the liquid pharmaceutical composition comprises or is characterised by adalimumab, and the liquid pharmaceutical composition further comprises or is further characterised by: a buffer system, either inositol or trehalose, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, and a PVP surfactant.

In a particular embodiment, the liquid pharmaceutical composition comprises or is characterised by adalimumab, and the liquid pharmaceutical composition further comprises or is further characterised by: a buffer system, either inositol or trehalose, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, either inositol or trehalose, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, either inositol or trehalose, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, either inositol or trehalose, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, and a tonicifier.

In a particular embodiment, the liquid pharmaceutical composition comprises or is characterised by adalimumab, and the liquid pharmaceutical composition further comprises or is further characterised by: a buffer system, either inositol or trehalose, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, a tonicifier, and a surfactant.

In a particular embodiment, the liquid pharmaceutical composition comprises or is characterised by adalimumab, and the liquid pharmaceutical composition further comprises or is further characterised by: a buffer system, either inositol or trehalose, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, a tonicifier, and a PVP surfactant.

In a particular embodiment, the liquid pharmaceutical composition comprises or is characterised by adalimumab, and the liquid pharmaceutical composition further comprises or is further characterised by: a buffer system, either inositol or trehalose, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, either inositol or trehalose, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, either inositol or trehalose, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, either inositol or trehalose, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: a buffer system, either inositol or trehalose, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: a buffer system, either inositol or trehalose, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: a buffer system, either inositol or trehalose, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, either inositol or trehalose, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, either inositol or trehalose, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, either inositol or trehalose, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, and either sodium gluconate or sodium chloride.

In a particular embodiment, the liquid pharmaceutical composition comprises or is characterised by adalimumab, and the liquid pharmaceutical composition further comprises or is further characterised by: a buffer system, either inositol or trehalose, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, either sodium gluconate or sodium chloride, and a surfactant.

In a particular embodiment, the liquid pharmaceutical composition comprises or is characterised by adalimumab, and the liquid pharmaceutical composition further comprises or is further characterised by: a buffer system, either inositol or trehalose, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: a buffer system, either inositol or trehalose, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, either inositol or trehalose, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, either inositol or trehalose, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, either inositol or trehalose, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: a buffer system, either inositol or trehalose, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: a buffer system, either inositol or trehalose, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: a buffer system, either inositol or trehalose, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, either inositol or trehalose, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, either inositol or trehalose, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, either inositol or trehalose, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, either inositol or trehalose, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, and Inositol.

In a particular embodiment, the liquid pharmaceutical composition comprises or is characterised by adalimumab, and the liquid pharmaceutical composition further comprises or is further characterised by: a buffer system, Inositol, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, and a surfactant.

In a particular embodiment, the liquid pharmaceutical composition comprises or is characterised by adalimumab, and the liquid pharmaceutical composition further comprises or is further characterised by: a buffer system, Inositol, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, and a PVP surfactant.

In a particular embodiment, the liquid pharmaceutical composition comprises or is characterised by adalimumab, and the liquid pharmaceutical composition further comprises or is further characterised by: a buffer system, Inositol, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, Inositol, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, Inositol, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, Inositol, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, and a tonicifier.

In a particular embodiment, the liquid pharmaceutical composition comprises or is characterised by adalimumab, and the liquid pharmaceutical composition further comprises or is further characterised by: a buffer system, Inositol, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, a tonicifier, and a surfactant.

In a particular embodiment, the liquid pharmaceutical composition comprises or is characterised by adalimumab, and the liquid pharmaceutical composition further comprises or is further characterised by: a buffer system, Inositol, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, a tonicifier, and a PVP surfactant.

In a particular embodiment, the liquid pharmaceutical composition comprises or is characterised by adalimumab, and the liquid pharmaceutical composition further comprises or is further characterised by: a buffer system, Inositol, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, Inositol, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, Inositol, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, Inositol, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: a buffer system, Inositol, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: a buffer system, Inositol, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: a buffer system, Inositol, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, Inositol, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, Inositol, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, Inositol, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, and either sodium gluconate or sodium chloride.

In a particular embodiment, the liquid pharmaceutical composition comprises or is characterised by adalimumab, and the liquid pharmaceutical composition further comprises or is further characterised by: a buffer system, Inositol, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, either sodium gluconate or sodium chloride, and a surfactant.

In a particular embodiment, the liquid pharmaceutical composition comprises or is characterised by adalimumab, and the liquid pharmaceutical composition further comprises or is further characterised by: a buffer system, Inositol, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: a buffer system, Inositol, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, Inositol, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, Inositol, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, Inositol, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: a buffer system, Inositol, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: a buffer system, Inositol, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: a buffer system, Inositol, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, Inositol, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, Inositol, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, Inositol, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, Inositol, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, and 10 to 40 mg/mL monocyclic sugar stabiliser.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, and a surfactant.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, and a PVP surfactant.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, and a tonicifier.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a tonicifier, and a surfactant.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a tonicifier, and a PVP surfactant.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, and either sodium gluconate or sodium chloride.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either sodium gluconate or sodium chloride, and a surfactant.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, 10 to 40 mg/mL monocyclic sugar stabiliser, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, and 10 to 40 mg/mL Inositol.

In a particular embodiment, the liquid pharmaceutical composition comprises or is characterised by adalimumab, and the liquid pharmaceutical composition further comprises or is further characterised by: a buffer system, 10 to 40 mg/mL Inositol, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, and a surfactant.

In a particular embodiment, the liquid pharmaceutical composition comprises or is characterised by adalimumab, and the liquid pharmaceutical composition further comprises or is further characterised by: a buffer system, 10 to 40 mg/mL Inositol, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, and a PVP surfactant.

In a particular embodiment, the liquid pharmaceutical composition comprises or is characterised by adalimumab, and the liquid pharmaceutical composition further comprises or is further characterised by: a buffer system, 10 to 40 mg/mL Inositol, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL Inositol, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL Inositol, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, 10 to 40 mg/mL Inositol, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, and a tonicifier.

In a particular embodiment, the liquid pharmaceutical composition comprises or is characterised by adalimumab, and the liquid pharmaceutical composition further comprises or is further characterised by: a buffer system, 10 to 40 mg/mL Inositol, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, a tonicifier, and a surfactant.

In a particular embodiment, the liquid pharmaceutical composition comprises or is characterised by adalimumab, and the liquid pharmaceutical composition further comprises or is further characterised by: a buffer system, 10 to 40 mg/mL Inositol, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, a tonicifier, and a PVP surfactant.

In a particular embodiment, the liquid pharmaceutical composition comprises or is characterised by adalimumab, and the liquid pharmaceutical composition further comprises or is further characterised by: a buffer system, 10 to 40 mg/mL Inositol, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL Inositol, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL Inositol, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, 10 to 40 mg/mL Inositol, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: a buffer system, 10 to 40 mg/mL Inositol, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: a buffer system, 10 to 40 mg/mL Inositol, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: a buffer system, 10 to 40 mg/mL Inositol, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL Inositol, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL Inositol, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, 10 to 40 mg/mL Inositol, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, and either sodium gluconate or sodium chloride.

In a particular embodiment, the liquid pharmaceutical composition comprises or is characterised by adalimumab, and the liquid pharmaceutical composition further comprises or is further characterised by: a buffer system, 10 to 40 mg/mL Inositol, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, either sodium gluconate or sodium chloride, and a surfactant.

In a particular embodiment, the liquid pharmaceutical composition comprises or is characterised by adalimumab, and the liquid pharmaceutical composition further comprises or is further characterised by: a buffer system, 10 to 40 mg/mL Inositol, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: a buffer system, 10 to 40 mg/mL Inositol, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL Inositol, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL Inositol, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, 10 to 40 mg/mL Inositol, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: a buffer system, 10 to 40 mg/mL Inositol, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: a buffer system, 10 to 40 mg/mL Inositol, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: a buffer system, 10 to 40 mg/mL Inositol, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL Inositol, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: a buffer system, 10 to 40 mg/mL Inositol, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: a buffer system, 10 to 40 mg/mL Inositol, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: a buffer system, 10 to 40 mg/mL Inositol, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system.

In a particular embodiment, the liquid pharmaceutical composition comprises or is characterised by adalimumab, and the liquid pharmaceutical composition further comprises or is further characterised by: 5 to about 15 mM buffer system, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, and a surfactant.

In a particular embodiment, the liquid pharmaceutical composition comprises or is characterised by adalimumab, and the liquid pharmaceutical composition further comprises or is further characterised by: 5 to about 15 mM buffer system, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, and a tonicifier.

In a particular embodiment, the liquid pharmaceutical composition comprises or is characterised by adalimumab, and the liquid pharmaceutical composition further comprises or is further characterised by: 5 to about 15 mM buffer system, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a tonicifier, and a surfactant.

In a particular embodiment, the liquid pharmaceutical composition comprises or is characterised by adalimumab, and the liquid pharmaceutical composition further comprises or is further characterised by: 5 to about 15 mM buffer system, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a tonicifier, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 5 to about 15 mM buffer system, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: 5 to about 15 mM buffer system, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, and either sodium gluconate or sodium chloride.

In a particular embodiment, the liquid pharmaceutical composition comprises or is characterised by adalimumab, and the liquid pharmaceutical composition further comprises or is further characterised by: 5 to about 15 mM buffer system, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either sodium gluconate or sodium chloride, and a surfactant.

In a particular embodiment, the liquid pharmaceutical composition comprises or is characterised by adalimumab, and the liquid pharmaceutical composition further comprises or is further characterised by: 5 to about 15 mM buffer system, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: 5 to about 15 mM buffer system, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: 5 to about 15 mM buffer system, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, and a sugar stabiliser.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, and a surfactant.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, and a tonicifier.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a tonicifier, and a surfactant.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a tonicifier, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, and either sodium gluconate or sodium chloride.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either sodium gluconate or sodium chloride, and a surfactant.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, a sugar stabiliser, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, and a monocyclic sugar stabiliser.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, and a surfactant.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, and a tonicifier.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a tonicifier, and a surfactant.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a tonicifier, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, and either sodium gluconate or sodium chloride.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either sodium gluconate or sodium chloride, and a surfactant.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, a monocyclic sugar stabiliser, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, and either inositol or trehalose.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, and a surfactant.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, and a tonicifier.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a tonicifier, and a surfactant.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a tonicifier, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, and either sodium gluconate or sodium chloride.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either sodium gluconate or sodium chloride, and a surfactant.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, either inositol or trehalose, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, and Inositol.

In a particular embodiment, the liquid pharmaceutical composition comprises or is characterised by adalimumab, and the liquid pharmaceutical composition further comprises or is further characterised by: 5 to about 15 mM buffer system, Inositol, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, and a surfactant.

In a particular embodiment, the liquid pharmaceutical composition comprises or is characterised by adalimumab, and the liquid pharmaceutical composition further comprises or is further characterised by: 5 to about 15 mM buffer system, Inositol, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, Inositol, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, Inositol, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, Inositol, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, Inositol, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, and a tonicifier.

In a particular embodiment, the liquid pharmaceutical composition comprises or is characterised by adalimumab, and the liquid pharmaceutical composition further comprises or is further characterised by: 5 to about 15 mM buffer system, Inositol, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, a tonicifier, and a surfactant.

In a particular embodiment, the liquid pharmaceutical composition comprises or is characterised by adalimumab, and the liquid pharmaceutical composition further comprises or is further characterised by: 5 to about 15 mM buffer system, Inositol, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, a tonicifier, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, Inositol, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, Inositol, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, Inositol, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, Inositol, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 5 to about 15 mM buffer system, Inositol, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: 5 to about 15 mM buffer system, Inositol, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, Inositol, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, Inositol, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, Inositol, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, Inositol, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, and either sodium gluconate or sodium chloride.

In a particular embodiment, the liquid pharmaceutical composition comprises or is characterised by adalimumab, and the liquid pharmaceutical composition further comprises or is further characterised by: 5 to about 15 mM buffer system, Inositol, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, either sodium gluconate or sodium chloride, and a surfactant.

In a particular embodiment, the liquid pharmaceutical composition comprises or is characterised by adalimumab, and the liquid pharmaceutical composition further comprises or is further characterised by: 5 to about 15 mM buffer system, Inositol, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, Inositol, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, Inositol, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, Inositol, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, Inositol, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: 5 to about 15 mM buffer system, Inositol, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: 5 to about 15 mM buffer system, Inositol, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, Inositol, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, Inositol, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, Inositol, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, Inositol, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, Inositol, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, and 10 to 40 mg/mL monocyclic sugar stabiliser.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, and a surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, and a tonicifier.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a tonicifier, and a surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a tonicifier, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, and either sodium gluconate or sodium chloride.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either sodium gluconate or sodium chloride, and a surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL monocyclic sugar stabiliser, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, and 10 to 40 mg/mL Inositol.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, and a surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, and a tonicifier.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a tonicifier, and a surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a tonicifier, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, and either sodium gluconate or sodium chloride.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either sodium gluconate or sodium chloride, and a surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 5 to about 15 mM buffer system, 10 to 40 mg/mL Inositol, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system.

In a particular embodiment, the liquid pharmaceutical composition comprises or is characterised by adalimumab, and the liquid pharmaceutical composition further comprises or is further characterised by: 10 mM acetate buffer system, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, and a surfactant.

In a particular embodiment, the liquid pharmaceutical composition comprises or is characterised by adalimumab, and the liquid pharmaceutical composition further comprises or is further characterised by: 10 mM acetate buffer system, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, and a PVP surfactant.

In a particular embodiment, the liquid pharmaceutical composition comprises or is characterised by adalimumab, and the liquid pharmaceutical composition further comprises or is further characterised by: 10 mM acetate buffer system, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, and a tonicifier.

In a particular embodiment, the liquid pharmaceutical composition comprises or is characterised by adalimumab, and the liquid pharmaceutical composition further comprises or is further characterised by: 10 mM acetate buffer system, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a tonicifier, and a surfactant.

In a particular embodiment, the liquid pharmaceutical composition comprises or is characterised by adalimumab, and the liquid pharmaceutical composition further comprises or is further characterised by: 10 mM acetate buffer system, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a tonicifier, and a PVP surfactant.

In a particular embodiment, the liquid pharmaceutical composition comprises or is characterised by adalimumab, and the liquid pharmaceutical composition further comprises or is further characterised by: 10 mM acetate buffer system, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 10 mM acetate buffer system, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: 10 mM acetate buffer system, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 10 mM acetate buffer system, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, and either sodium gluconate or sodium chloride.

In a particular embodiment, the liquid pharmaceutical composition comprises or is characterised by adalimumab, and the liquid pharmaceutical composition further comprises or is further characterised by: 10 mM acetate buffer system, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either sodium gluconate or sodium chloride, and a surfactant.

In a particular embodiment, the liquid pharmaceutical composition comprises or is characterised by adalimumab, and the liquid pharmaceutical composition further comprises or is further characterised by: 10 mM acetate buffer system, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: 10 mM acetate buffer system, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: 10 mM acetate buffer system, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: 10 mM acetate buffer system, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: 10 mM acetate buffer system, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, and a sugar stabiliser.

In a particular embodiment, the liquid pharmaceutical composition comprises or is characterised by adalimumab, and the liquid pharmaceutical composition further comprises or is further characterised by: 10 mM acetate buffer system, a sugar stabiliser, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, and a surfactant.

In a particular embodiment, the liquid pharmaceutical composition comprises or is characterised by adalimumab, and the liquid pharmaceutical composition further comprises or is further characterised by: 10 mM acetate buffer system, a sugar stabiliser, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, and a PVP surfactant.

In a particular embodiment, the liquid pharmaceutical composition comprises or is characterised by adalimumab, and the liquid pharmaceutical composition further comprises or is further characterised by: 10 mM acetate buffer system, a sugar stabiliser, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, a sugar stabiliser, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, a sugar stabiliser, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, a sugar stabiliser, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, and a tonicifier.

In a particular embodiment, the liquid pharmaceutical composition comprises or is characterised by adalimumab, and the liquid pharmaceutical composition further comprises or is further characterised by: 10 mM acetate buffer system, a sugar stabiliser, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, a tonicifier, and a surfactant.

In a particular embodiment, the liquid pharmaceutical composition comprises or is characterised by adalimumab, and the liquid pharmaceutical composition further comprises or is further characterised by: 10 mM acetate buffer system, a sugar stabiliser, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, a tonicifier, and a PVP surfactant.

In a particular embodiment, the liquid pharmaceutical composition comprises or is characterised by adalimumab, and the liquid pharmaceutical composition further comprises or is further characterised by: 10 mM acetate buffer system, a sugar stabiliser, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, a sugar stabiliser, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, a sugar stabiliser, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, a sugar stabiliser, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 10 mM acetate buffer system, a sugar stabiliser, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: 10 mM acetate buffer system, a sugar stabiliser, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 10 mM acetate buffer system, a sugar stabiliser, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, a sugar stabiliser, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, a sugar stabiliser, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, a sugar stabiliser, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, and either sodium gluconate or sodium chloride.

In a particular embodiment, the liquid pharmaceutical composition comprises or is characterised by adalimumab, and the liquid pharmaceutical composition further comprises or is further characterised by: 10 mM acetate buffer system, a sugar stabiliser, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, either sodium gluconate or sodium chloride, and a surfactant.

In a particular embodiment, the liquid pharmaceutical composition comprises or is characterised by adalimumab, and the liquid pharmaceutical composition further comprises or is further characterised by: 10 mM acetate buffer system, a sugar stabiliser, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: 10 mM acetate buffer system, a sugar stabiliser, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, a sugar stabiliser, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, a sugar stabiliser, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, a sugar stabiliser, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: 10 mM acetate buffer system, a sugar stabiliser, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: 10 mM acetate buffer system, a sugar stabiliser, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: 10 mM acetate buffer system, a sugar stabiliser, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, a sugar stabiliser, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, a sugar stabiliser, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a sugar stabiliser, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, a sugar stabiliser, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, and a monocyclic sugar stabiliser.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, and a surfactant.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, and a PVP surfactant.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, and a tonicifier.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a tonicifier, and a surfactant.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a tonicifier, and a PVP surfactant.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, and either sodium gluconate or sodium chloride.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either sodium gluconate or sodium chloride, and a surfactant.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, a monocyclic sugar stabiliser, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, and either inositol or trehalose.

In a particular embodiment, the liquid pharmaceutical composition comprises or is characterised by adalimumab, and the liquid pharmaceutical composition further comprises or is further characterised by: 10 mM acetate buffer system, either inositol or trehalose, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, and a surfactant.

In a particular embodiment, the liquid pharmaceutical composition comprises or is characterised by adalimumab, and the liquid pharmaceutical composition further comprises or is further characterised by: 10 mM acetate buffer system, either inositol or trehalose, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, and a PVP surfactant.

In a particular embodiment, the liquid pharmaceutical composition comprises or is characterised by adalimumab, and the liquid pharmaceutical composition further comprises or is further characterised by: 10 mM acetate buffer system, either inositol or trehalose, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, either inositol or trehalose, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, either inositol or trehalose, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, either inositol or trehalose, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, and a tonicifier.

In a particular embodiment, the liquid pharmaceutical composition comprises or is characterised by adalimumab, and the liquid pharmaceutical composition further comprises or is further characterised by: 10 mM acetate buffer system, either inositol or trehalose, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, a tonicifier, and a surfactant.

In a particular embodiment, the liquid pharmaceutical composition comprises or is characterised by adalimumab, and the liquid pharmaceutical composition further comprises or is further characterised by: 10 mM acetate buffer system, either inositol or trehalose, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, a tonicifier, and a PVP surfactant.

In a particular embodiment, the liquid pharmaceutical composition comprises or is characterised by adalimumab, and the liquid pharmaceutical composition further comprises or is further characterised by: 10 mM acetate buffer system, either inositol or trehalose, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, either inositol or trehalose, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, either inositol or trehalose, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, either inositol or trehalose, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 10 mM acetate buffer system, either inositol or trehalose, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: 10 mM acetate buffer system, either inositol or trehalose, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 10 mM acetate buffer system, either inositol or trehalose, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, either inositol or trehalose, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, either inositol or trehalose, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, either inositol or trehalose, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, and either sodium gluconate or sodium chloride.

In a particular embodiment, the liquid pharmaceutical composition comprises or is characterised by adalimumab, and the liquid pharmaceutical composition further comprises or is further characterised by: 10 mM acetate buffer system, either inositol or trehalose, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, either sodium gluconate or sodium chloride, and a surfactant.

In a particular embodiment, the liquid pharmaceutical composition comprises or is characterised by adalimumab, and the liquid pharmaceutical composition further comprises or is further characterised by: 10 mM acetate buffer system, either inositol or trehalose, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: 10 mM acetate buffer system, either inositol or trehalose, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, either inositol or trehalose, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, either inositol or trehalose, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, either inositol or trehalose, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: 10 mM acetate buffer system, either inositol or trehalose, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: 10 mM acetate buffer system, either inositol or trehalose, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: 10 mM acetate buffer system, either inositol or trehalose, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, either inositol or trehalose, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, either inositol or trehalose, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, either inositol or trehalose, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, either inositol or trehalose, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, and Inositol.

In a particular embodiment, the liquid pharmaceutical composition comprises or is characterised by adalimumab, and the liquid pharmaceutical composition further comprises or is further characterised by: 10 mM acetate buffer system, Inositol, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, and a surfactant.

In a particular embodiment, the liquid pharmaceutical composition comprises or is characterised by adalimumab, and the liquid pharmaceutical composition further comprises or is further characterised by: 10 mM acetate buffer system, Inositol, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, and a PVP surfactant.

In a particular embodiment, the liquid pharmaceutical composition comprises or is characterised by adalimumab, and the liquid pharmaceutical composition further comprises or is further characterised by: 10 mM acetate buffer system, Inositol, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, Inositol, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, Inositol, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, Inositol, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, and a tonicifier.

In a particular embodiment, the liquid pharmaceutical composition comprises or is characterised by adalimumab, and the liquid pharmaceutical composition further comprises or is further characterised by: 10 mM acetate buffer system, Inositol, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, a tonicifier, and a surfactant.

In a particular embodiment, the liquid pharmaceutical composition comprises or is characterised by adalimumab, and the liquid pharmaceutical composition further comprises or is further characterised by: 10 mM acetate buffer system, Inositol, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, a tonicifier, and a PVP surfactant.

In a particular embodiment, the liquid pharmaceutical composition comprises or is characterised by adalimumab, and the liquid pharmaceutical composition further comprises or is further characterised by: 10 mM acetate buffer system, Inositol, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, Inositol, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, Inositol, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, Inositol, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 10 mM acetate buffer system, Inositol, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: 10 mM acetate buffer system, Inositol, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 10 mM acetate buffer system, Inositol, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, Inositol, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, Inositol, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, Inositol, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, and either sodium gluconate or sodium chloride.

In a particular embodiment, the liquid pharmaceutical composition comprises or is characterised by adalimumab, and the liquid pharmaceutical composition further comprises or is further characterised by: 10 mM acetate buffer system, Inositol, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, either sodium gluconate or sodium chloride, and a surfactant.

In a particular embodiment, the liquid pharmaceutical composition comprises or is characterised by adalimumab, and the liquid pharmaceutical composition further comprises or is further characterised by: 10 mM acetate buffer system, Inositol, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: 10 mM acetate buffer system, Inositol, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, Inositol, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, Inositol, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, Inositol, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: 10 mM acetate buffer system, Inositol, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: 10 mM acetate buffer system, Inositol, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: 10 mM acetate buffer system, Inositol, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, Inositol, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, Inositol, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, Inositol, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, Inositol, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, and 10 to 40 mg/mL monocyclic sugar stabiliser.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, and a surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, and a PVP surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, and a tonicifier.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a tonicifier, and a surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a tonicifier, and a PVP surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, and either sodium gluconate or sodium chloride.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either sodium gluconate or sodium chloride, and a surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL monocyclic sugar stabiliser, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, and 10 to 40 mg/mL Inositol.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, and a surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, and a PVP surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, and a tonicifier.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a tonicifier, and a surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a tonicifier, and a PVP surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, and a metal (hydroxy)mono-/di-carboxylate salt tonicifier.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a metal (hydroxy)mono-/di-carboxylate salt tonicifier, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a metal (hydroxy)mono-/di-carboxylate salt tonicifier, and a surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a metal (hydroxy)mono-/di-carboxylate salt tonicifier, and a PVP surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, and either sodium gluconate or sodium chloride.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either sodium gluconate or sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either sodium gluconate or sodium chloride, and a surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either sodium gluconate or sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either sodium gluconate or sodium chloride, and a PVP surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either sodium gluconate or sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either sodium gluconate or sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either sodium gluconate or sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, and either 45 mM and 230 mM sodium gluconate or 25 mM to 75 mM sodium chloride.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either 45 mM and 230 mM sodium gluconate or 25 mM to 75 mM sodium chloride, and a surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either 45 mM and 230 mM sodium gluconate or 25 mM to 75 mM sodium chloride, and a PVP surfactant.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by adalimumab, and the liquid pharmaceutical composition further comprises or is further characterised by: 10 mM acetate buffer system, 10 to 40 mg/mL Inositol, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: wherein the composition has a pH between 5.0 and 5.5.

In a particular embodiment, the liquid pharmaceutical composition comprises or is characterised by a buffer system, and the liquid pharmaceutical composition further comprises or is further characterised by: a surfactant.

In a particular embodiment, the liquid pharmaceutical composition comprises or is characterised by a buffer system, and the liquid pharmaceutical composition further comprises or is further characterised by: a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a PVP surfactant.

In a particular embodiment, the liquid pharmaceutical composition comprises or is characterised by a buffer system, and the liquid pharmaceutical composition further comprises or is further characterised by: a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: a tonicifier.

In a particular embodiment, the liquid pharmaceutical composition comprises or is characterised by a buffer system, and the liquid pharmaceutical composition further comprises or is further characterised by: a tonicifier, and wherein the composition has a pH between 5.0 and 5.5.

In a particular embodiment, the liquid pharmaceutical composition comprises or is characterised by a buffer system, and the liquid pharmaceutical composition further comprises or is further characterised by: a tonicifier, and a surfactant.

In a particular embodiment, the liquid pharmaceutical composition comprises or is characterised by a buffer system, and the liquid pharmaceutical composition further comprises or is further characterised by: a tonicifier, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a tonicifier, and a PVP surfactant.

In a particular embodiment, the liquid pharmaceutical composition comprises or is characterised by a buffer system, and the liquid pharmaceutical composition further comprises or is further characterised by: a tonicifier, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a tonicifier, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a tonicifier, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a tonicifier, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: a metal (hydroxy)mono-/di-carboxylate salt tonicifier.

In a particular embodiment, the liquid pharmaceutical composition comprises or is characterised by a buffer system, and the liquid pharmaceutical composition further comprises or is further characterised by: a metal (hydroxy)mono-/di-carboxylate salt tonicifier, and wherein the composition has a pH between 5.0 and 5.5.

In a particular embodiment, the liquid pharmaceutical composition comprises or is characterised by a buffer system, and the liquid pharmaceutical composition further comprises or is further characterised by: a metal (hydroxy)mono-/di-carboxylate salt tonicifier, and a surfactant.

In a particular embodiment, the liquid pharmaceutical composition comprises or is characterised by a buffer system, and the liquid pharmaceutical composition further comprises or is further characterised by: a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a metal (hydroxy)mono-/di-carboxylate salt tonicifier, and a PVP surfactant.

In a particular embodiment, the liquid pharmaceutical composition comprises or is characterised by a buffer system, and the liquid pharmaceutical composition further comprises or is further characterised by: a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: either sodium gluconate or sodium chloride.

In a particular embodiment, the liquid pharmaceutical composition comprises or is characterised by a buffer system, and the liquid pharmaceutical composition further comprises or is further characterised by: either sodium gluconate or sodium chloride, and wherein the composition has a pH between 5.0 and 5.5.

In a particular embodiment, the liquid pharmaceutical composition comprises or is characterised by a buffer system, and the liquid pharmaceutical composition further comprises or is further characterised by: either sodium gluconate or sodium chloride, and a surfactant.

In a particular embodiment, the liquid pharmaceutical composition comprises or is characterised by a buffer system, and the liquid pharmaceutical composition further comprises or is further characterised by: either sodium gluconate or sodium chloride, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: either sodium gluconate or sodium chloride, and a PVP surfactant.

In a particular embodiment, the liquid pharmaceutical composition comprises or is characterised by a buffer system, and the liquid pharmaceutical composition further comprises or is further characterised by: either sodium gluconate or sodium chloride, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: either sodium gluconate or sodium chloride, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: either sodium gluconate or sodium chloride, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: either 45 mM and 230 mM sodium gluconate or 25 mM to 75 mM sodium chloride.

In a particular embodiment, the liquid pharmaceutical composition comprises or is characterised by a buffer system, and the liquid pharmaceutical composition further comprises or is further characterised by: either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by a buffer system, and the liquid pharmaceutical composition further comprises or is further characterised by: either 45 mM and 230 mM sodium gluconate or 25 mM to 75 mM sodium chloride, and a surfactant.

In a particular embodiment, the liquid pharmaceutical composition comprises or is characterised by a buffer system, and the liquid pharmaceutical composition further comprises or is further characterised by: either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: either 45 mM and 230 mM sodium gluconate or 25 mM to 75 mM sodium chloride, and a PVP surfactant.

In a particular embodiment, the liquid pharmaceutical composition comprises or is characterised by a buffer system, and the liquid pharmaceutical composition further comprises or is further characterised by: either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser.

In a particular embodiment, the liquid pharmaceutical composition comprises or is characterised by a buffer system, and the liquid pharmaceutical composition further comprises or is further characterised by: a sugar stabiliser, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, and a surfactant.

In a particular embodiment, the liquid pharmaceutical composition comprises or is characterised by a buffer system, and the liquid pharmaceutical composition further comprises or is further characterised by: a sugar stabiliser, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, and a PVP surfactant.

In a particular embodiment, the liquid pharmaceutical composition comprises or is characterised by a buffer system, and the liquid pharmaceutical composition further comprises or is further characterised by: a sugar stabiliser, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: a sugar stabiliser, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: a sugar stabiliser, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: a sugar stabiliser, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, and a tonicifier.

In a particular embodiment, the liquid pharmaceutical composition comprises or is characterised by a buffer system, and the liquid pharmaceutical composition further comprises or is further characterised by: a sugar stabiliser, a tonicifier, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, a tonicifier, and a surfactant.

In a particular embodiment, the liquid pharmaceutical composition comprises or is characterised by a buffer system, and the liquid pharmaceutical composition further comprises or is further characterised by: a sugar stabiliser, a tonicifier, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, a tonicifier, and a PVP surfactant.

In a particular embodiment, the liquid pharmaceutical composition comprises or is characterised by a buffer system, and the liquid pharmaceutical composition further comprises or is further characterised by: a sugar stabiliser, a tonicifier, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, a tonicifier, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: a sugar stabiliser, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, a tonicifier, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: a sugar stabiliser, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, a tonicifier, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: a sugar stabiliser, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, and a metal (hydroxy)mono-/di-carboxylate salt tonicifier.

In a particular embodiment, the liquid pharmaceutical composition comprises or is characterised by a buffer system, and the liquid pharmaceutical composition further comprises or is further characterised by: a sugar stabiliser, a metal (hydroxy)mono-/dicarboxylate salt tonicifier, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, a metal (hydroxy)mono-/dicarboxylate salt tonicifier, and a surfactant.

In a particular embodiment, the liquid pharmaceutical composition comprises or is characterised by a buffer system, and the liquid pharmaceutical composition further comprises or is further characterised by: a sugar stabiliser, a metal (hydroxy)mono-/dicarboxylate salt tonicifier, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, a metal (hydroxy)mono-/dicarboxylate salt tonicifier, and a PVP surfactant.

In a particular embodiment, the liquid pharmaceutical composition comprises or is characterised by a buffer system, and the liquid pharmaceutical composition further comprises or is further characterised by: a sugar stabiliser, a metal (hydroxy)mono-/dicarboxylate salt tonicifier, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, a metal (hydroxy)mono-/dicarboxylate salt tonicifier, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: a sugar stabiliser, a metal (hydroxy)mono-/dicarboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, a metal (hydroxy)mono-/dicarboxylate salt tonicifier, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: a sugar stabiliser, a metal (hydroxy)mono-/dicarboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, a metal (hydroxy)mono-/dicarboxylate salt tonicifier, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: a sugar stabiliser, a metal (hydroxy)mono-/dicarboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, and either sodium gluconate or sodium chloride.

In a particular embodiment, the liquid pharmaceutical composition comprises or is characterised by a buffer system, and the liquid pharmaceutical composition further comprises or is further characterised by: a sugar stabiliser, either sodium gluconate or sodium chloride, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, either sodium gluconate or sodium chloride, and a surfactant.

In a particular embodiment, the liquid pharmaceutical composition comprises or is characterised by a buffer system, and the liquid pharmaceutical composition further comprises or is further characterised by: a sugar stabiliser, either sodium gluconate or sodium chloride, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, either sodium gluconate or sodium chloride, and a PVP surfactant.

In a particular embodiment, the liquid pharmaceutical composition comprises or is characterised by a buffer system, and the liquid pharmaceutical composition further comprises or is further characterised by: a sugar stabiliser, either sodium gluconate or sodium chloride, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, either sodium gluconate or sodium chloride, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: a sugar stabiliser, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, either sodium gluconate or sodium chloride, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: a sugar stabiliser, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: a sugar stabiliser, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, and either 45 mM and 230 mM sodium gluconate or 25 mM to 75 mM sodium chloride.

In a particular embodiment, the liquid pharmaceutical composition comprises or is characterised by a buffer system, and the liquid pharmaceutical composition further comprises or is further characterised by: a sugar stabiliser, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, either 45 mM and 230 mM sodium gluconate or 25 mM to 75 mM sodium chloride, and a surfactant.

In a particular embodiment, the liquid pharmaceutical composition comprises or is characterised by a buffer system, and the liquid pharmaceutical composition further comprises or is further characterised by: a sugar stabiliser, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, either 45 mM and 230 mM sodium gluconate or 25 mM to 75 mM sodium chloride, and a PVP surfactant.

In a particular embodiment, the liquid pharmaceutical composition comprises or is characterised by a buffer system, and the liquid pharmaceutical composition further comprises or is further characterised by: a sugar stabiliser, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: a sugar stabiliser, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: a sugar stabiliser, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a sugar stabiliser, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: a sugar stabiliser, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser.

In a particular embodiment, the liquid pharmaceutical composition comprises or is characterised by a buffer system, and the liquid pharmaceutical composition further comprises or is further characterised by: a monocyclic sugar stabiliser, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, and a surfactant.

In a particular embodiment, the liquid pharmaceutical composition comprises or is characterised by a buffer system, and the liquid pharmaceutical composition further comprises or is further characterised by: a monocyclic sugar stabiliser, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, and a PVP surfactant.

In a particular embodiment, the liquid pharmaceutical composition comprises or is characterised by a buffer system, and the liquid pharmaceutical composition further comprises or is further characterised by: a monocyclic sugar stabiliser, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: a monocyclic sugar stabiliser, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: a monocyclic sugar stabiliser, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: a monocyclic sugar stabiliser, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, and a tonicifier.

In a particular embodiment, the liquid pharmaceutical composition comprises or is characterised by a buffer system, and the liquid pharmaceutical composition further comprises or is further characterised by: a monocyclic sugar stabiliser, a tonicifier, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, a tonicifier, and a surfactant.

In a particular embodiment, the liquid pharmaceutical composition comprises or is characterised by a buffer system, and the liquid pharmaceutical composition further comprises or is further characterised by: a monocyclic sugar stabiliser, a tonicifier, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, a tonicifier, and a PVP surfactant.

In a particular embodiment, the liquid pharmaceutical composition comprises or is characterised by a buffer system, and the liquid pharmaceutical composition further comprises or is further characterised by: a monocyclic sugar stabiliser, a tonicifier, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, a tonicifier, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: a monocyclic sugar stabiliser, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, a tonicifier, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: a monocyclic sugar stabiliser, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, a tonicifier, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: a monocyclic sugar stabiliser, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, and a metal (hydroxy)mono-/di-carboxylate salt tonicifier.

In a particular embodiment, the liquid pharmaceutical composition comprises or is characterised by a buffer system, and the liquid pharmaceutical composition further comprises or is further characterised by: a monocyclic sugar stabiliser, a metal (hydroxy)mono-/di-carboxylate salt tonicifier, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, a metal (hydroxy)mono-/di-carboxylate salt tonicifier, and a surfactant.

In a particular embodiment, the liquid pharmaceutical composition comprises or is characterised by a buffer system, and the liquid pharmaceutical composition further comprises or is further characterised by: a monocyclic sugar stabiliser, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, a metal (hydroxy)mono-/di-carboxylate salt tonicifier, and a PVP surfactant.

In a particular embodiment, the liquid pharmaceutical composition comprises or is characterised by a buffer system, and the liquid pharmaceutical composition further comprises or is further characterised by: a monocyclic sugar stabiliser, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: a monocyclic sugar stabiliser, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: a monocyclic sugar stabiliser, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: a monocyclic sugar stabiliser, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, and either sodium gluconate or sodium chloride.

In a particular embodiment, the liquid pharmaceutical composition comprises or is characterised by a buffer system, and the liquid pharmaceutical composition further comprises or is further characterised by: a monocyclic sugar stabiliser, either sodium gluconate or sodium chloride, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, either sodium gluconate or sodium chloride, and a surfactant.

In a particular embodiment, the liquid pharmaceutical composition comprises or is characterised by a buffer system, and the liquid pharmaceutical composition further comprises or is further characterised by: a monocyclic sugar stabiliser, either sodium gluconate or sodium chloride, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, either sodium gluconate or sodium chloride, and a PVP surfactant.

In a particular embodiment, the liquid pharmaceutical composition comprises or is characterised by a buffer system, and the liquid pharmaceutical composition further comprises or is further characterised by: a monocyclic sugar stabiliser, either sodium gluconate or sodium chloride, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, either sodium gluconate or sodium chloride, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: a monocyclic sugar stabiliser, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, either sodium gluconate or sodium chloride, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: a monocyclic sugar stabiliser, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: a monocyclic sugar stabiliser, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, and either 45 mM and 230 mM sodium gluconate or 25 mM to 75 mM sodium chloride.

In a particular embodiment, the liquid pharmaceutical composition comprises or is characterised by a buffer system, and the liquid pharmaceutical composition further comprises or is further characterised by: a monocyclic sugar stabiliser, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, either 45 mM and 230 mM sodium gluconate or 25 mM to 75 mM sodium chloride, and a surfactant.

In a particular embodiment, the liquid pharmaceutical composition comprises or is characterised by a buffer system, and the liquid pharmaceutical composition further comprises or is further characterised by: a monocyclic sugar stabiliser, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, either 45 mM and 230 mM sodium gluconate or 25 mM to 75 mM sodium chloride, and a PVP surfactant.

In a particular embodiment, the liquid pharmaceutical composition comprises or is characterised by a buffer system, and the liquid pharmaceutical composition further comprises or is further characterised by: a monocyclic sugar stabiliser, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: a monocyclic sugar stabiliser, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: a monocyclic sugar stabiliser, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: a monocyclic sugar stabiliser, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: a monocyclic sugar stabiliser, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose.

In a particular embodiment, the liquid pharmaceutical composition comprises or is characterised by a buffer system, and the liquid pharmaceutical composition further comprises or is further characterised by: either inositol or trehalose, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, and a surfactant.

In a particular embodiment, the liquid pharmaceutical composition comprises or is characterised by a buffer system, and the liquid pharmaceutical composition further comprises or is further characterised by: either inositol or trehalose, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, and a PVP surfactant.

In a particular embodiment, the liquid pharmaceutical composition comprises or is characterised by a buffer system, and the liquid pharmaceutical composition further comprises or is further characterised by: either inositol or trehalose, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: either inositol or trehalose, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: either inositol or trehalose, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: either inositol or trehalose, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, and a tonicifier.

In a particular embodiment, the liquid pharmaceutical composition comprises or is characterised by a buffer system, and the liquid pharmaceutical composition further comprises or is further characterised by: either inositol or trehalose, a tonicifier, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, a tonicifier, and a surfactant.

In a particular embodiment, the liquid pharmaceutical composition comprises or is characterised by a buffer system, and the liquid pharmaceutical composition further comprises or is further characterised by: either inositol or trehalose, a tonicifier, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, a tonicifier, and a PVP surfactant.

In a particular embodiment, the liquid pharmaceutical composition comprises or is characterised by a buffer system, and the liquid pharmaceutical composition further comprises or is further characterised by: either inositol or trehalose, a tonicifier, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, a tonicifier, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: either inositol or trehalose, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, a tonicifier, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: either inositol or trehalose, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, a tonicifier, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: either inositol or trehalose, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, and a metal (hydroxy)mono-/di-carboxylate salt tonicifier.

In a particular embodiment, the liquid pharmaceutical composition comprises or is characterised by a buffer system, and the liquid pharmaceutical composition further comprises or is further characterised by: either inositol or trehalose, a metal (hydroxy)mono-/di-carboxylate salt tonicifier, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, a metal (hydroxy)mono-/di-carboxylate salt tonicifier, and a surfactant.

In a particular embodiment, the liquid pharmaceutical composition comprises or is characterised by a buffer system, and the liquid pharmaceutical composition further comprises or is further characterised by: either inositol or trehalose, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, a metal (hydroxy)mono-/di-carboxylate salt tonicifier, and a PVP surfactant.

In a particular embodiment, the liquid pharmaceutical composition comprises or is characterised by a buffer system, and the liquid pharmaceutical composition further comprises or is further characterised by: either inositol or trehalose, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: either inositol or trehalose, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: either inositol or trehalose, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: either inositol or trehalose, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, and either sodium gluconate or sodium chloride.

In a particular embodiment, the liquid pharmaceutical composition comprises or is characterised by a buffer system, and the liquid pharmaceutical composition further comprises or is further characterised by: either inositol or trehalose, either sodium gluconate or sodium chloride, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, either sodium gluconate or sodium chloride, and a surfactant.

In a particular embodiment, the liquid pharmaceutical composition comprises or is characterised by a buffer system, and the liquid pharmaceutical composition further comprises or is further characterised by: either inositol or trehalose, either sodium gluconate or sodium chloride, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, either sodium gluconate or sodium chloride, and a PVP surfactant.

In a particular embodiment, the liquid pharmaceutical composition comprises or is characterised by a buffer system, and the liquid pharmaceutical composition further comprises or is further characterised by: either inositol or trehalose, either sodium gluconate or sodium chloride, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, either sodium gluconate or sodium chloride, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: either inositol or trehalose, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, either sodium gluconate or sodium chloride, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: either inositol or trehalose, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: either inositol or trehalose, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, and either 45 mM and 230 mM sodium gluconate or 25 mM to 75 mM sodium chloride.

In a particular embodiment, the liquid pharmaceutical composition comprises or is characterised by a buffer system, and the liquid pharmaceutical composition further comprises or is further characterised by: either inositol or trehalose, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, either 45 mM and 230 mM sodium gluconate or 25 mM to 75 mM sodium chloride, and a surfactant.

In a particular embodiment, the liquid pharmaceutical composition comprises or is characterised by a buffer system, and the liquid pharmaceutical composition further comprises or is further characterised by: either inositol or trehalose, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, either 45 mM and 230 mM sodium gluconate or 25 mM to 75 mM sodium chloride, and a PVP surfactant.

In a particular embodiment, the liquid pharmaceutical composition comprises or is characterised by a buffer system, and the liquid pharmaceutical composition further comprises or is further characterised by: either inositol or trehalose, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: either inositol or trehalose, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: either inositol or trehalose, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: either inositol or trehalose, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: either inositol or trehalose, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol.

In a particular embodiment, the liquid pharmaceutical composition comprises or is characterised by a buffer system, and the liquid pharmaceutical composition further comprises or is further characterised by: Inositol, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, and a surfactant.

In a particular embodiment, the liquid pharmaceutical composition comprises or is characterised by a buffer system, and the liquid pharmaceutical composition further comprises or is further characterised by: Inositol, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, and a PVP surfactant.

In a particular embodiment, the liquid pharmaceutical composition comprises or is characterised by a buffer system, and the liquid pharmaceutical composition further comprises or is further characterised by: Inositol, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: Inositol, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: Inositol, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: Inositol, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, and a tonicifier.

In a particular embodiment, the liquid pharmaceutical composition comprises or is characterised by a buffer system, and the liquid pharmaceutical composition further comprises or is further characterised by: Inositol, a tonicifier, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, a tonicifier, and a surfactant.

In a particular embodiment, the liquid pharmaceutical composition comprises or is characterised by a buffer system, and the liquid pharmaceutical composition further comprises or is further characterised by: Inositol, a tonicifier, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, a tonicifier, and a PVP surfactant.

In a particular embodiment, the liquid pharmaceutical composition comprises or is characterised by a buffer system, and the liquid pharmaceutical composition further comprises or is further characterised by: Inositol, a tonicifier, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, a tonicifier, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: Inositol, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, a tonicifier, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: Inositol, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, a tonicifier, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: Inositol, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, and a metal (hydroxy)mono-/dicarboxylate salt tonicifier.

In a particular embodiment, the liquid pharmaceutical composition comprises or is characterised by a buffer system, and the liquid pharmaceutical composition further comprises or is further characterised by: Inositol, a metal (hydroxy)mono-/di-carboxylate salt tonicifier, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, a metal (hydroxy)mono-/di-carboxylate salt tonicifier, and a surfactant.

In a particular embodiment, the liquid pharmaceutical composition comprises or is characterised by a buffer system, and the liquid pharmaceutical composition further comprises or is further characterised by: Inositol, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, a metal (hydroxy)mono-/di-carboxylate salt tonicifier, and a PVP surfactant.

In a particular embodiment, the liquid pharmaceutical composition comprises or is characterised by a buffer system, and the liquid pharmaceutical composition further comprises or is further characterised by: Inositol, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: Inositol, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: Inositol, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: Inositol, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, and either sodium gluconate or sodium chloride.

In a particular embodiment, the liquid pharmaceutical composition comprises or is characterised by a buffer system, and the liquid pharmaceutical composition further comprises or is further characterised by: Inositol, either sodium gluconate or sodium chloride, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, either sodium gluconate or sodium chloride, and a surfactant.

In a particular embodiment, the liquid pharmaceutical composition comprises or is characterised by a buffer system, and the liquid pharmaceutical composition further comprises or is further characterised by: Inositol, either sodium gluconate or sodium chloride, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, either sodium gluconate or sodium chloride, and a PVP surfactant.

In a particular embodiment, the liquid pharmaceutical composition comprises or is characterised by a buffer system, and the liquid pharmaceutical composition further comprises or is further characterised by: Inositol, either sodium gluconate or sodium chloride, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, either sodium gluconate or sodium chloride, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: Inositol, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, either sodium gluconate or sodium chloride, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: Inositol, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: Inositol, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, and either 45 mM and 230 mM sodium gluconate or 25 mM to 75 mM sodium chloride.

In a particular embodiment, the liquid pharmaceutical composition comprises or is characterised by a buffer system, and the liquid pharmaceutical composition further comprises or is further characterised by: Inositol, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, either 45 mM and 230 mM sodium gluconate or 25 mM to 75 mM sodium chloride, and a surfactant.

In a particular embodiment, the liquid pharmaceutical composition comprises or is characterised by a buffer system, and the liquid pharmaceutical composition further comprises or is further characterised by: Inositol, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, either 45 mM and 230 mM sodium gluconate or 25 mM to 75 mM sodium chloride, and a PVP surfactant.

In a particular embodiment, the liquid pharmaceutical composition comprises or is characterised by a buffer system, and the liquid pharmaceutical composition further comprises or is further characterised by: Inositol, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: Inositol, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: Inositol, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: Inositol, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: Inositol, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, and a surfactant.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, and a PVP surfactant.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, and a tonicifier.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a tonicifier, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a tonicifier, and a surfactant.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a tonicifier, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a tonicifier, and a PVP surfactant.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a tonicifier, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a tonicifier, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a tonicifier, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a tonicifier, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, and a metal (hydroxy)mono-/di-carboxylate salt tonicifier.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a metal (hydroxy)mono-/di-carboxylate salt tonicifier, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a metal (hydroxy)mono-/di-carboxylate salt tonicifier, and a surfactant.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a metal (hydroxy)mono-/di-carboxylate salt tonicifier, and a PVP surfactant.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, and either sodium gluconate or sodium chloride.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either sodium gluconate or sodium chloride, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either sodium gluconate or sodium chloride, and a surfactant.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either sodium gluconate or sodium chloride, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either sodium gluconate or sodium chloride, and a PVP surfactant.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either sodium gluconate or sodium chloride, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either sodium gluconate or sodium chloride, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either sodium gluconate or sodium chloride, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, and either 45 mM and 230 mM sodium gluconate or 25 mM to 75 mM sodium chloride.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, and a surfactant.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, and a PVP surfactant.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: 10 to 40 mg/mL monocyclic sugar stabiliser, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol.

In a particular embodiment, the liquid pharmaceutical composition comprises or is characterised by a buffer system, and the liquid pharmaceutical composition further comprises or is further characterised by: 10 to 40 mg/mL Inositol, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, and a surfactant.

In a particular embodiment, the liquid pharmaceutical composition comprises or is characterised by a buffer system, and the liquid pharmaceutical composition further comprises or is further characterised by: 10 to 40 mg/mL Inositol, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, and a PVP surfactant.

In a particular embodiment, the liquid pharmaceutical composition comprises or is characterised by a buffer system, and the liquid pharmaceutical composition further comprises or is further characterised by: 10 to 40 mg/mL Inositol, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: 10 to 40 mg/mL Inositol, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: 10 to 40 mg/mL Inositol, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: 10 to 40 mg/mL Inositol, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, and a tonicifier.

In a particular embodiment, the liquid pharmaceutical composition comprises or is characterised by a buffer system, and the liquid pharmaceutical composition further comprises or is further characterised by: 10 to 40 mg/mL Inositol, a tonicifier, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, a tonicifier, and a surfactant.

In a particular embodiment, the liquid pharmaceutical composition comprises or is characterised by a buffer system, and the liquid pharmaceutical composition further comprises or is further characterised by: 10 to 40 mg/mL Inositol, a tonicifier, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, a tonicifier, and a PVP surfactant.

In a particular embodiment, the liquid pharmaceutical composition comprises or is characterised by a buffer system, and the liquid pharmaceutical composition further comprises or is further characterised by: 10 to 40 mg/mL Inositol, a tonicifier, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, a tonicifier, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: 10 to 40 mg/mL Inositol, a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, a tonicifier, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: 10 to 40 mg/mL Inositol, a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, a tonicifier, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: 10 to 40 mg/mL Inositol, a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, and a metal (hydroxy)mono-/di-carboxylate salt tonicifier.

In a particular embodiment, the liquid pharmaceutical composition comprises or is characterised by a buffer system, and the liquid pharmaceutical composition further comprises or is further characterised by: 10 to 40 mg/mL Inositol, a metal (hydroxy)mono-/di-carboxylate salt tonicifier, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, a metal (hydroxy)mono-/di-carboxylate salt tonicifier, and a surfactant.

In a particular embodiment, the liquid pharmaceutical composition comprises or is characterised by a buffer system, and the liquid pharmaceutical composition further comprises or is further characterised by: 10 to 40 mg/mL Inositol, a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, a metal (hydroxy)mono-/di-carboxylate salt tonicifier, and a PVP surfactant.

In a particular embodiment, the liquid pharmaceutical composition comprises or is characterised by a buffer system, and the liquid pharmaceutical composition further comprises or is further characterised by: 10 to 40 mg/mL Inositol, a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: 10 to 40 mg/mL Inositol, a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: 10 to 40 mg/mL Inositol, a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: 10 to 40 mg/mL Inositol, a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, and either sodium gluconate or sodium chloride.

In a particular embodiment, the liquid pharmaceutical composition comprises or is characterised by a buffer system, and the liquid pharmaceutical composition further comprises or is further characterised by: 10 to 40 mg/mL Inositol, either sodium gluconate or sodium chloride, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, either sodium gluconate or sodium chloride, and a surfactant.

In a particular embodiment, the liquid pharmaceutical composition comprises or is characterised by a buffer system, and the liquid pharmaceutical composition further comprises or is further characterised by: 10 to 40 mg/mL Inositol, either sodium gluconate or sodium chloride, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, either sodium gluconate or sodium chloride, and a PVP surfactant.

In a particular embodiment, the liquid pharmaceutical composition comprises or is characterised by a buffer system, and the liquid pharmaceutical composition further comprises or is further characterised by: 10 to 40 mg/mL Inositol, either sodium gluconate or sodium chloride, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, either sodium gluconate or sodium chloride, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: 10 to 40 mg/mL Inositol, either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, either sodium gluconate or sodium chloride, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: 10 to 40 mg/mL Inositol, either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: 10 to 40 mg/mL Inositol, either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, and either 45 mM and 230 mM sodium gluconate or 25 mM to 75 mM sodium chloride.

In a particular embodiment, the liquid pharmaceutical composition comprises or is characterised by a buffer system, and the liquid pharmaceutical composition further comprises or is further characterised by: 10 to 40 mg/mL Inositol, either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, either 45 mM and 230 mM sodium gluconate or 25 mM to 75 mM sodium chloride, and a surfactant.

In a particular embodiment, the liquid pharmaceutical composition comprises or is characterised by a buffer system, and the liquid pharmaceutical composition further comprises or is further characterised by: 10 to 40 mg/mL Inositol, either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, either 45 mM and 230 mM sodium gluconate or 25 mM to 75 mM sodium chloride, and a PVP surfactant.

In a particular embodiment, the liquid pharmaceutical composition comprises or is characterised by a buffer system, and the liquid pharmaceutical composition further comprises or is further characterised by: 10 to 40 mg/mL Inositol, either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by a buffer system, and the liquid pharmaceutical composition further comprises or is further characterised by: 10 to 40 mg/mL Inositol, either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by a buffer system, and the liquid pharmaceutical composition further comprises or is further characterised by: 10 to 40 mg/mL Inositol, either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a buffer system, and the liquid pharmaceutical composition further comprises or is further characterised by: 10 to 40 mg/mL Inositol, either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by a buffer system, and the liquid pharmaceutical composition further comprises or is further characterised by: 10 to 40 mg/mL Inositol, either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: a surfactant.

In a particular embodiment, the liquid pharmaceutical composition comprises or is characterised by a sugar stabiliser, and the liquid pharmaceutical composition further comprises or is further characterised by: a surfactant, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: a PVP surfactant.

In a particular embodiment, the liquid pharmaceutical composition comprises or is characterised by a sugar stabiliser, and the liquid pharmaceutical composition further comprises or is further characterised by: a PVP surfactant, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: a PVP surfactant having a weight average molecular weight of between 1900 and 3100 or Polysorbate 80.

In a particular embodiment, the liquid pharmaceutical composition comprises or is characterised by a sugar stabiliser, and the liquid pharmaceutical composition further comprises or is further characterised by: a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: 0.01 to 2 mg/mL of either a PVP surfactant or Polysorbate 80.

In a particular embodiment, the liquid pharmaceutical composition comprises or is characterised by a sugar stabiliser, and the liquid pharmaceutical composition further comprises or is further characterised by: 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: 0.01 to 1 mg/mL PVP surfactant having a weight average molecular weight of between 1900 and 3100.

In a particular embodiment, the liquid pharmaceutical composition comprises or is characterised by a sugar stabiliser, and the liquid pharmaceutical composition further comprises or is further characterised by: 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: a tonicifier.

In a particular embodiment, the liquid pharmaceutical composition comprises or is characterised by a sugar stabiliser, and the liquid pharmaceutical composition further comprises or is further characterised by: a tonicifier, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: a tonicifier, and a surfactant.

In a particular embodiment, the liquid pharmaceutical composition comprises or is characterised by a sugar stabiliser, and the liquid pharmaceutical composition further comprises or is further characterised by: a tonicifier, a surfactant, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: a tonicifier, and a PVP surfactant.

In a particular embodiment, the liquid pharmaceutical composition comprises or is characterised by a sugar stabiliser, and the liquid pharmaceutical composition further comprises or is further characterised by: a tonicifier, a PVP surfactant, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: a tonicifier, and a PVP surfactant having a weight average molecular weight of between 1900 and 3100 or Polysorbate 80.

In a particular embodiment, the liquid pharmaceutical composition comprises or is characterised by a sugar stabiliser, and the liquid pharmaceutical composition further comprises or is further characterised by: a tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: a tonicifier, and 0.01 to 2 mg/mL of either a PVP surfactant or Polysorbate 80.

In a particular embodiment, the liquid pharmaceutical composition comprises or is characterised by a sugar stabiliser, and the liquid pharmaceutical composition further comprises or is further characterised by: a tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: a tonicifier, and 0.01 to 1 mg/mL PVP surfactant having a weight average molecular weight of between 1900 and 3100.

In a particular embodiment, the liquid pharmaceutical composition comprises or is characterised by a sugar stabiliser, and the liquid pharmaceutical composition further comprises or is further characterised by: a tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: a metal (hydroxy)mono-/di-carboxylate salt tonicifier.

In a particular embodiment, the liquid pharmaceutical composition comprises or is characterised by a sugar stabiliser, and the liquid pharmaceutical composition further comprises or is further characterised by: a metal (hydroxy)mono-/di-carboxylate salt tonicifier, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: a metal (hydroxy)mono-/di-carboxylate salt tonicifier, and a surfactant.

In a particular embodiment, the liquid pharmaceutical composition comprises or is characterised by a sugar stabiliser, and the liquid pharmaceutical composition further comprises or is further characterised by: a metal (hydroxy)mono-/di-carboxylate salt tonicifier, a surfactant, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: a metal (hydroxy)mono-/di-carboxylate salt tonicifier, and a PVP surfactant.

In a particular embodiment, the liquid pharmaceutical composition comprises or is characterised by a sugar stabiliser, and the liquid pharmaceutical composition further comprises or is further characterised by: a metal (hydroxy)mono-/di-carboxylate salt tonicifier, a PVP surfactant, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: a metal (hydroxy)mono-/di-carboxylate salt tonicifier, and a PVP surfactant having a weight average molecular weight of between 1900 and 3100 or Polysorbate 80.

In a particular embodiment, the liquid pharmaceutical composition comprises or is characterised by a sugar stabiliser, and the liquid pharmaceutical composition further comprises or is further characterised by: a metal (hydroxy)mono-/di-carboxylate salt tonicifier, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: a metal (hydroxy)mono-/di-carboxylate salt tonicifier, and 0.01 to 2 mg/mL of either a PVP surfactant or Polysorbate 80.

In a particular embodiment, the liquid pharmaceutical composition comprises or is characterised by a sugar stabiliser, and the liquid pharmaceutical composition further comprises or is further characterised by: a metal (hydroxy)mono-/di-carboxylate salt tonicifier, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: a metal (hydroxy)mono-/di-carboxylate salt tonicifier, and 0.01 to 1 mg/mL PVP surfactant having a weight average molecular weight of between 1900 and 3100.

In a particular embodiment, the liquid pharmaceutical composition comprises or is characterised by a sugar stabiliser, and the liquid pharmaceutical composition further comprises or is further characterised by: a metal (hydroxy)mono-/di-carboxylate salt tonicifier, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: either sodium gluconate or sodium chloride.

In a particular embodiment, the liquid pharmaceutical composition comprises or is characterised by a sugar stabiliser, and the liquid pharmaceutical composition further comprises or is further characterised by: either sodium gluconate or sodium chloride, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: either sodium gluconate or sodium chloride, and a surfactant.

In a particular embodiment, the liquid pharmaceutical composition comprises or is characterised by a sugar stabiliser, and the liquid pharmaceutical composition further comprises or is further characterised by: either sodium gluconate or sodium chloride, a surfactant, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: either sodium gluconate or sodium chloride, and a PVP surfactant.

In a particular embodiment, the liquid pharmaceutical composition comprises or is characterised by a sugar stabiliser, and the liquid pharmaceutical composition further comprises or is further characterised by: either sodium gluconate or sodium chloride, a PVP surfactant, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: either sodium gluconate or sodium chloride, and a PVP surfactant having a weight average molecular weight of between 1900 and 3100 or Polysorbate 80.

In a particular embodiment, the liquid pharmaceutical composition comprises or is characterised by a sugar stabiliser, and the liquid pharmaceutical composition further comprises or is further characterised by: either sodium gluconate or sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: either sodium gluconate or sodium chloride, and 0.01 to 2 mg/mL of either a PVP surfactant or Polysorbate 80.

In a particular embodiment, the liquid pharmaceutical composition comprises or is characterised by a sugar stabiliser, and the liquid pharmaceutical composition further comprises or is further characterised by: either sodium gluconate or sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: either sodium gluconate or sodium chloride, and 0.01 to 1 mg/mL PVP surfactant having a weight average molecular weight of between 1900 and 3100.

In a particular embodiment, the liquid pharmaceutical composition comprises or is characterised by a sugar stabiliser, and the liquid pharmaceutical composition further comprises or is further characterised by: either sodium gluconate or sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: either 45 mM and 230 mM sodium gluconate or 25 mM to 75 mM sodium chloride.

In a particular embodiment, the liquid pharmaceutical composition comprises or is characterised by a sugar stabiliser, and the liquid pharmaceutical composition further comprises or is further characterised by: either 45 mM and 230 mM sodium gluconate or 25 mM to 75 mM sodium chloride, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: either 45 mM and 230 mM sodium gluconate or 25 mM to 75 mM sodium chloride, and a surfactant.

In a particular embodiment, the liquid pharmaceutical composition comprises or is characterised by a sugar stabiliser, and the liquid pharmaceutical composition further comprises or is further characterised by: either 45 mM and 230 mM sodium gluconate or 25 mM to 75 mM sodium chloride, a surfactant, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: either 45 mM and 230 mM sodium gluconate or 25 mM to 75 mM sodium chloride, and a PVP surfactant.

In a particular embodiment, the liquid pharmaceutical composition comprises or is characterised by a sugar stabiliser, and the liquid pharmaceutical composition further comprises or is further characterised by: either 45 mM and 230 mM sodium gluconate or 25 mM to 75 mM sodium chloride, a PVP surfactant, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: either 45 mM and 230 mM sodium gluconate or 25 mM to 75 mM sodium chloride, and a PVP surfactant having a weight average molecular weight of between 1900 and 3100 or Polysorbate 80.

In a particular embodiment, the liquid pharmaceutical composition comprises or is characterised by a sugar stabiliser, and the liquid pharmaceutical composition further comprises or is further characterised by: either 45 mM and 230 mM sodium gluconate or 25 mM to 75 mM sodium chloride, a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: either 45 mM and 230 mM sodium gluconate or 25 mM to 75 mM sodium chloride, and 0.01 to 2 mg/mL of either a PVP surfactant or Polysorbate 80.

In a particular embodiment, the liquid pharmaceutical composition comprises or is characterised by a sugar stabiliser, and the liquid pharmaceutical composition further comprises or is further characterised by: either 45 mM and 230 mM sodium gluconate or 25 mM to 75 mM sodium chloride, 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a sugar stabiliser, and the liquid pharmaceutical composition further comprises or is further characterised by: either 45 mM and 230 mM sodium gluconate or 25 mM to 75 mM sodium chloride, and 0.01 to 1 mg/mL PVP surfactant having a weight average molecular weight of between 1900 and 3100.

In a particular embodiment, the liquid pharmaceutical composition comprises or is characterised by a sugar stabiliser, and the liquid pharmaceutical composition further comprises or is further characterised by: either 45 mM and 230 mM sodium gluconate or 25 mM to 75 mM sodium chloride, 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a tonicifier, and the liquid pharmaceutical composition further comprises or is further characterised by: wherein the composition has a pH between 5.0 and 5.5.

In a particular embodiment, the liquid pharmaceutical composition comprises or is characterised by a tonicifier, and the liquid pharmaceutical composition further comprises or is further characterised by: a surfactant.

In a particular embodiment, the liquid pharmaceutical composition comprises or is characterised by a tonicifier, and the liquid pharmaceutical composition further comprises or is further characterised by: a surfactant, and wherein the composition has a pH between 5.0 and 5.5.

In a particular embodiment, the liquid pharmaceutical composition comprises or is characterised by a tonicifier, and the liquid pharmaceutical composition further comprises or is further characterised by: a PVP surfactant.

In a particular embodiment, the liquid pharmaceutical composition comprises or is characterised by a tonicifier, and the liquid pharmaceutical composition further comprises or is further characterised by: a PVP surfactant, and wherein the composition has a pH between 5.0 and 5.5.

In a particular embodiment, the liquid pharmaceutical composition comprises or is characterised by a tonicifier, and the liquid pharmaceutical composition further comprises or is further characterised by: a PVP surfactant having a weight average molecular weight of between 1900 and 3100 or Polysorbate 80.

In a particular embodiment, the liquid pharmaceutical composition comprises or is characterised by a tonicifier, and the liquid pharmaceutical composition further comprises or is further characterised by: a PVP surfactant having a weight average molecular weight of between 1900 and 3100 or Polysorbate 80, and wherein the composition has a pH between 5.0 and 5.5.

In a particular embodiment, the liquid pharmaceutical composition comprises or is characterised by a tonicifier, and the liquid pharmaceutical composition further comprises or is further characterised by: 0.01 to 2 mg/mL of either a PVP surfactant or Polysorbate 80.

In a particular embodiment, the liquid pharmaceutical composition comprises or is characterised by a tonicifier, and the liquid pharmaceutical composition further comprises or is further characterised by: 0.01 to 2 mg/mL of either a PVP surfactant or Polysorbate 80, and wherein the composition has a pH between 5.0 and 5.5.

In a particular embodiment, the liquid pharmaceutical composition comprises or is characterised by a tonicifier, and the liquid pharmaceutical composition further comprises or is further characterised by: 0.01 to 1 mg/mL PVP surfactant having a weight average molecular weight of between 1900 and 3100.

In a particular embodiment, the liquid pharmaceutical composition comprises or is characterised by a tonicifier, and the liquid pharmaceutical composition further comprises or is further characterised by: 0.01 to 1 mg/mL PVP surfactant having a weight average molecular weight of between 1900 and 3100, and wherein the composition has a pH between 5.0 and 5.5.

In a particular embodiment, the liquid pharmaceutical composition comprises or is characterised by a surfactant, and the liquid pharmaceutical composition further comprises or is further characterised by: wherein the composition has a pH between 5.0 and 5.5.

### Method of manufacturing a liquid pharmaceutical composition

The present invention provides a method of manufacturing a liquid pharmaceutical composition, suitably as defined herein. The method suitably comprises mixing together, in any particular order deemed appropriate, any relevant components required to form a liquid pharmaceutical composition as defined herein. The skilled person may refer to the Examples or techniques well known in the art for forming liquid pharmaceutical compositions (especially those for injection *via* syringe). Different embodiments will suitably require different combinations of components to be mixed, potentially in different amounts. The skilled person can readily deduce such combinations and amounts by reference to the foregoing disclosure relating to the liquid pharmaceutical composition.

Suitably the method involves mixing together the relevant components suitably, in a diluent (e.g. water), suitably so that all of the components are (substantially or entirely) dissolved in the diluent.

The method may involve first preparing a pre-mixture (or pre-solution) of some or all components (optionally with some or all of the diluent) excluding adalimumab, and adalimumab may then itself (optionally with or pre-dissolved in some of the diluent) be mixed with the pre-mixture (or pre-solution) to afford the liquid pharmaceutical composition, or a composition to which final components are then added to furnish the final liquid pharmaceutical composition. Most suitably, the pre-mixture contains all components except for the adalimumab and optionally also some diluent (which may be used to pre-dissolve adalimumab), suitably so that adalimumab is added to a mixture which offers optimal stabilisation of adalimumab. Suitably the aforementioned pre-mixture is prepared with the desired pH for the final liquid pharmaceutical formulation.

Suitably, the method involves forming a buffer system, suitably a buffer system comprising a buffering agent as defined herein. The buffer system is suitably formed in a pre-mixture prior to the addition of adalimumab, though the buffer system may optionally be formed with adalimumab present. The buffer system may be formed through simply mixing the buffering agent (supplied ready-made) with its acid/base conjugate (suitably in appropriate relative quantities to provide the desired pH - this can be determined by the skilled person either theoretically or experimentally). In the case of an acetate buffer system, this means mixing sodium acetate with acetic acid. Alternatively, the buffer system may be formed through adding a strong acid (e.g. HCl) to the buffering agent (e.g. sodium acetate) in order to form *in situ* the acid/base conjugate (e.g. acetic acid) (again suitably in appropriate relative quantities to provide the desired pH). Alternatively, the buffer system may be formed through adding a strong base (e.g. sodium hydroxide) to the acid/base conjugate (e.g. acetic acid) of the buffering agent (e.g. sodium acetate) in order to form *in situ* the buffering agent (again suitably in appropriate relative quantities to provide the desired pH). The pH of either the pre-mixture of final liquid pharmaceutical composition may be judiciously adjusted by adding the required quantity of strong base or strong acid, or even a quantity of buffering agent or acid/base conjugate.

In certain embodiments, the buffering agent and/or buffer system is preformed as a separate mixture, and the buffer system is transferred to a precursor of the liquid pharmaceutical composition (comprising some or all components save for the buffering agent and/or buffer system, suitably comprising adalimumab and potentially only adalimumab) *via* buffer exchange (e.g. using diafiltration until the relevant concentrations or osmolality is reached). Additional excipients may be added thereafter if necessary in order to produce the final liquid pharmaceutical composition. The pH may be adjusted once or before all the components are present.

Any, some, or all components may be pre-dissolved or pre-mixed with a diluent prior to mixing with other components.

The final liquid pharmaceutical composition may be filtered, suitably to remove particulate matter. Suitably filtration is through filters sized at or below 1 µm, suitably at 0.22µm. Suitably, filtration is through either PES filteres or PVDF filters, suitably with 0.22 µm PES filters.

The present invention also provides a liquid pharmaceutical composition obtainable by, obtained by, or directly obtained by the method of manufacture herein described.

### Drug-delivery Device

The present invention provides a drug delivery device comprising a liquid pharmaceutical composition as defined herein. Suitably the drug delivery device comprises a chamber within which the pharmaceutical composition resides. Suitably the drug delivery device is sterile.

The drug delivery device may a vial, ampoule, syringe, injection pen (e.g. essentially incorporating a syringe), or intravenous bag. Most suitably the drug delivery device is a syringe, suitably an injection pen. Suitably the syringe is a glass syringe. Suitably the syringe comprises a needle, suitably a 29G ½" needle.

The present invention provides a method of manufacturing a drug delivery device, suitably as defined herein, the method comprising incorporating a liquid pharmaceutical composition as defined herein within a drug delivery device. Such manufacture typically involves charging the liquid pharmaceutical composition as defined herein to a syringe, suitably *via* a needle affixed thereto. The needle may thereafter be removed, replaced, or remain.

According to an eleventh aspect of the present invention there is provided a drug delivery device obtainable by, obtained by, or directly obtained by a method of manufacture defined herein.

### Package

The present invention provides a package comprising a liquid pharmaceutical composition as defined herein. Suitably the package comprises a drug delivery device as defined herein, suitably a plurality of drug delivery devices. The package may comprise any suitable container for containing one or more drug delivery devices.

The present invention provides a method of manufacturing a package, the method comprising incorporating a liquid pharmaceutical composition as defined herein within a package. Suitably this is achieved by incorporating said liquid pharmaceutical composition within one or more drug delivery devices, and thereafter incorporating the one or more pre-filled drug delivery devices into a container present within the package.

The present invention provides a package obtainable by, obtained by, or directly obtained by a method of manufacture defined herein.

### Kit of Parts

The present invention provides a kit of parts comprising a drug delivery device (without the liquid pharmaceutical composition incorporated therein), a liquid pharmaceutical composition as defined herein (optionally contained in a separate package or container), and optionally a set of instructions with directions regarding the administration (e.g. sub-cutaneous) of the liquid pharmaceutical composition. The user may then fill the drug delivery device with the liquid pharmaceutical composition (which may be provided in a vial or ampoule or such like) prior to administration.

### Uses of Pharmaceutical Liquid Composition and Methods of Treatment

The present invention also provides a method of treating a disease or medical disorder; a liquid pharmaceutical composition for use in therapy; a use of a liquid pharmaceutical composition in the manufacture of a medicament for the treatment of a disease or disorder; a method of treating a tumour necrosis factor-alpha (TNF-α)-related autoimmune disease; a liquid pharmaceutical composition for use in the treatment of a tumour necrosis factor-alpha (TNF-α)-related autoimmune disease; a use of a liquid pharmaceutical composition in the manufacture of a medicament for the treatment of a tumour necrosis factor-alpha (TNF-α)-related autoimmune disease; a method of treating rheumatoid arthritis, psoriatic arthritis, ankylosing spondylitis, Crohn's disease, ulcerative colitis, moderate to severe chronic psoriasis and/or juvenile idiopathic arthritis; a liquid pharmaceutical composition for use in the treatment of rheumatoid arthritis, psoriatic arthritis, ankylosing spondylitis, Crohn's disease, ulcerative colitis, moderate to severe chronic psoriasis and/or juvenile idiopathic arthritis; and a use of a liquid pharmaceutical composition in the manufacture of a medicament for the treatment of rheumatoid arthritis, psoriatic arthritis, ankylosing spondylitis, Crohn's disease, ulcerative colitis, moderate to severe chronic psoriasis and/or juvenile idiopathic arthritis; as defined herein.

The liquid pharmaceutical compositions defined herein may be used to treat any one or more of the aforementioned diseases or medical disorders. In a particular embodiment, the liquid pharmaceutical compositions are used to treat rheumatoid arthritis, Crohn's disease and psoriasis.

The liquid pharmaceutical compositions are suitably parenterally administered, suitably *via* sub-cutaneous injection.

### EXAMPLES

Stability studies performed upon a wide variety of liquid adalimumab formations helped the inventors to identify some of the key parameters influencing adalimumab formulation stability, especially the stability of adalimumab itself. Such stability studies generally involved exposing the formulations to stress (e.g. heat, light, agitation, long-term storage) and taking appropriate time course measurements (i.e. before and after stress). By way of example, purity measurements were generally taken using a bioanalyzer; unfolding temperatures were generally measured using DSF; isoform profiles were obtained with iCE280; protein content was assessed by OD; protein aggregation was determined *via* SE-HPLC; turbity was assessed using nephelometry; whilst other measurements, such as pH and osmolality, were taken using well known methods.

On the basis of the results from these stability studies, a number of formulations were selected as promising candidates for further study, including those shown in Table 1 below:

**Table 1 - Promising Formulation Candidates for Further Study**

| **No.** | **Active (mg/mL)** | **Buffer (mM)** | **Stabiliser (mg/mL)** | **Tonicifier (mg/mL)** | **Surfactant (mg/mL)** | **pH** |
|---|---|---|---|---|---|---|
| 1 | Adalimumab | Acetate | Trehalose dihydrate | NaCl | Kollidon 12 PF | 5.2 |
| | (50) | (10) | (76)* | (2.92)** | (0..01-1)^{#} | |
| 2 | Adalimumab | Acetate | Inositol | NaCl | Polysorbate 80 | 5.2 |
| | (50) | (10) | (10-40)^{##} | (2.92)** | (1)*** | |
| 3 | Adalimumab | Acetate | Inositol | NaCl | Kollidon 12 PF | 5.2 |
| | (50) | (10) | (10-40)^{##} | (2.92)** | (0.01-1)^{#} | |
| 4 | Adalimumab | Acetate | Inositol | Sodium gluconate | Kollidon 12 PF | 5.2 |
| | (50) | (10) | (10-40)^{##} | (10-50)^{###} | (0.01-1)^{#} | |

| | | | | | | |
|---|---|---|---|---|---|---|
| *corresponding to 200 mM trehalose; **corresponding to 50 mM NaCl; ***corresponding to 0.76 mM polysorbate 80; #corresponding to approx 0.003-0.5 mM Kollidon; ##corresponding to approx 55-225 mM Inositol; ###corresponding to about 46-230 mM sodium gluconate. | | | | | | |

Formulations having one or more components present in a range of concentrations can be extrapolated to a plurality of formulations covering at least both end points and a mid point (i.e. half way between the two end points) of the given range. Multiple permutations may exist where multiple components are present in a range of concentrations.

The formulations of Table 1 (including any extrapolations and permutations) can be manufactured utilising a preformulated, surfactant-free DS material, which can be obtained by methods well known in the art from commercially available Humira^{®}. An aliquot of the DS may be diafiltrated with 10 mM sodium acetate/acetic acid buffer at pH 5.0 until a three-fold volume exchange with the buffer has been achieved. Then the required excipients may be added to the buffer-exchanged DS materials and the pH adjusted to the target by addition of a diluted solution of sodium hydroxide. Each formulation may be subsequently filtered through 0.22 µm PES filters.

Such formulations can be readily incorporated within pre-filled glass syringes with 29G ½" needles).

The various stability studies performed by the Applicant and the foregoing specification supports the technical contribution made to the art by the present invention and various sub-inventions disclosed herein. Moreover, the specific embodiments disclosed herein define highly credible candidate formulations which may, amongst other things, be used during the process of lead generation, optimisation, and ultimate product development in the relevant field.

The following Examples are included merely to illustrate the invention are not intended to be limiting.

### Materials and Equipment

Acetic acid (glacial) 100%, Adalimumab DS, Citric Acid Monohydrate, Dibasic sodium phosphate dihydrate, Myo-Inositol, Kollidon 12PF, Mannitol, Monobasic sodium phosphate dehydrate, Polysorbate 80, Sodium chloride, Sodium citrate, Sodium gluconate (D-Gluconic acid sodium salt), Sodium hydroxide solution 30% were ingredients used in the following Examples, and were all readily commercially sourced or produced in-house.

The following analytical equipment was deployed during data collection:

| **Item** | **Mod.** | **Manufacturer** |
|---|---|---|
| Analytical scales | AX205, PG2002-S | Mettler Toledo |
| CE-SDS | PA 800 Plus | AB Sciex |
| Calibrated pipettes | P2, P10, P20, P100, P200, P1000 | Gilson |
| Densimeter | DE45 Delta range | Mettler Toledo |
| HPLC | Alliance | Waters |
| iCE3 | Fast IEF Analyzer | Convergent Bioscience |
| Osmometer | Osmomat 030/D | Gonotec |
| Particle analyzer | PAMAS SVSS | Aminstruments |
| pH meters | Seven Multi | Mettler Toledo |
| Refrigerators | +2-8°C | Angelantoni |
| Rheometer | Discovery HR-3 | TA Instruments |
| Spectrofluorimeter | Fluromax-3 | Jobin Yvon |
| Thermostatic cabinets | +25°C, +40°C | Angelantoni |
| Turbidimeter | 2100AN IS | Hach Lange |
| UV Spectrophotometer | Lambda 35 | Perkin Elmer |

### Analytical Methods and Protocols

The aforementioned analytical equipment was deployed in the following analytical methods:

| **No.** | **Analytical Method** | **Scope of the test** |
|---|---|---|
| 1 | CE-SDS (Red and Non-Red) | Protein purity and fragments |
| 2 | Fluorescence | III structure |
| 3 | iCE3 | Isoforms profile |
| 4 | OD | Protein Content |
| 5 | SE-HPLC | Aggregates determination |
| 6 | Osmolality | Osmolality of solution |
| 7 | pH | pH determination |

### 1. Purity- Bioanalyzer (CE-SDS)

A 2100 Bioanalyzer was used. Protocols can be found in the relevant instruction manual. However, the protocols have been additionally refined as follows.

### Solutions:

### Gel- Dye Mix ( Staining solution ):

Add 25µL of 230plus dye concentrate to a protein 230plus gel matrix tube. Vortex well, and spin down the tube for 15 seconds. Transfer to a spin filter and centrifuge at 2500rpm for at least 20min. The solution is ready to use. Store the solution at +5±3°C for not more than 4 weeks.

### Destaining Solution:

Pipette 650µL of gel matrix into a spin filter. Centrifuge at 2500rpm for at least 25 min. Store the solution at +5±3°C for not more than 4 weeks.

### Sample Buffer:

It is recommended to divide the 200µL of sample buffer into aliquots of 25µL and defreeze aliquot for eaeh chip. Store the Sample buffer stock solution and the aliquots at -20°C, not longer than the expiring date provided by the supplier.

### Maleimide Stock Solution:

Dissolve 23.4 mg of Male imide in ImL MilliQ water (O.24M). Vortex well the solution. Subsequently dilute the solution 1:4 with MilliQ water. (e.g. 50µLStock Sol. + 150µLMilliQ). The final concentration of the diluted Maleimide solution is 60mM. (Since no data is available yet on the stability of this solution, it must be prepared freshly before starting each analytical session).

### OTf-Solution:

For the analysis of Adalimurnab samples the reducing solution must be prepared with 1M DTT, therefore dissolve 154.0 mg DTT in 1mL MilliQ Water.

### Non-reducing solution:

Add 1 µl. of MilliQ water to a sample buffer aliquot (25 µL) and vortex for 5 seconds. Use the non-reducing solution within its preparation day.

Reducing Solution:

Add 1 µl, of the according DTf-Solution to a sample buffer aliquot (25 µL) and vortex for 5 seconds. Use the reducing solution within its preparation day.

Sample Preparation:
- Samples are analyzed at the concentration ranging between 2.4 - 3 mg/ml..
- If it's necessary the samples can be diluted to the target concentration using Milli Q water.

Samples are prepared according to the Reagent Kit Guide using the reducing and non reducing sample buffers according to the instruction in the Reagent Kit Guide and also mentioned above. It is strongly recommended to use, differently from the guide, greater volumes to achieve reproducible and accurate results. An example how to prepare the ladder and the samples is reported below:

**Sample preparation solution Reducing and Non Reducing condition.**

| Reagent | Volume µl | Total Volume µL |
|---|---|---|
| Sample diluted at 3mg/ml | 3 µL | 6 µL |
| Sample buffer (reducing or not reducing | 2 µL | |
| Maleimide solution | 1 µL | |
| Samples have to be mixed {via vortex} well and spinned down All Samples and the Ladder are heated 5 minutes at 70°C | | |
| MilliQ water | 84 µL | 90 µL |
| vortex well and spin down | | |
| Loading 6 µL all Sample and Ladder | | |

Note 1: For the IPCs whose concentration is between 2.4 mg/ml and 3.0mg/mL, the sample preparation follows the table above but the volume of MilliQ Water added after sample heating is calculated in order to reach a final protein concentration of 0.1 mg/ml.

An example of sample preparation for a sample having concentration between 2.4 and 3.0 mg/mL, is reported here below:

**Sample preparation solution Reducing and Non Reducing condition**

| Reagent | Volume µl | Total Volume µL |
|---|---|---|
| Sample (2.6 mg/mL) | 3 µL | 6 µL |
| Sample buffer (reducing or not reducing) | 2 µL | |
| Maleimide solution | 1 µL | |
| Samples have to be mixed {via vortex} well and spinned down All Samples and the Ladder are heated 5 minutes at 70°C | | |
| MilliQ water | 72 µL | 78 µL |
| vortex well and spin down | | |
| Loading 6 µL all Sample and Ladder | | |

Note 2: All wells have to be loaded. If the sample number is lower than the available wells, the empty wells can be used for additional duplicates or blank samples.
Preparing the System and the Chip:
- To clean the system before and as well after an analysis fill the "Electrode Cleaner" with 600IIL MilliQ Water and place it into the Agilent 2100 Bioanalyzer, close the lid and let the system cease. No further action is required.
- Adjust the base-plate of the chip priming station to position "*A*" and the syringe clip to its middle position.

**Preparing the chip**

| System Preparation |
|---|
| Insert a new Protein Chip in the priming station |
| Pipette 12µL of Gel-Dye Mix in the well marked G (Up right) |
| Set the plunger at 1mL and close the chip priming station. |
| Press the plunger until it is held by the clip |
| Wait 60 seconds and then release the clip |
| Wait 5 seconds and slowly pull back the plunger to the 1 mL mark |
| Open the chip priming station |
| Remove the solution in this well |
| Pipette 12µL of Gel-Dye Mix, into the well marked G (UP right) and in all remaining wells marked with G |
| Pipette 12µL of Destaining Solution in the well marked with DS |

### Loading of the Ladder and the Sample:

- Transfer 6µL of each sample into a sample well and as well 6µL of the ladder in the dedicated well, which is clearly indicated with a ladder symbol.

Place the chip into the Agilent 2100 Bioanalyzer and start the analysis within 5 min.

**Example of sample set**

| **Well** | **Sample** | **Amount µL** |
|---|---|---|
| **1** | Blank | **6** |
| **2** | Blank | **6** |
| **3** | Unknown sample1 rep1 | **6** |
| **4** | Unknown sample1 rep2 | **6** |
| **5** | Unknown sample2 rep1 | **6** |
| **6** | Unknown sample2 rep2 | **6** |
| **7** | Unknown sample3 rep1 | **6** |
| **8** | Unknown sample3 rep2 | **6** |
| **9** | Current Reference Material rep1 | **6** |
| **10** | Current Reference Material rep2 | **6** |
| **Ladder** | **Ladder** | **6** |

### Data Analysis and evaluation of the results:

To gain results the following minimum operations have to be executed
- Place the chip in the specific spot and close the lid.
- In the instrument context select Assay - Electrophoresis- Protein- Protein 230 Plus.
- Click on START to start the analysis, which is completed within 30 minutes.
- The raw data are shown by clicking on "Data Analysis" where all experiments, carried out at the day, are listed. Click on the experiment of interest and select it.
- The gel generated from the chosen experiment is automatically opened.
- Data can be shown as an electropherograrn or gel-like image.

Detailed information regarding the integration of the peaks in the electropherogram (to gain the purity data) is included in the manual of the software. The purity of a sample is automatically given by the system by automatic integration, but if needed, the manual integration can be applied.

Results:

In non reducing condition the results are indicated as %Purity, and %LMW (sum of peaks before monomer).

In reducing condition the results are indicated as % Purity, as sum of heavy chain and light chain.

The indicative molecular weight values are reported in the table below:

**Indicative molecular weight of Adalimumab**

| Condition | Results | KDa |
|---|---|---|
| **Non Reducing** | **Monomer** | 151 |
| **Reducing** | LC | 27 |
| | HC | 58 |

### 2. Unfolding Temperature - DSF

DSF (differential scanning fluorimetry) was performed as follows:

2 microliters of Sypro Orange (Orange protein gel stain, cod. S6650, Life Technologies) previously diluted 500-fold in water for injection were added to 20 microliters of drug product solution. Upon addition of Sypro Orange, the DP solutions (triplicate preparation) are filled in 96-well plates (MicroAmp Fast 96-W Reaction Plate 0.1 mL, cod. 4346907). The plates are then sealed with a protective, transparent cover (MicroAmp Optical Adhesive Film, cod. 4311971) and then subjected to centrifugation to remove air bubbles. The plates are then inserted in the 7500 Fast Real-Time AB Applied Biosystem PCR system and scanned for emission profiles at temperatures from room temperature to 90 - 100°C. The dependence of intensity of fluorescence emission on temperature is a curve which typically shows an inversion point/discontinuation at the denaturation temperature, parameter used to compared the different compositions.

### 3. Isoforms profile - iCE280

clEF by iCE280 (isoforms profile): After purification and removal of salts with centrifugation in Amicon Ultra-4 centrifugal devices (cut off 10 kDa), the samples were pre-diluted to the concentration of 5.0 mg/mL with purified water. A second dilution was then made to 1.0 mg/mL with a solution composed of: methyl cellulose, Pharmalyte 5-8 (GE Healthcare), Pharmalyte 8 - 10.5 (GE Healthcare), low pl marker 7.05 (Protein Simple), high pl marker 9.50 (Protein Simple) and purified water. Upon dilution the samples were centrifuged at 10000 rpm for 3 minutes. An additional centrifugation step ( 2 minutes at 7000 rpm) is then conducted on 150 microL of each sample transferred in glass inserts. The clEF (capillary isoelectric focusing) was carried out with the iCE280 system by Protein Simple, using capillary cartridges Fc with 100 micron ID coating and total length of 50 nm (Cat. No. 101700/101701 by Protein Simple). The separation of the various isoforms is made using 100 mM sodium hydroxide (in 0.1% methyl cellulose) as a cathodic solution and 80 mM o-phosphoric acid (in 0.1% methyl cellulose) as an anodic solution. The electropherogram is acquired at 280 nm over pre-focusing and focusing times of 1 and 6 minutes respectively, at a voltage of 1500 V (pre-focusing) and 3000 V (focusing).

### 4. Protein content - OD

OD (protein content) measurements were taken on samples which were initially diluted gravimetrically (triplicate independent dilutions were made) with relevant buffer or placebo from starting concentration to about 10 mg/mL. The diluted solutions were tested for absorbance at 280 and 320 nm in 0.1 cm pathlength quartz cuvettes, at room temperature, with a double-beam spectrophotometer (Lambda35 by Perkin Elmer). The value of 1.35 was used as molar extinction coefficient of Adalimumab.

### 5. Aggregates determination - SE-HPLC

The samples were diluted with DPBS 1X to a concentration of 0.5 mL and injected (20 microL injection volume) in a Column TSK gel Super SW3000 4.6mm ID X30.0cm cod.18675 by Tosoh maintaining isocratic conditions (mobile phase: 50mM Sodium Phosphate + 0.4M Sodium perchlorate, pH 6.3 ± 0.1). UV detection was made at 214 nm at a flow rate of 0.35 mL. The duration of each analytical run was 15 minutes. Prior to the analysis the samples were maintained at 2-8°C in the autosampler of the Waters Alliance HPLC systems used for this test.

### 6. Osmolality determination - Osmolality

Osmolality was measured based on the cryoscopic characteristic of the solutions. The tests were conducted with an Osmomat 030-D by Gonotech subjecting 50 microL of the sample to freezing. The freezing temperature depends on the osmolality of the solution (i.e. on the presence of agents dissolved such as salts, sugars, other ionic and non ionic species, etc).

### 7. pH determination - pH

pH was determined using potentiometric measurements conducted at room room temperature with Mettler Toledo Seven Multi pH meters.

### Storage Conditions for Stress Testing

Various formulations were subjected to the aforementioned analyses following their storage (and thermal stress testing) under the conditions indicated:

| **Thermal stress** | | **Stability time (weeks)** | | |
|---|---|---|---|---|
| **Methods** | **Test** | **0** | **2w** | **4w** |
| OD | Content | x | - | 40°C |
| SE-HPLC | Aggregates | x | 40°C | 40°C |
| CE-SDS | Purity/Fragments | x | 40°C | 40°C |
| iCE3 | Isoforms profile | x | 40°C | 40°C |
| pH | pH | x | 40°C | 40°C |
| Fluorescence | III structure | x | 40°C | 40°C |
| Osmolality | Osmolality | x | - | - |

### Control and Test Formulations

Table 2 provides details the Control and Test Formulations 1-4 used in the stability studies that follow. All formulations contain 50 mg/mL adalimumab, 10 mM acetate buffer (0.6 mg/mL acetic acid + q.b. NaOH to pH5.2). The formulations additionally contain a sugar stabiliser, surfactant, and tonicifier as described in Table 2.

**Table 2 - Control and Test Formulations**

| **Sample ID** | **Sugar Stabiliser** (mg/mL) | **Surfactant** (mg/mL) | **Tonicifier (mg/mL)** |
|---|---|---|---|
| Control | Trehalose (75.67) | PS80 (1) | NaCl (2.92) |
| 1 | Trehalose (75.67) | Kollidon 12PF (1) | NaCl (2.92) |
| 2 | Inositol (35) | PS80 (1) | NaCl (2.92) |
| 3 | Inositol (35) | Kollidon 12PF (1) | NaCl (2.92) |
| 4 | Inositol (35) | Kollidon 12PF (1) | Sodium gluconate (10.91) |

All the formulations in Table 2 are manufactured starting from a preformulated, surfactant-free, drug substance (adalimumab) material.

An aliquot of the drug substance is diafiltrated with 10 mM sodium acetate/acetic acid buffer at pH 5.2 (as mentioned above) until a three-fold volume exchange with the buffer is achieved. The required excipients are then added to the buffer-exchanged drug substance materials and, if necessary, the pH is (re)adjusted to the target pH by addition of a diluted solution of sodium hydroxide (or hydrochloric acid). Each formulation was filtered through 0.22 µm PES filters.

Osmolality values (≤ 40 mOsm/kg) were generally used to indicate a satisfactory degree of buffer exchange, with a minimal residual of species coming from the originating DS.

The "control" formulation is a robust and stable comparator formulation upon which stability stress tests have already demonstrated it's viability as a drug product.

### RESULTS OF STABILITY STUDIES

All of the formulations of Table 2 were subjected to the aforementioned analytical tests at various stages in the storage cycle. Since previous stability studies already carried out by the present applicant had indicated the Control formulation to be effective and relatively stable under stress testing, the inventors were particularly interested in the comparative performance of Formulations 1-4.

The results of the tests are presented below.

### Stability at 40°C±2°C

### pH (potentiometric) Stability

There were no statistically discernable differences in pH changes, occuring over the 1 month period of stress testing, between the control and any of the Formulations 1-4. As such, by this measure all of Formulations 1-4 performed as well as the control..

### Protein content (by OD)

As illustrated in Table 3 below, there were no statistically discernable differences in protein content variation, occurring over the 1 month period of stress testing, between the control and any of the Formulations 1-4. As such, by this measure all of Formulations 1-4 performed as well as the control.

**Table 3: Protein content (mg/mL) evaluation up to 4 weeks under stressed conditions**

| | **Stability point** | | |
|---|---|---|---|
| **Sample ID** | **T0** | **2W *40°C*** | **4W *40°C*** |
| Control | 49.2 | 50.5 | 48.9 |
| 1 | 49.3 | 49.0 | 49.1 |
| 2 | 50.1 | 49.3 | 49.7 |
| 3 | 50.4 | 50.3 | 49.5 |
| 4 | 49.2 | 49.0 | 50.1 |

### Electrophoretic purity (by CE-SDS)

The capillary electrophoretic technique aims to highlight both intact forms - heavy chain (HC), non glycosylated heavy chain (NGHC) and light chain (LC) - under reducing conditions, and impurities - fragments and low molecular weights (LMW) - under non-reducing conditions. In this study the impurities were taken into account and the purity extrapolated accordingly.

The %LMW (i.e. impurities count) at Time 0 was very similar for all of the tested formulations, including the control formulation (ADAM DP Acetate). As such, by this measure all of Formulations 1-4 performed substantially as well as the control.

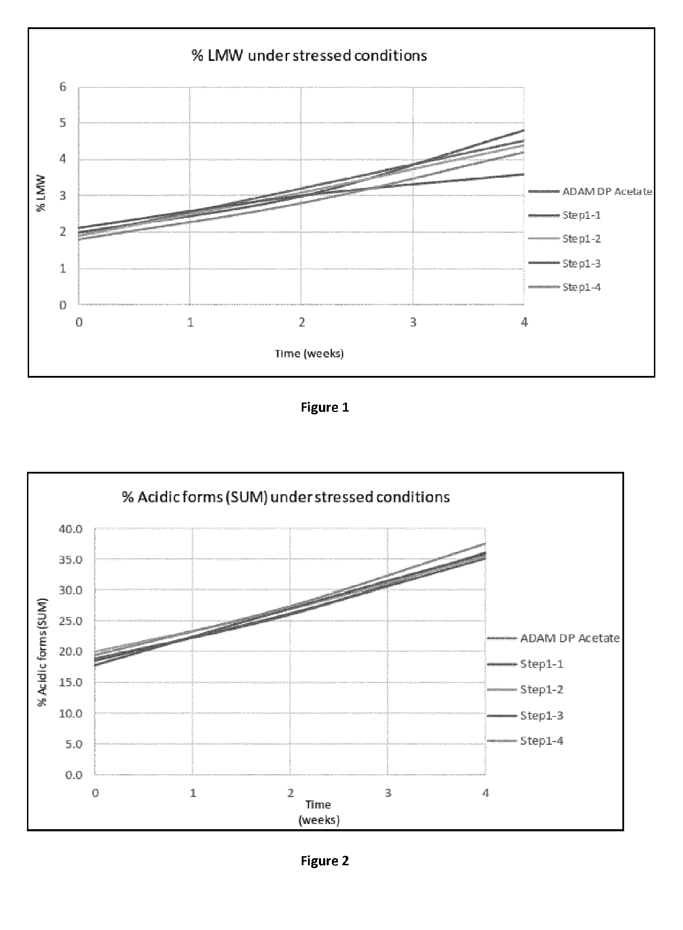

FIG. 1 shows a graphical representation of the change in low molecular weight fragments/impurities (%LMW) over time, under stress conditions, for each of the candidate formulations 1-4 and the control formulation.

As illustrated by FIG. 1 and Table 4 below, all of candidate formulations 1-4 showed comparable changes in LMWs to those of observed for the control formulation when exposed to stressed conditions for up to 1 month: in all cases there was an increase of LMWs of approximately 1.0% per week. As the percentage of LMW increases, the intact forms decrease proportionally.

**Table 4: Impurities (%LMW) evaluation over time (up to 4 weeks) under stressed conditions**

| | **%LMW** | | | |
|---|---|---|---|---|
| **Sample ID** | *T0* | *2W 40°C* | *4W 40°C* | **%LMW: SLOPE/week** |
| Control | 1.9 | 3.2 | 4.5 | **0.7** |
| 1 | 2.0 | 3.0 | 4.8 | **0.7** |
| 2 | 1.9 | 3.1 | 4.4 | **0.6** |
| 3 | 2.1 | 3.0 | 3.6 | **0.4** |
| 4 | 1.8 | 2.8 | 4.2 | **0.6** |

### Aggregates by SE- HPLC

Baseline SE-HPLC measurements at Time 0 showed that the %HMW (i.e. aggregates count - high molecular weights) was very similar for all of the tested formulations, including the control formulation (ADAM DP Acetate). As such, by this measure all of Formulations 1-4 performed substantially as well as the control.

As shown in Table 5 below, minimal differences were observed in the HMWs changes across all the tested formulations, including the control, after 1 month at 40°C: increases in HMWs in the range + 0.1 - 0.2% per week were observed, independent of the formulation in question.

**Table 5: Aggregates (%HMW) evaluation up to 4 weeks under stressed conditions**

| | **%HMW** | | | |
|---|---|---|---|---|
| **Sample ID** | *T0* | *2W 40°C* | *4W 40°C* | **%HMW: SLOPE/week** |
| Control | 0.2 | 0.5 | 0.7 | 0.1 |
| 1 | 0.2 | 0.5 | 0.5 | 0.1 |
| 2 | 0.2 | 0.5 | 0.9 | 0.2 |
| 3 | 0.2 | 0.5 | 0.6 | 0.1 |
| 4 | 0.2 | 0.4 | 0.5 | 0.1 |

### Isoforms profile by iCE3

Capillary isoelectric-focusing performed by iCE is a technique enabling an assessment of the pl (isoelectric point) range along with determination of the abundance of selected isoform clusters.

In this study, the effect of thermal stress upon adalimumab within the different formulations was investigated through evaluating three different types of isoform clusters: acidic, intact and basic forms.

Baseline measurements for the control at Time 0 were comparable to Time 0 baseline measurements for all of the other formulations under investigation.

As illustrated in Table 6 below, isoform profiles change to substantially the same extent over a 1 month period under stressed conditions (+40±2°C, UR 75%±5%) for all formulations, including the control: there is an increase of total acidic forms (Cluster1+Cluster2) of about 4.0% per week. As such, by this measure all of Formulations 1-4 performed substantially as well as the control.

FIG. 2 is a graph showing the formation of "acidic forms" (summation of all) for all of the relevant test formulations (including control) over the relevant time period under stressed conditions.

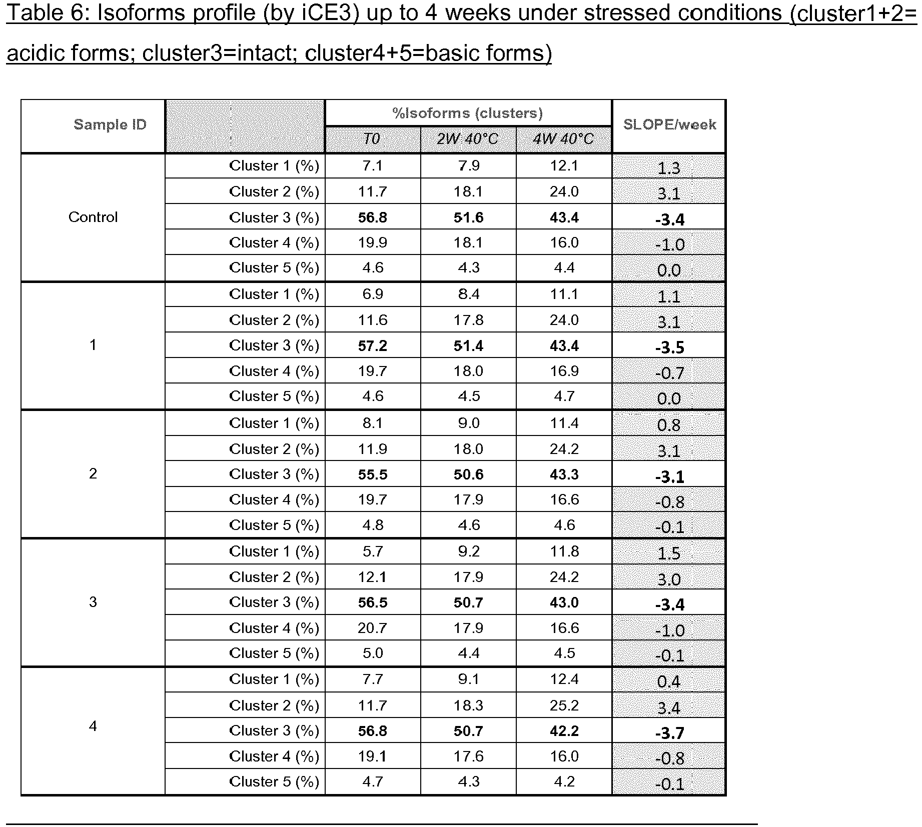

### Fluorescence

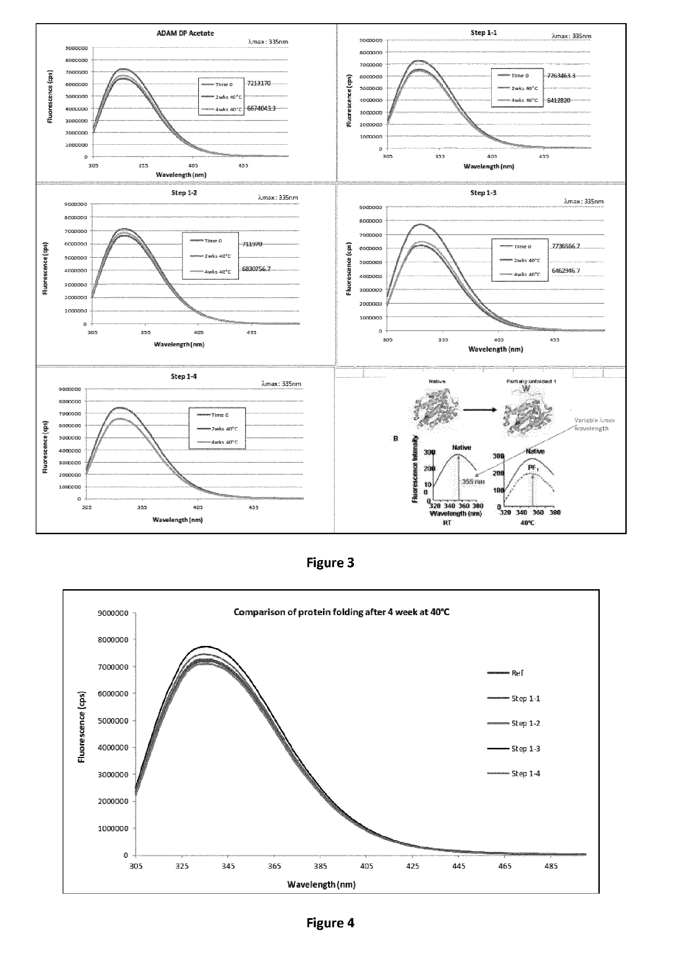

There were no statistically discernable differences in the changes in protein folding, occuring over the 1 month period of stress testing, between the control and any of the Formulations 1-4: : λₘₐₓ from about 7000000 cps to 6000000 cps were encountered for all samples after 4 weeks at 40°C (see FIGs.3 and 4). As such, by this measure all of Formulations 1-4 performed substantially as well as the control.

FIG. 3 illustrates the various fluorescence spectra for each of the tested formulations (including the control) at each point in time under stress testing and how decreases in intensity at the native λₘₐₓ of 355 nm is indicative of partial protein unfolding over time.

FIG. 4 illustrates overlayed fluorescence spectra for each of the tested formulations (including the control) after 4 weeks at 40°C.

### Osmolality

Osmolality is a measure of osmotic pressure exerted by a real solution across a semipermeable membrane, and is expressed in osmoles or milliosmoles per kilogram of solvent (Osmol/kg or mOsmol/kg, respectively). The osmolality of the samples under investigation were determined by measuring freezing point depression.

The Osmolality was performed by diluting the samples (dilution 1:3). The results reported in Table 7 below are expressed as per USP 38 ("A solution can be diluted to bring it within the range of measurement of the osmometer, if necessary, but the results must be expressed as that of the diluted solution and must NOT be multiplied by a dilution factor to calculate the osmolality of the original solution, unless otherwise indicated in the monograph. The molal osmotic coefficient is a function of concentration. Therefore, it changes with dilution.").

**Table 7: Osmolality of formulas under investigation (1:3 diluted sample)**

| **Sample ID** | **Formula** | **Osmolality (mOsmol/Kg)** |
|---|---|---|
| Control | START | 117 |
| 1 | Change 1 Factor: Surfactant | 116 |
| 2 | Change 1 Factor: Stabiliser | 112 |
| 3 | Change 2 Factors: Surf & Stab | 110 |
| 4 | Change 3 Factors | 106 |

As shown in Table 7 above, no major changes were observed in osmolality among samples analysed over the relevant time period. As such, by this measure all of Formulations 1-4 performed substantially as well as the control.

### ABBREVIATIONS

- DoE: Design of experiment
- DP: Drug product
- DS: Drug substance
- DSF: Differential scanning fluorimetry
- OD: Optical density
- PES: Polyethersulphone
- rpm: rounds per minute
- RT: Room Temperature
- SE-HPLC: Size exclusion high performance liquid chromatography
- SMI: Summary manufacturing instructions
- SOP: Standard operating procedure
- WI: Working instruction

## Claims

1. A liquid pharmaceutical composition comprising adalimumab and at least one component selected from the group consisting of: a polyvinylpyrrolidone (PVP) surfactant, a monocyclic sugar stabiliser (which includes any sugar stabiliser that is capable of adopting a molecular structure charactarised as a monocyclic sugar stabiliser), and a metal (hydroxy)mono-/di-carboxylate salt tonicifier;
wherein the composition comprises a surfactant (which is optionally the PVP surfactant) on the proviso that the surfactant is a surfactant other than polysorbate 80 where the composition comprises a trehalose sugar stabiliser.

2. The liquid pharmaceutical composition as claimed in claim 1, wherein the composition comprises adalimumab and at least one component selected from the group consisting of: a polyvinylpyrrolidone (PVP) surfactant, an inositol sugar stabiliser, and a gluconate salt toncifier;
wherein the composition comprises a surfactant (which is optionally the PVP surfactant) on the proviso that the surfactant is a surfactant other than polysorbate 80 where the composition comprises a trehalose sugar stabiliser.

3. The liquid pharmaceutical composition as claimed in any preceding claim, wherein the composition comprises an acetate buffer system (or acetate buffering agent).

4. The liquid pharmaceutical composition as claimed in any preceding claim, wherein the surfactant is the polyvinylpyrrolidone (PVP) surfactant.

5. The liquid pharmaceutical composition as claimed in claim 4, wherein the PVP surfactant has a weight average molecular weight M_{w} of between 1900 and 3100.

6. The liquid pharmaceutical composition as claimed in any preceding claim, wherein the composition comprises at least one component selected from the group consisting of: an inositol sugar stabiliser, and a gluconate salt toncifier.

7. The liquid pharmaceutical composition as claimed in claim 6, wherein the composition comprises both an inositol sugar stabiliser, and a gluconate salt toncifier.

8. The liquid pharmaceutical composition as claimed in claim 1, wherein the composition comprises or is **characterised by** any one of the following combinations of components and/or properties:
i. adalimumab, a PVP surfactant.
ii. adalimumab, a PVP surfactant having a weight average molecular weight of between 1900 and 3100.
iii. adalimumab, 0.01 to 1 mg/mL of a PVP surfactant.
iv. adalimumab, 0.01 to 1 mg/mL PVP surfactant having a weight average molecular weight of between 1900 and 3100.
v. adalimumab, a metal (hydroxy)mono-/di-carboxylate salt tonicifier.
vi. adalimumab, a metal (hydroxy)mono-/di-carboxylate salt tonicifier, and a PVP surfactant.
vii. adalimumab, a metal (hydroxy)mono-/di-carboxylate salt tonicifier, and a PVP surfactant having a weight average molecular weight of between 1900 and 3100.
viii. adalimumab, a metal (hydroxy)mono-/di-carboxylate salt tonicifier, and 0.01 to 1 mg/mL of a PVP surfactant.
ix. adalimumab, a metal (hydroxy)mono-/di-carboxylate salt tonicifier, and 0.01 to 1 mg/mL PVP surfactant having a weight average molecular weight of between 1900 and 3100.
x. adalimumab, sodium gluconate.
xi. adalimumab, sodium gluconate, and a PVP surfactant.
xii. adalimumab, sodium gluconate, and a PVP surfactant having a weight average molecular weight of between 1900 and 3100.
xiii. adalimumab, sodium gluconate, and 0.01 to 1 mg/mL of a PVP surfactant.
xiv. adalimumab, sodium gluconate, and 0.01 to 1 mg/mL PVP surfactant having a weight average molecular weight of between 1900 and 3100.
xv. adalimumab, 45 mM to 230 mM metal (hydroxy)mono-/di-carboxylate salt tonicifier.
xvi. adalimumab, 45 mM to 230 mM metal (hydroxy)mono-/di-carboxylate salt tonicifier, and a PVP surfactant.
xvii. adalimumab, 45 mM to 230 mM metal (hydroxy)mono-/di-carboxylate salt tonicifier, and a PVP surfactant having a weight average molecular weight of between 1900 and 3100.
xviii. adalimumab, 45 mM to 230 mM metal (hydroxy)mono-/di-carboxylate salt tonicifier, and 0.01 to 1 mg/mL of a PVP surfactant.
xix. adalimumab, 45 mM to 230 mM metal (hydroxy)mono-/di-carboxylate salt tonicifier, and 0.01 to 1 mg/mL PVP surfactant having a weight average molecular weight of between 1900 and 3100.
xx. adalimumab, 45 mM to 230 mM sodium gluconate.
xxi. adalimumab, 45 mM to 230 mM sodium gluconate, and a PVP surfactant.
xxii. adalimumab, 45 mM to 230 mM sodium gluconate, and a PVP surfactant having a weight average molecular weight of between 1900 and 3100.
xxiii. adalimumab, 45 mM to 230 mM sodium gluconate, and 0.01 to 1 mg/mL of a PVP surfactant.
xxiv. adalimumab, 45 mM to 230 mM sodium gluconate, and 0.01 to 1 mg/mL PVP surfactant having a weight average molecular weight of between 1900 and 3100.
xxv. adalimumab, a monocyclic sugar stabiliser.
xxvi. adalimumab, a monocyclic sugar stabiliser, and a PVP surfactant.
xxvii. adalimumab, a monocyclic sugar stabiliser, and a PVP surfactant having a weight average molecular weight of between 1900 and 3100.
xxviii. adalimumab, a monocyclic sugar stabiliser, and 0.01 to 1 mg/mL of a PVP surfactant.
xxix. adalimumab, a monocyclic sugar stabiliser, and 0.01 to 1 mg/mL PVP surfactant having a weight average molecular weight of between 1900 and 3100.
xxx. adalimumab, a monocyclic sugar stabiliser, and a metal (hydroxy)mono-/dicarboxylate salt tonicifier.
xxxi. adalimumab, a monocyclic sugar stabiliser, a metal (hydroxy)mono-/dicarboxylate salt tonicifier, and a PVP surfactant.
xxxii. adalimumab, a monocyclic sugar stabiliser, a metal (hydroxy)mono-/dicarboxylate salt tonicifier, and a PVP surfactant having a weight average molecular weight of between 1900 and 3100.
xxxiii. adalimumab, a monocyclic sugar stabiliser, a metal (hydroxy)mono-/dicarboxylate salt tonicifier, and 0.01 to 1 mg/mL of a PVP surfactant.
xxxiv. adalimumab, a monocyclic sugar stabiliser, a metal (hydroxy)mono-/dicarboxylate salt tonicifier, and 0.01 to 1 mg/mL PVP surfactant having a weight average molecular weight of between 1900 and 3100.
xxxv. adalimumab, a monocyclic sugar stabiliser, and sodium gluconate.
xxxvi. adalimumab, a monocyclic sugar stabiliser, sodium gluconate, and a PVP surfactant.
xxxvii. adalimumab, a monocyclic sugar stabiliser, sodium gluconate, and a PVP surfactant having a weight average molecular weight of between 1900 and 3100.
xxxviii. adalimumab, a monocyclic sugar stabiliser, sodium gluconate, and 0.01 to 1 mg/mL of a PVP surfactant.
xxxix. adalimumab, a monocyclic sugar stabiliser, sodium gluconate, and 0.01 to 1 mg/mL PVP surfactant having a weight average molecular weight of between 1900 and 3100.
xl. adalimumab, a monocyclic sugar stabiliser, and 45 mM to 230 mM metal (hydroxy)mono-/di-carboxylate salt tonicifier.
xli. adalimumab, a monocyclic sugar stabiliser, 45 mM to 230 mM metal (hydroxy)mono-/di-carboxylate salt tonicifier, and a PVP surfactant.
xlii. adalimumab, a monocyclic sugar stabiliser, 45 mM to 230 mM metal (hydroxy)mono-/di-carboxylate salt tonicifier, and a PVP surfactant having a weight average molecular weight of between 1900 and 3100.
xliii. adalimumab, a monocyclic sugar stabiliser, 45 mM to 230 mM metal (hydroxy)mono-/di-carboxylate salt tonicifier, and 0.01 to 1 mg/mL of a PVP surfactant.
xliv. adalimumab, a monocyclic sugar stabiliser, 45 mM to 230 mM metal (hydroxy)mono-/di-carboxylate salt tonicifier, and 0.01 to 1 mg/mL PVP surfactant having a weight average molecular weight of between 1900 and 3100.
xlv. adalimumab, a monocyclic sugar stabiliser, and 45 mM to 230 mM sodium gluconate.
xlvi. adalimumab, a monocyclic sugar stabiliser, 45 mM to 230 mM sodium gluconate, and a PVP surfactant.
xlvii. adalimumab, a monocyclic sugar stabiliser, 45 mM to 230 mM sodium gluconate, and a PVP surfactant having a weight average molecular weight of between 1900 and 3100.
xlviii. adalimumab, a monocyclic sugar stabiliser, 45 mM to 230 mM sodium gluconate, and 0.01 to 1 mg/mL of a PVP surfactant.
xlix. adalimumab, a monocyclic sugar stabiliser, 45 mM to 230 mM sodium gluconate, and 0.01 to 1 mg/mL PVP surfactant having a weight average molecular weight of between 1900 and 3100.
l. adalimumab, Inositol.
li. adalimumab, Inositol, and a PVP surfactant.
lii. adalimumab, Inositol, and a PVP surfactant having a weight average molecular weight of between 1900 and 3100.
liii. adalimumab, Inositol, and 0.01 to 1 mg/mL of a PVP surfactant.
liv. adalimumab, Inositol, and 0.01 to 1 mg/mL PVP surfactant having a weight average molecular weight of between 1900 and 3100.
lv. adalimumab, Inositol, and a metal (hydroxy)mono-/di-carboxylate salt tonicifier.
Ivi. adalimumab, Inositol, a metal (hydroxy)mono-/di-carboxylate salt tonicifier, and a PVP surfactant.
Ivii. adalimumab, Inositol, a metal (hydroxy)mono-/di-carboxylate salt tonicifier, and a PVP surfactant having a weight average molecular weight of between 1900 and 3100.
lviii. adalimumab, Inositol, a metal (hydroxy)mono-/di-carboxylate salt tonicifier, and 0.01 to 1 mg/mL of a PVP surfactant.
lix. adalimumab, Inositol, a metal (hydroxy)mono-/di-carboxylate salt tonicifier, and 0.01 to 1 mg/mL PVP surfactant having a weight average molecular weight of between 1900 and 3100.
lx. adalimumab, Inositol, and sodium gluconate.
lxi. adalimumab, Inositol, sodium gluconate, and a PVP surfactant.
lxii. adalimumab, Inositol, sodium gluconate, and a PVP surfactant having a weight average molecular weight of between 1900 and 3100.
lxiii. adalimumab, Inositol, sodium gluconate, and 0.01 to 1 mg/mL of a PVP surfactant.
lxiv. adalimumab, Inositol, sodium gluconate, and 0.01 to 1 mg/mL PVP surfactant having a weight average molecular weight of between 1900 and 3100.
lxv. adalimumab, Inositol, and 45 mM to 230 mM metal (hydroxy)mono-/dicarboxylate salt tonicifier.
lxvi. adalimumab, Inositol, 45 mM to 230 mM metal (hydroxy)mono-/di-carboxylate salt tonicifier, and a PVP surfactant.
lxvii. adalimumab, Inositol, 45 mM to 230 mM metal (hydroxy)mono-/di-carboxylate salt tonicifier, and a PVP surfactant having a weight average molecular weight of between 1900 and 3100.
lxviii. adalimumab, Inositol, 45 mM to 230 mM metal (hydroxy)mono-/di-carboxylate salt tonicifier, and 0.01 to 1 mg/mL of a PVP surfactant.
lxix. adalimumab, Inositol, 45 mM to 230 mM metal (hydroxy)mono-/di-carboxylate salt tonicifier, and 0.01 to 1 mg/mL PVP surfactant having a weight average molecular weight of between 1900 and 3100.
lxx. adalimumab, Inositol, and 45 mM to 230 mM sodium gluconate.
lxxi. adalimumab, Inositol, 45 mM to 230 mM sodium gluconate, and a PVP surfactant.
lxxii. adalimumab, Inositol, 45 mM to 230 mM sodium gluconate, and a PVP surfactant having a weight average molecular weight of between 1900 and 3100.
lxxiii. adalimumab, Inositol, 45 mM to 230 mM sodium gluconate, and 0.01 to 1 mg/mL of a PVP surfactant.
lxxiv. adalimumab, Inositol, 45 mM to 230 mM sodium gluconate, and 0.01 to 1 mg/mL PVP surfactant having a weight average molecular weight of between 1900 and 3100.
lxxv. adalimumab, 10 to 40 mg/mL monocyclic sugar stabiliser.
lxxvi. adalimumab, 10 to 40 mg/mL monocyclic sugar stabiliser, and a PVP surfactant. lxxvii. adalimumab, 10 to 40 mg/mL monocyclic sugar stabiliser, and a PVP surfactant having a weight average molecular weight of between 1900 and 3100.
lxxviii. adalimumab, 10 to 40 mg/mL monocyclic sugar stabiliser, and 0.01 to 1 mg/mL of a PVP surfactant.
lxxix. adalimumab, 10 to 40 mg/mL monocyclic sugar stabiliser, and 0.01 to 1 mg/mL PVP surfactant having a weight average molecular weight of between 1900 and 3100.
lxxx. adalimumab, 10 to 40 mg/mL monocyclic sugar stabiliser, and a metal (hydroxy)mono-/di-carboxylate salt tonicifier.
lxxxi. adalimumab, 10 to 40 mg/mL monocyclic sugar stabiliser, a metal (hydroxy)mono-/di-carboxylate salt tonicifier, and a PVP surfactant.
lxxxii. adalimumab, 10 to 40 mg/mL monocyclic sugar stabiliser, a metal (hydroxy)mono-/di-carboxylate salt tonicifier, and a PVP surfactant having a weight average molecular weight of between 1900 and 3100.
lxxxiii. adalimumab, 10 to 40 mg/mL monocyclic sugar stabiliser, a metal (hydroxy)mono-/di-carboxylate salt tonicifier, and 0.01 to 1 mg/mL of a PVP surfactant.
lxxxiv. adalimumab, 10 to 40 mg/mL monocyclic sugar stabiliser, a metal (hydroxy)mono-/di-carboxylate salt tonicifier, and 0.01 to 1 mg/mL PVP surfactant having a weight average molecular weight of between 1900 and 3100.
lxxxv. adalimumab, 10 to 40 mg/mL monocyclic sugar stabiliser, and sodium gluconate. lxxxvi. adalimumab, 10 to 40 mg/mL monocyclic sugar stabiliser, sodium gluconate, and a PVP surfactant.
lxxxvii. adalimumab, 10 to 40 mg/mL monocyclic sugar stabiliser, sodium gluconate, and a PVP surfactant having a weight average molecular weight of between 1900 and 3100.
lxxxviii. adalimumab, 10 to 40 mg/mL monocyclic sugar stabiliser, sodium gluconate, and 0.01 to 1 mg/mL of a PVP surfactant.
lxxxix. adalimumab, 10 to 40 mg/mL monocyclic sugar stabiliser, sodium gluconate, and 0.01 to 1 mg/mL PVP surfactant having a weight average molecular weight of between 1900 and 3100.
xc. adalimumab, 10 to 40 mg/mL monocyclic sugar stabiliser, and 45 mM to 230 mM metal (hydroxy)mono-/di-carboxylate salt tonicifier.
xci. adalimumab, 10 to 40 mg/mL monocyclic sugar stabiliser, 45 mM to 230 mM metal (hydroxy)mono-/di-carboxylate salt tonicifier, and a PVP surfactant.
xcii. adalimumab, 10 to 40 mg/mL monocyclic sugar stabiliser, 45 mM to 230 mM metal (hydroxy)mono-/di-carboxylate salt tonicifier, and a PVP surfactant having a weight average molecular weight of between 1900 and 3100.
xciii. adalimumab, 10 to 40 mg/mL monocyclic sugar stabiliser, 45 mM to 230 mM metal (hydroxy)mono-/di-carboxylate salt tonicifier, and 0.01 to 1 mg/mL of a PVP surfactant.
xciv. adalimumab, 10 to 40 mg/mL monocyclic sugar stabiliser, 45 mM to 230 mM metal (hydroxy)mono-/di-carboxylate salt tonicifier, and 0.01 to 1 mg/mL PVP surfactant having a weight average molecular weight of between 1900 and 3100.
xcv. adalimumab, 10 to 40 mg/mL monocyclic sugar stabiliser, and 45 mM to 230 mM sodium gluconate.
xcvi. adalimumab, 10 to 40 mg/mL monocyclic sugar stabiliser, 45 mM to 230 mM sodium gluconate, and a PVP surfactant.
xcvii. adalimumab, 10 to 40 mg/mL monocyclic sugar stabiliser, 45 mM to 230 mM sodium gluconate, and a PVP surfactant having a weight average molecular weight of between 1900 and 3100.
xcviii. adalimumab, 10 to 40 mg/mL monocyclic sugar stabiliser, 45 mM to 230 mM sodium gluconate, and 0.01 to 1 mg/mL of a PVP surfactant.
xcix. adalimumab, 10 to 40 mg/mL monocyclic sugar stabiliser, 45 mM to 230 mM sodium gluconate, and 0.01 to 1 mg/mL PVP surfactant having a weight average molecular weight of between 1900 and 3100.
c. adalimumab, 10 to 40 mg/mL Inositol.
ci. adalimumab, 10 to 40 mg/mL Inositol, and a PVP surfactant.
cii. adalimumab, 10 to 40 mg/mL Inositol, and a PVP surfactant having a weight average molecular weight of between 1900 and 3100.
ciii. adalimumab, 10 to 40 mg/mL Inositol, and 0.01 to 1 mg/mL of a PVP surfactant.
civ. adalimumab, 10 to 40 mg/mL Inositol, and 0.01 to 1 mg/mL PVP surfactant having a weight average molecular weight of between 1900 and 3100.
cv. adalimumab, 10 to 40 mg/mL Inositol, and a metal (hydroxy)mono-/di-carboxylate salt tonicifier.
cvi. adalimumab, 10 to 40 mg/mL Inositol, a metal (hydroxy)mono-/di-carboxylate salt tonicifier, and a PVP surfactant.
cvii. adalimumab, 10 to 40 mg/mL Inositol, a metal (hydroxy)mono-/di-carboxylate salt tonicifier, and a PVP surfactant having a weight average molecular weight of between 1900 and 3100.
cviii. adalimumab, 10 to 40 mg/mL Inositol, a metal (hydroxy)mono-/di-carboxylate salt tonicifier, and 0.01 to 1 mg/mL of a PVP surfactant.
cix. adalimumab, 10 to 40 mg/mL Inositol, a metal (hydroxy)mono-/di-carboxylate salt tonicifier, and 0.01 to 1 mg/mL PVP surfactant having a weight average molecular weight of between 1900 and 3100.
cx. adalimumab, 10 to 40 mg/mL Inositol, and sodium gluconate.
cxi. adalimumab, 10 to 40 mg/mL Inositol, sodium gluconate, and a PVP surfactant. cxii. adalimumab, 10 to 40 mg/mL Inositol, sodium gluconate, and a PVP surfactant having a weight average molecular weight of between 1900 and 3100.
cxiii. adalimumab, 10 to 40 mg/mL Inositol, sodium gluconate, and 0.01 to 1 mg/mL of a PVP surfactant.
cxiv. adalimumab, 10 to 40 mg/mL Inositol, sodium gluconate, and 0.01 to 1 mg/mL PVP surfactant having a weight average molecular weight of between 1900 and 3100.
cxv. adalimumab, 10 to 40 mg/mL Inositol, and 45 mM to 230 mM metal (hydroxy)mono-/di-carboxylate salt tonicifier.
cxvi. adalimumab, 10 to 40 mg/mL Inositol, 45 mM to 230 mM metal (hydroxy)mono-/di-carboxylate salt tonicifier, and a PVP surfactant.
cxvii. adalimumab, 10 to 40 mg/mL Inositol, 45 mM to 230 mM metal (hydroxy)mono-/di-carboxylate salt tonicifier, and a PVP surfactant having a weight average molecular weight of between 1900 and 3100.
cxviii. adalimumab, 10 to 40 mg/mL Inositol, 45 mM to 230 mM metal (hydroxy)mono-/di-carboxylate salt tonicifier, and 0.01 to 1 mg/mL of a PVP surfactant.
cxix. adalimumab, 10 to 40 mg/mL Inositol, 45 mM to 230 mM metal (hydroxy)mono-/di-carboxylate salt tonicifier, and 0.01 to 1 mg/mL PVP surfactant having a weight average molecular weight of between 1900 and 3100.
cxx. adalimumab, 10 to 40 mg/mL Inositol, and 45 mM to 230 mM sodium gluconate. cxxi. adalimumab, 10 to 40 mg/mL Inositol, 45 mM to 230 mM sodium gluconate, and a PVP surfactant.
cxxii. adalimumab, 10 to 40 mg/mL Inositol, 45 mM to 230 mM sodium gluconate, and a PVP surfactant having a weight average molecular weight of between 1900 and 3100.
cxxiii. adalimumab, 10 to 40 mg/mL Inositol, 45 mM to 230 mM sodium gluconate, and 0.01 to 1 mg/mL of a PVP surfactant.
cxxiv. adalimumab, 10 to 40 mg/mL Inositol, 45 mM to 230 mM sodium gluconate, and 0.01 to 1 mg/mL PVP surfactant having a weight average molecular weight of between 1900 and 3100.
cxxv. a monocyclic sugar stabiliser, a PVP surfactant.
cxxvi. a monocyclic sugar stabiliser, a PVP surfactant having a weight average molecular weight of between 1900 and 3100.
cxxvii. a monocyclic sugar stabiliser, 0.01 to 1 mg/mL of a PVP surfactant.
cxxviii. a monocyclic sugar stabiliser, 0.01 to 1 mg/mL PVP surfactant having a weight average molecular weight of between 1900 and 3100.
cxxix. a monocyclic sugar stabiliser, a metal (hydroxy)mono-/di-carboxylate salt tonicifier.
cxxx. a monocyclic sugar stabiliser, a metal (hydroxy)mono-/di-carboxylate salt tonicifier, and a PVP surfactant.
cxxxi. a monocyclic sugar stabiliser, a metal (hydroxy)mono-/di-carboxylate salt tonicifier, and a PVP surfactant having a weight average molecular weight of between 1900 and 3100.
cxxxii. a monocyclic sugar stabiliser, a metal (hydroxy)mono-/di-carboxylate salt tonicifier, and 0.01 to 1 mg/mL of a PVP surfactant.
cxxxiii. a monocyclic sugar stabiliser, a metal (hydroxy)mono-/di-carboxylate salt tonicifier, and 0.01 to 1 mg/mL PVP surfactant having a weight average molecular weight of between 1900 and 3100.
cxxxiv. a monocyclic sugar stabiliser, sodium gluconate.
cxxxv. a monocyclic sugar stabiliser, sodium gluconate, and a PVP surfactant.
cxxxvi. a monocyclic sugar stabiliser, sodium gluconate, and a PVP surfactant having a weight average molecular weight of between 1900 and 3100.
cxxxvii. a monocyclic sugar stabiliser, sodium gluconate, and 0.01 to 1 mg/mL of a PVP surfactant.
cxxviii. a monocyclic sugar stabiliser, sodium gluconate, and 0.01 to 1 mg/mL PVP surfactant having a weight average molecular weight of between 1900 and 3100.
cxxxix. a monocyclic sugar stabiliser, 45 mM to 230 mM metal (hydroxy)mono-/dicarboxylate salt tonicifier.
cxl. a monocyclic sugar stabiliser, 45 mM to 230 mM metal (hydroxy)mono-/dicarboxylate salt tonicifier, and a PVP surfactant.
cxli. a monocyclic sugar stabiliser, 45 mM to 230 mM metal (hydroxy)mono-/dicarboxylate salt tonicifier, and a PVP surfactant having a weight average molecular weight of between 1900 and 3100.
cxlii. a monocyclic sugar stabiliser, 45 mM to 230 mM metal (hydroxy)mono-/dicarboxylate salt tonicifier, and 0.01 to 1 mg/mL of a PVP surfactant.
cxliii. a monocyclic sugar stabiliser, 45 mM to 230 mM metal (hydroxy)mono-/dicarboxylate salt tonicifier, and 0.01 to 1 mg/mL PVP surfactant having a weight average molecular weight of between 1900 and 3100.
cxliv. a monocyclic sugar stabiliser, 45 mM to 230 mM sodium gluconate.
cxlv. a monocyclic sugar stabiliser, 45 mM to 230 mM sodium gluconate, and a PVP surfactant.
cxlvi. a monocyclic sugar stabiliser, 45 mM to 230 mM sodium gluconate, and a PVP surfactant having a weight average molecular weight of between 1900 and 3100.
cxlvii. a monocyclic sugar stabiliser, 45 mM to 230 mM sodium gluconate, and 0.01 to 1 mg/mL of a PVP surfactant.
cxlviii. a monocyclic sugar stabiliser, 45 mM to 230 mM sodium gluconate, and 0.01 to 1 mg/mL PVP surfactant having a weight average molecular weight of between 1900 and 3100.
cxlix. a metal (hydroxy)mono-/di-carboxylate salt tonicifier, a PVP surfactant.
cl. a metal (hydroxy)mono-/di-carboxylate salt tonicifier, a PVP surfactant having a weight average molecular weight of between 1900 and 3100.
cli. a metal (hydroxy)mono-/di-carboxylate salt tonicifier, 0.01 to 1 mg/mL of a PVP surfactant.
clii. a metal (hydroxy)mono-/di-carboxylate salt tonicifier, 0.01 to 1 mg/mL PVP surfactant having a weight average molecular weight of between 1900 and 3100.

9. The liquid pharmaceutical composition as claimed in any preceding claim, wherein the composition has a pH between pH 5.0 and 5.5.

10. The liquid pharmaceutical composition as claimed in any preceding claim, wherein the composition is either (substantially or entirely) free of arginine or comprises arginine in a concentration of at most 0.1 mM.

11. The liquid pharmaceutical composition as claimed in any preceding claim, wherein the composition is either (substantially or entirely) free of amino acids or comprises one or more amino acids in a (collective) concentration of at most 0.1 mM.

12. The liquid pharmaceutical composition as claimed in any preceding claim, wherein the composition comprises a sugar stabiliser selected from the group consisting of inositol (e.g. the inositol sugar stabiliser of claim 2), trehalose, mannitol, sucrose, sorbitol, maltose, lactose, xylitol, arabitol, erythritol, lactitol, and maltitol.

13. The liquid pharmaceutical composition as claimed in any preceding claim, wherein the composition comprises a sugar stabiliser, wherein the sugar stabiliser is the monocyclic sugar stabiliser.

14. The liquid pharmaceutical composition as claimed in claim 12, wherein the sugar stabiliser is selected from the group consisting of inositol (e.g. the inositol sugar stabiliser of claim 2) and trehalose.

15. The liquid pharmaceutical composition as claimed in any preceding claim, wherein the composition comprises a metal salt tonicifier.

16. The liquid pharmaceutical composition as claimed in claim 15, wherein the metal salt tonicifier is selected from the group consisting of a metal halide and the metal (hydroxy)mono-/di-carboxylate salt tonicifier.

17. The liquid pharmaceutical composition as claimed in claim 16, wherein the metal salt tonicifier is selected from the group consisting of sodium chloride and sodium gluconate.

18. The liquid pharmaceutical composition as claimed in any preceding claim, wherein the osmolality of the composition is between 220 and 400 mOsm/kg.

19. The liquid pharmaceutical composition as claimed in any preceding claim, wherein the composition comprises:
- 40 to about 60 mg/mL adalimumab;
- 5 to about 15 mM sodium acetate/acetic acid buffer system;
- 175 to about 225 mM trehalose;
- 25 to about 75 mM sodium chloride;
- 0.01 to 1 mg/mL PVP having a weight average molecular weight of between 1900 and 3100; and
- water (for injection);
wherein the composition:
• has a pH between 5.1 and 5.3;
• is free of arginine or comprises arginine in a concentration of at most 0.001 mM;
• is free of amino acids or comprises one or more amino acids in a (collective) concentration of at most 0.001 mM.
• is free of surfactants with the exception of PVP having a weight average molecular weight of between 1900 and 3100 or comprises one or more of said surfactants, excluding PVP having a weight average molecular weight of between 1900 and 3100, in a (collective) concentration of at most 0.0001 mM; and/or
• is free of phosphate buffering agents (e.g. sodium dihydrogen phosphate, disodium hydrogen phosphate) or comprises a phosphate buffer system in a concentration of at most 0.001 mM.

20. The liquid pharmaceutical composition as claimed in any of claims 1 to 18, wherein the composition comprises:
- 40 to about 60 mg/mL adalimumab;
- 5 to about 15 mM sodium acetate/acetic acid buffer system;
- 10 to 40 mg/mL inositol;
- 25 to about 75 mM sodium chloride;
- 0.5 mg/mL to about 1.5 mg/mL polysorbate 80; and
- water (for injection);
wherein the composition:
• has a pH between 5.1 and 5.3;
• is (substantially or entirely) free of arginine (suitably L-arginine) or comprises arginine in a concentration of at most 0.001 mM;
• is (substantially or entirely) free of amino acids or comprises one or more amino acids in a (collective) concentration of at most 0.001 mM.
• is (substantially or entirely) free of surfactants with the exception of polysorbate 80 or comprises one or more of said surfactants (excluding polysorbate 80) in a (collective) concentration of at most 0.0001 mM; and/or
• is (substantially or entirely) free of phosphate buffering agents (e.g. sodium dihydrogen phosphate, disodium hydrogen phosphate) or comprises a phosphate buffer system in a concentration of at most 0.001 mM.

21. The liquid pharmaceutical composition as claimed in any of claims 1 to 18, wherein the composition comprises:
- 40 to about 60 mg/mL adalimumab;
- 5 to about 15 mM sodium acetate/acetic acid buffer system;
- 10 to 40 mg/mL inositol;
- 25 to about 75 mM sodium chloride;
- 0.01 to 1 mg/mL PVP having a weight average molecular weight of between 1900 and 3100; and
- water (for injection);
wherein the composition:
• has a pH between 5.1 and 5.3;
• is (substantially or entirely) free of arginine (suitably L-arginine) or comprises arginine in a concentration of at most 0.001 mM;
• is (substantially or entirely) free of amino acids or comprises one or more amino acids in a (collective) concentration of at most 0.001 mM.
• is (substantially or entirely) free of surfactants with the exception of PVP having a weight average molecular weight of between 1900 and 3100 or comprises one or more of said surfactants (excluding PVP having a weight average molecular weight of between 1900 and 3100) in a (collective) concentration of at most 0.0001 mM; and/or
• is (substantially or entirely) free of phosphate buffering agents (e.g. sodium dihydrogen phosphate, disodium hydrogen phosphate) or comprises a phosphate buffer system in a concentration of at most 0.001 mM.

22. The liquid pharmaceutical composition as claimed in any of claims 1 to 18, wherein the composition comprises:
- 40 to about 60 mg/mL adalimumab;
- 5 to about 15 mM sodium acetate/acetic acid buffer system;
- 10 to 40 mg/mL inositol;
- 45 mM and 230 mM sodium gluconate;
- 0.01 to 1 mg/mL PVP having a weight average molecular weight of between 1900 and 3100; and
- water (for injection);
wherein the composition:
• has a pH between 5.1 and 5.3;
• is (substantially or entirely) free of arginine (suitably L-arginine) or comprises arginine in a concentration of at most 0.001 mM;
• is (substantially or entirely) free of amino acids or comprises one or more amino acids in a (collective) concentration of at most 0.001 mM.
• is (substantially or entirely) free of surfactants with the exception of PVP having a weight average molecular weight of between 1900 and 3100 or comprises one or more of said surfactants (excluding PVP having a weight average molecular weight of between 1900 and 3100) in a (collective) concentration of at most 0.0001 mM; and/or
• is (substantially or entirely) free of phosphate buffering agents (e.g. sodium dihydrogen phosphate, disodium hydrogen phosphate) or comprises a phosphate buffer system in a concentration of at most 0.001 mM.

23. The liquid pharmaceutical composition as claimed in any of claims 1 to 18, wherein the composition consists of:
- 50 mg/mL adalimumab;
- 10 mM sodium acetate/acetic acid buffer system;
- 200 mM trehalose;
- 50 mM sodium chloride;
- 0.01 to 1 mg/mL PVP having a weight average molecular weight of between 1900 and 3100; and
- water (for injection);
wherein the composition has a pH of 5.2.

24. The liquid pharmaceutical composition as claimed in any of claims 1 to 18, wherein the composition consists of:
- 50 mg/mL adalimumab;
- 10 mM sodium acetate/acetic acid buffer system;
- 10 to 40 mg/mL inositol (or 55 mM to 225mM inositol);
- 50 mM sodium chloride;
- 1 mg/mL polysorbate 80; and
- water (for injection);
wherein the composition has a pH of 5.2.

25. The liquid pharmaceutical composition as claimed in any of claims 1 to 18, wherein the composition consists of:
- 50 mg/mL adalimumab;
- 10 mM sodium acetate/acetic acid buffer system;
- 10 to 40 mg/mL inositol (or 55 mM to 225mM inositol);
- 50 mM sodium chloride;
- 0.01 to 1 mg/mL PVP having a weight average molecular weight of between 1900 and 3100; and
- water (for injection);
wherein the composition has a pH of 5.2.

26. The liquid pharmaceutical composition as claimed in any of claims 1 to 18, wherein the composition consists of:
- 50 mg/mL adalimumab;
- 10 mM sodium acetate/acetic acid buffer system;
- 10 to 40 mg/mL inositol (or 55 mM to 225mM inositol);
- 45 mM and 230 mM sodium gluconate;
- 0.01 to 1 mg/mL PVP having a weight average molecular weight of between 1900 and 3100; and
- water (for injection);
wherein the composition has a pH of 5.2.

27. A drug delivery device comprising a liquid pharmaceutical composition as claimed in any preceding claim.

28. A liquid pharmaceutical composition as claimed in any of claims 1 to 26 for use in the treatment of rheumatoid arthritis, psoriatic arthritis, ankylosing spondylitis, Crohn's disease, ulcerative colitis, moderate to severe chronic psoriasis and/or juvenile idiopathic arthritis.
